(19) Europäisches Patentamt
European Patent Office
Office européen des brevets

(11) EP 3 909 580 A1

(12) **EUROPEAN PATENT APPLICATION**
published in accordance with Art. 153(4) EPC

(43) Date of publication:
**17.11.2021 Bulletin 2021/46**

(21) Application number: **19895941.3**

(22) Date of filing: **10.12.2019**

(51) Int Cl.:
*A61K 31/4184* (2006.01) *A61K 31/454* (2006.01)
*A61K 31/4745* (2006.01) *A61K 31/502* (2006.01)
*A61K 31/5025* (2006.01) *A61K 31/55* (2006.01)
*A61K 45/00* (2006.01) *A61P 35/00* (2006.01)
*A61P 43/00* (2006.01) *C07K 16/28* (2006.01)
*A61K 47/68* (2017.01) *C12N 15/13* (2006.01)

(86) International application number:
**PCT/JP2019/048171**

(87) International publication number:
**WO 2020/122034 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.12.2018 JP 2018231948**

(71) Applicant: **Daiichi Sankyo Company, Limited**
**Tokyo 103-8426 (JP)**

(72) Inventors:
- **OGITANI Yusuke**
  **Tokyo 103-8426 (JP)**
- **OKAJIMA Daisuke**
  **Tokyo 103-8426 (JP)**
- **HASHIMOTO Yuuri**
  **Tokyo 103-8426 (JP)**
- **SUZUKI Hirokazu**
  **Tokyo 103-8426 (JP)**

(74) Representative: **Nieuwenhuys, William Francis**
**Marks & Clerk LLP**
**15 Fetter Lane**
**London EC4A 1BW (GB)**

(54) **COMBINATION OF ANTIBODY-DRUG CONJUGATE WITH PARP INHIBITOR**

(57) A pharmaceutical composition, wherein an antibody-drug conjugate in which a drug-linker represented by the following formula (wherein A represents a connecting position to an antibody) is conjugated to the antibody via a thioether bond, and a PARP inhibitor are administered in combination, and/or a method of treatment, wherein the antibody-drug conjugate and a PARP inhibitor are administrated in combination to a subject.

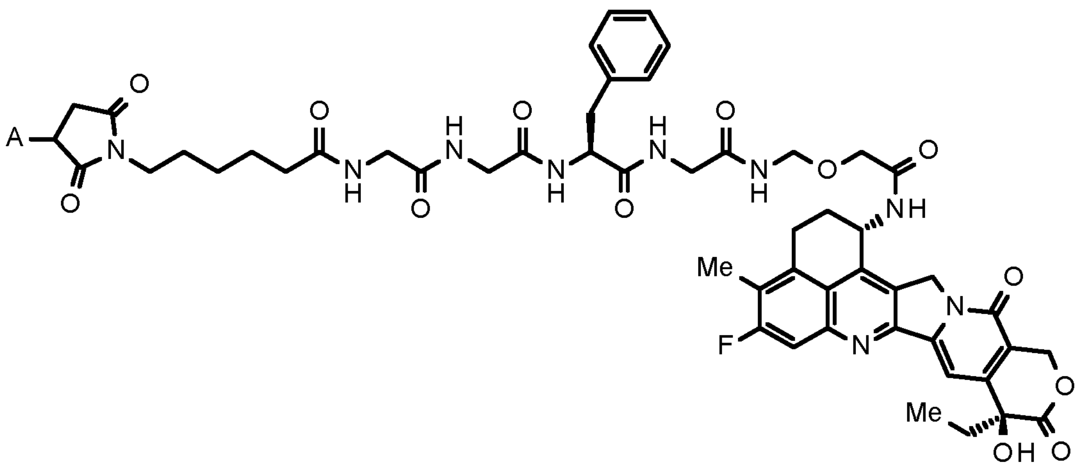

EP 3 909 580 A1

[Figure 1]

SEQ ID No: 1 - Amino acid sequence of a heavy chain of the anti-HER2 antibody

EVQLVESGGGLVQPGGSLRLSCAASGFNIKDTYIHWVR
QAPGKGLEWVARIYPTNGYTRYADSVKGRFTISADTSK
NTAYLQMNSLRAEDTAVYYCSRWGGDGFYAMDYWGQGT
LVTVSSASTKGPSVFPLAPSSKSTSGGTAALGCLVKDY
FPEPVTVSWNSGALTSGVHTFPAVLQSSGLYSLSSVVT
VPSSSLGTQTYICNVNHKPSNTKVDKKVEPKSCDKTHT
CPPCPAPELLGGPSVFLFPPKPKDTLMISRTPEVTCVV
VDVSHEDPEVKFNWYVDGVEVHNAKTKPREEQYNSTYR
VVSVLTVLHQDWLNGKEYKCKVSNKALPAPIEKTISKA
KGQPREPQVYTLPPSREEMTKNQVSLTCLVKGFYPSDI
AVEWESNGQPENNYKTTPPVLDSDGSFFLYSKLTVDKS
RWQQGNVFSCSVMHEALHNHYTQKSLSLSPGK

SEQUENCE LISTING

<110>  DAIICHI SANKYO COMPANY, LIMITED

<120>  COMBINATION OF ANTIBODY-DRUG CONJUGATE AND PARP INHIBITOR

<130>  DSFP1931WO

<150>  JP2018-231948
<151>  2018-12-11

<160>  12

<170>  PatentIn version 3.5

<210>  1
<211>  450
<212>  PRT
<213>  Artificial Sequence

<220>
<223>  Heavy chain of anti-HER2 antibody

<400>  1

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160
```

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp

405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

Gly Lys
450

<210> 2
<211> 214
<212> PRT
<213> Artificial Sequence

<220>
<223> Light chain of anti-HER2 antibody

<400> 2

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 3
<211> 447
<212> PRT
<213> Artificial Sequence

<220>
<223> Heavy chain of anti-HER3 antibody

<400> 3

Gln Val Gln Leu Gln Gln Trp Gly Ala Gly Leu Leu Lys Pro Ser Glu
1 5 10 15

Thr Leu Ser Leu Thr Cys Ala Val Tyr Gly Gly Ser Phe Ser Gly Tyr
20 25 30

Tyr Trp Ser Trp Ile Arg Gln Pro Pro Gly Lys Gly Leu Glu Trp Ile
35 40 45

Gly Glu Ile Asn His Ser Gly Ser Thr Asn Tyr Asn Pro Ser Leu Lys
50 55 60

Ser Arg Val Thr Ile Ser Val Glu Thr Ser Lys Asn Gln Phe Ser Leu
65 70 75 80

Lys Leu Ser Ser Val Thr Ala Ala Asp Thr Ala Val Tyr Tyr Cys Ala
85 90 95

Arg Asp Lys Trp Thr Trp Tyr Phe Asp Leu Trp Gly Arg Gly Thr Leu
100 105 110

Val Thr Val Ser Ser Ala Ser Thr Lys Gly Pro Ser Val Phe Pro Leu
115 120 125

Ala Pro Ser Ser Lys Ser Thr Ser Gly Gly Thr Ala Ala Leu Gly Cys
130 135 140

```
Leu Val Lys Asp Tyr Phe Pro Glu Pro Val Thr Val Ser Trp Asn Ser
145                 150                 155                 160

Gly Ala Leu Thr Ser Gly Val His Thr Phe Pro Ala Val Leu Gln Ser
                165                 170                 175

Ser Gly Leu Tyr Ser Leu Ser Ser Val Val Thr Val Pro Ser Ser Ser
            180                 185                 190

Leu Gly Thr Gln Thr Tyr Ile Cys Asn Val Asn His Lys Pro Ser Asn
        195                 200                 205

Thr Lys Val Asp Lys Arg Val Glu Pro Lys Ser Cys Asp Lys Thr His
    210                 215                 220

Thr Cys Pro Pro Cys Pro Ala Pro Glu Leu Leu Gly Gly Pro Ser Val
225                 230                 235                 240

Phe Leu Phe Pro Pro Lys Pro Lys Asp Thr Leu Met Ile Ser Arg Thr
                245                 250                 255

Pro Glu Val Thr Cys Val Val Val Asp Val Ser His Glu Asp Pro Glu
            260                 265                 270

Val Lys Phe Asn Trp Tyr Val Asp Gly Val Glu Val His Asn Ala Lys
        275                 280                 285

Thr Lys Pro Arg Glu Glu Gln Tyr Asn Ser Thr Tyr Arg Val Val Ser
    290                 295                 300

Val Leu Thr Val Leu His Gln Asp Trp Leu Asn Gly Lys Glu Tyr Lys
305                 310                 315                 320

Cys Lys Val Ser Asn Lys Ala Leu Pro Ala Pro Ile Glu Lys Thr Ile
                325                 330                 335

Ser Lys Ala Lys Gly Gln Pro Arg Glu Pro Gln Val Tyr Thr Leu Pro
            340                 345                 350

Pro Ser Arg Glu Glu Met Thr Lys Asn Gln Val Ser Leu Thr Cys Leu
        355                 360                 365

Val Lys Gly Phe Tyr Pro Ser Asp Ile Ala Val Glu Trp Glu Ser Asn
    370                 375                 380

Gly Gln Pro Glu Asn Asn Tyr Lys Thr Thr Pro Pro Val Leu Asp Ser
385                 390                 395                 400
```

Asp Gly Ser Phe Phe Leu Tyr Ser Lys Leu Thr Val Asp Lys Ser Arg
405 410 415

Trp Gln Gln Gly Asn Val Phe Ser Cys Ser Val Met His Glu Ala Leu
420 425 430

His Asn His Tyr Thr Gln Lys Ser Leu Ser Leu Ser Pro Gly Lys
435 440 445

<210> 4
<211> 220
<212> PRT
<213> Artificial Sequence

<220>
<223> Light chain of anti-HER3 antibody

<400> 4

Asp Ile Glu Met Thr Gln Ser Pro Asp Ser Leu Ala Val Ser Leu Gly
1 5 10 15

Glu Arg Ala Thr Ile Asn Cys Arg Ser Ser Gln Ser Val Leu Tyr Ser
20 25 30

Ser Ser Asn Arg Asn Tyr Leu Ala Trp Tyr Gln Gln Asn Pro Gly Gln
35 40 45

Pro Pro Lys Leu Leu Ile Tyr Trp Ala Ser Thr Arg Glu Ser Gly Val
50 55 60

Pro Asp Arg Phe Ser Gly Ser Gly Ser Gly Thr Asp Phe Thr Leu Thr
65 70 75 80

Ile Ser Ser Leu Gln Ala Glu Asp Val Ala Val Tyr Tyr Cys Gln Gln
85 90 95

Tyr Tyr Ser Thr Pro Arg Thr Phe Gly Gln Gly Thr Lys Val Glu Ile
100 105 110

Lys Arg Thr Val Ala Ala Pro Ser Val Phe Ile Phe Pro Pro Ser Asp
115 120 125

Glu Gln Leu Lys Ser Gly Thr Ala Ser Val Val Cys Leu Leu Asn Asn
130 135 140

Phe Tyr Pro Arg Glu Ala Lys Val Gln Trp Lys Val Asp Asn Ala Leu
145 150 155 160

Gln Ser Gly Asn Ser Gln Glu Ser Val Thr Glu Gln Asp Ser Lys Asp
165 170 175

Ser Thr Tyr Ser Leu Ser Ser Thr Leu Thr Leu Ser Lys Ala Asp Tyr
180 185 190

Glu Lys His Lys Val Tyr Ala Cys Glu Val Thr His Gln Gly Leu Ser
195 200 205

Ser Pro Val Thr Lys Ser Phe Asn Arg Gly Glu Cys
210 215 220

<210> 5
<211> 470
<212> PRT
<213> Artificial Sequence

<220>
<223> Heavy chain of anti-TROP2 antibody

<400> 5

Met Lys His Leu Trp Phe Phe Leu Leu Leu Val Ala Ala Pro Arg Trp
1 5 10 15

Val Leu Ser Gln Val Gln Leu Val Gln Ser Gly Ala Glu Val Lys Lys
20 25 30

Pro Gly Ala Ser Val Lys Val Ser Cys Lys Ala Ser Gly Tyr Thr Phe
35 40 45

Thr Thr Ala Gly Met Gln Trp Val Arg Gln Ala Pro Gly Gln Gly Leu
50 55 60

Glu Trp Met Gly Trp Ile Asn Thr His Ser Gly Val Pro Lys Tyr Ala
65 70 75 80

Glu Asp Phe Lys Gly Arg Val Thr Ile Ser Ala Asp Thr Ser Thr Ser
85 90 95

Thr Ala Tyr Leu Gln Leu Ser Ser Leu Lys Ser Glu Asp Thr Ala Val
100 105 110

Tyr Tyr Cys Ala Arg Ser Gly Phe Gly Ser Ser Tyr Trp Tyr Phe Asp
115 120 125

Val Trp Gly Gln Gly Thr Leu Val Thr Val Ser Ser Ala Ser Thr Lys
130 135 140

```
Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Arg Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Glu Glu Met Thr Lys Asn
    370                 375                 380

Gln Val Ser Leu Thr Cys Leu Val Lys Gly Phe Tyr Pro Ser Asp Ile
385                 390                 395                 400
```

Ala Val Glu Trp Glu Ser Asn Gly Gln Pro Glu Asn Asn Tyr Lys Thr
405 410 415

Thr Pro Pro Val Leu Asp Ser Asp Gly Ser Phe Phe Leu Tyr Ser Lys
420 425 430

Leu Thr Val Asp Lys Ser Arg Trp Gln Gln Gly Asn Val Phe Ser Cys
435 440 445

Ser Val Met His Glu Ala Leu His Asn His Tyr Thr Gln Lys Ser Leu
450 455 460

Ser Leu Ser Pro Gly Lys
465 470

<210> 6
<211> 234
<212> PRT
<213> Artificial Sequence

<220>
<223> Light chain of anti-TROP2 antibody

<400> 6

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

Ala Ser Val Gly Asp Arg Val Thr Ile Thr Cys Lys Ala Ser Gln Asp
35 40 45

Val Ser Thr Ala Val Ala Trp Tyr Gln Gln Lys Pro Gly Lys Ala Pro
50 55 60

Lys Leu Leu Ile Tyr Ser Ala Ser Tyr Arg Tyr Thr Gly Val Pro Ser
65 70 75 80

Arg Phe Ser Gly Ser Gly Ser Gly Thr Asp Phe Thr Leu Thr Ile Ser
85 90 95

Ser Leu Gln Pro Glu Asp Phe Ala Val Tyr Tyr Cys Gln Gln His Tyr
100 105 110

Ile Thr Pro Leu Thr Phe Gly Gln Gly Thr Lys Leu Glu Ile Lys Arg
115 120 125

```
Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230


<210>  7
<211>  471
<212>  PRT
<213>  Artificial Sequence

<220>
<223>  Heavy chain of anti-B7-H3 antibody

<400>  7

Met Lys His Leu Trp Phe Phe Leu Leu Leu Val Ala Ala Pro Arg Trp
1               5                   10                  15

Val Leu Ser Gln Val Gln Leu Val Gln Ser Gly Ala Glu Val Lys Lys
            20                  25                  30

Pro Gly Ser Ser Val Lys Val Ser Cys Lys Ala Ser Gly Tyr Thr Phe
        35                  40                  45

Thr Asn Tyr Val Met His Trp Val Arg Gln Ala Pro Gly Gln Gly Leu
    50                  55                  60

Glu Trp Met Gly Tyr Ile Asn Pro Tyr Asn Asp Asp Val Lys Tyr Asn
65                  70                  75                  80

Glu Lys Phe Lys Gly Arg Val Thr Ile Thr Ala Asp Glu Ser Thr Ser
                85                  90                  95

Thr Ala Tyr Met Glu Leu Ser Ser Leu Arg Ser Glu Asp Thr Ala Val
```

```
            100                 105                 110

Tyr Tyr Cys Ala Arg Trp Gly Tyr Tyr Gly Ser Pro Leu Tyr Tyr Phe
        115                 120                 125

Asp Tyr Trp Gly Gln Gly Thr Leu Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270

Asp Thr Leu Met Ile Ser Arg Thr Pro Glu Val Thr Cys Val Val Val
        275                 280                 285

Asp Val Ser His Glu Asp Pro Glu Val Lys Phe Asn Trp Tyr Val Asp
    290                 295                 300

Gly Val Glu Val His Asn Ala Lys Thr Lys Pro Arg Glu Glu Gln Tyr
305                 310                 315                 320

Asn Ser Thr Tyr Arg Val Val Ser Val Leu Thr Val Leu His Gln Asp
                325                 330                 335

Trp Leu Asn Gly Lys Glu Tyr Lys Cys Lys Val Ser Asn Lys Ala Leu
            340                 345                 350
```

```
Pro Ala Pro Ile Glu Lys Thr Ile Ser Lys Ala Lys Gly Gln Pro Arg
        355                 360                 365

Glu Pro Gln Val Tyr Thr Leu Pro Pro Ser Arg Glu Glu Met Thr Lys
    370                 375                 380

Asn Gln Val Ser Leu Thr Cys Leu Val Lys Gly Phe Tyr Pro Ser Asp
385                 390                 395                 400

Ile Ala Val Glu Trp Glu Ser Asn Gly Gln Pro Glu Asn Asn Tyr Lys
                405                 410                 415

Thr Thr Pro Pro Val Leu Asp Ser Asp Gly Ser Phe Phe Leu Tyr Ser
            420                 425                 430

Lys Leu Thr Val Asp Lys Ser Arg Trp Gln Gln Gly Asn Val Phe Ser
        435                 440                 445

Cys Ser Val Met His Glu Ala Leu His Asn His Tyr Thr Gln Lys Ser
    450                 455                 460

Leu Ser Leu Ser Pro Gly Lys
465                 470


<210>  8
<211>  233
<212>  PRT
<213>  Artificial Sequence

<220>
<223>  Light chain of anti-B7-H3 antibody

<400>  8

Met Val Leu Gln Thr Gln Val Phe Ile Ser Leu Leu Leu Trp Ile Ser
1               5                   10                  15

Gly Ala Tyr Gly Glu Ile Val Leu Thr Gln Ser Pro Ala Thr Leu Ser
            20                  25                  30

Leu Ser Pro Gly Glu Arg Ala Thr Leu Ser Cys Arg Ala Ser Ser Arg
        35                  40                  45

Leu Ile Tyr Met His Trp Tyr Gln Gln Lys Pro Gly Gln Ala Pro Arg
    50                  55                  60

Pro Leu Ile Tyr Ala Thr Ser Asn Leu Ala Ser Gly Ile Pro Ala Arg
65                  70                  75                  80

Phe Ser Gly Ser Gly Ser Gly Thr Asp Phe Thr Leu Thr Ile Ser Ser
```

```
                85                  90                  95

Leu Glu Pro Glu Asp Phe Ala Val Tyr Tyr Cys Gln Gln Trp Asn Ser
            100                 105                 110

Asn Pro Pro Thr Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230


<210>  9
<211>  472
<212>  PRT
<213>  Artificial Sequence

<220>
<223>  Heavy chain of anti-GPR20 antibody

<400>  9

Met Lys His Leu Trp Phe Phe Leu Leu Leu Val Ala Ala Pro Arg Trp
1               5                   10                  15

Val Leu Ser Glu Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Ser Tyr Tyr Ile Ser Trp Ile Arg Gln Ala Pro Gly Gln Gly Leu
    50                  55                  60
```

```
Lys Tyr Met Gly Phe Ile Asn Pro Gly Ser Gly His Thr Asn Tyr Asn
65                  70                  75                  80

Glu Lys Phe Lys Gly Arg Val Thr Ile Thr Ala Asp Lys Ser Ser Ser
                85                  90                  95

Thr Ala Thr Met Glu Leu Ser Ser Leu Arg Ser Glu Asp Thr Ala Val
            100                 105                 110

Tyr Tyr Cys Ala Arg Gly Ala Gly Gly Phe Leu Arg Ile Ile Thr Lys
        115                 120                 125

Phe Asp Tyr Trp Gly Gln Gly Thr Leu Val Thr Val Ser Ser Ala Ser
    130                 135                 140

Thr Lys Gly Pro Ser Val Phe Pro Leu Ala Pro Ser Ser Lys Ser Thr
145                 150                 155                 160

Ser Gly Gly Thr Ala Ala Leu Gly Cys Leu Val Lys Asp Tyr Phe Pro
                165                 170                 175

Glu Pro Val Thr Val Ser Trp Asn Ser Gly Ala Leu Thr Ser Gly Val
            180                 185                 190

His Thr Phe Pro Ala Val Leu Gln Ser Ser Gly Leu Tyr Ser Leu Ser
        195                 200                 205

Ser Val Val Thr Val Pro Ser Ser Ser Leu Gly Thr Gln Thr Tyr Ile
    210                 215                 220

Cys Asn Val Asn His Lys Pro Ser Asn Thr Lys Val Asp Lys Arg Val
225                 230                 235                 240

Glu Pro Lys Ser Cys Asp Lys Thr His Thr Cys Pro Pro Cys Pro Ala
                245                 250                 255

Pro Glu Leu Leu Gly Gly Pro Ser Val Phe Leu Phe Pro Pro Lys Pro
            260                 265                 270

Lys Asp Thr Leu Met Ile Ser Arg Thr Pro Glu Val Thr Cys Val Val
        275                 280                 285

Val Asp Val Ser His Glu Asp Pro Glu Val Lys Phe Asn Trp Tyr Val
    290                 295                 300

Asp Gly Val Glu Val His Asn Ala Lys Thr Lys Pro Arg Glu Glu Gln
```

305 310 315 320

Tyr Asn Ser Thr Tyr Arg Val Val Ser Val Leu Thr Val Leu His Gln
325 330 335

Asp Trp Leu Asn Gly Lys Glu Tyr Lys Cys Lys Val Ser Asn Lys Ala
340 345 350

Leu Pro Ala Pro Ile Glu Lys Thr Ile Ser Lys Ala Lys Gly Gln Pro
355 360 365

Arg Glu Pro Gln Val Tyr Thr Leu Pro Pro Ser Arg Glu Glu Met Thr
370 375 380

Lys Asn Gln Val Ser Leu Thr Cys Leu Val Lys Gly Phe Tyr Pro Ser
385 390 395 400

Asp Ile Ala Val Glu Trp Glu Ser Asn Gly Gln Pro Glu Asn Asn Tyr
405 410 415

Lys Thr Thr Pro Pro Val Leu Asp Ser Asp Gly Ser Phe Phe Leu Tyr
420 425 430

Ser Lys Leu Thr Val Asp Lys Ser Arg Trp Gln Gln Gly Asn Val Phe
435 440 445

Ser Cys Ser Val Met His Glu Ala Leu His Asn His Tyr Thr Gln Lys
450 455 460

Ser Leu Ser Leu Ser Pro Gly Lys
465 470

<210> 10
<211> 234
<212> PRT
<213> Artificial Sequence

<220>
<223> Light chain of anti-GPR20 antibody

<400> 10

Met Val Leu Gln Thr Gln Val Phe Ile Ser Leu Leu Leu Trp Ile Ser
1 5 10 15

Gly Ala Tyr Gly Asp Thr Gln Leu Thr Gln Ser Pro Ser Ser Leu Ser
20 25 30

Ala Ser Val Gly Asp Arg Val Thr Ile Thr Cys Arg Ala Ser Lys Ser
35 40 45

Val Ser Thr Tyr Ile His Trp Tyr Gln Gln Lys Pro Gly Lys Gln Pro
    50                  55                  60

Lys Leu Leu Ile Tyr Ser Ala Gly Asn Leu Glu Ser Gly Val Pro Ser
65                  70                  75                  80

Arg Phe Ser Gly Ser Gly Ser Gly Thr Asp Phe Thr Leu Thr Ile Ser
                85                  90                  95

Ser Leu Gln Pro Glu Asp Phe Ala Asn Tyr Tyr Cys Gln Gln Ile Asn
            100                 105                 110

Glu Leu Pro Tyr Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230

<210> 11
<211> 471
<212> PRT
<213> Artificial Sequence

<220>
<223> Heavy chain of anti-CDH6 antibody

<400> 11

Met Lys His Leu Trp Phe Phe Leu Leu Leu Val Ala Ala Pro Arg Trp
1               5                   10                  15

```
Val Leu Ser Glu Val Gln Leu Val Gln Ser Gly Ala Glu Val Lys Lys
            20                  25                  30

Pro Gly Ala Ser Val Lys Val Ser Cys Lys Ala Ser Gly Tyr Thr Phe
        35                  40                  45

Thr Arg Asn Phe Met His Trp Val Arg Gln Ala Pro Gly Gln Gly Leu
    50                  55                  60

Glu Trp Met Gly Trp Ile Tyr Pro Gly Asp Gly Glu Thr Glu Tyr Ala
65                  70                  75                  80

Gln Lys Phe Gln Gly Arg Val Thr Ile Thr Ala Asp Thr Ser Thr Ser
                85                  90                  95

Thr Ala Tyr Met Glu Leu Ser Ser Leu Arg Ser Glu Asp Thr Ala Val
            100                 105                 110

Tyr Tyr Cys Ala Arg Gly Val Tyr Gly Gly Phe Ala Gly Gly Tyr Phe
        115                 120                 125

Asp Phe Trp Gly Gln Gly Thr Leu Val Thr Val Ser Ser Ala Ser Thr
    130                 135                 140

Lys Gly Pro Ser Val Phe Pro Leu Ala Pro Ser Ser Lys Ser Thr Ser
145                 150                 155                 160

Gly Gly Thr Ala Ala Leu Gly Cys Leu Val Lys Asp Tyr Phe Pro Glu
                165                 170                 175

Pro Val Thr Val Ser Trp Asn Ser Gly Ala Leu Thr Ser Gly Val His
            180                 185                 190

Thr Phe Pro Ala Val Leu Gln Ser Ser Gly Leu Tyr Ser Leu Ser Ser
        195                 200                 205

Val Val Thr Val Pro Ser Ser Ser Leu Gly Thr Gln Thr Tyr Ile Cys
    210                 215                 220

Asn Val Asn His Lys Pro Ser Asn Thr Lys Val Asp Lys Arg Val Glu
225                 230                 235                 240

Pro Lys Ser Cys Asp Lys Thr His Thr Cys Pro Pro Cys Pro Ala Pro
                245                 250                 255

Glu Leu Leu Gly Gly Pro Ser Val Phe Leu Phe Pro Pro Lys Pro Lys
            260                 265                 270
```

Asp Thr Leu Met Ile Ser Arg Thr Pro Glu Val Thr Cys Val Val Val
275 280 285

Asp Val Ser His Glu Asp Pro Glu Val Lys Phe Asn Trp Tyr Val Asp
290 295 300

Gly Val Glu Val His Asn Ala Lys Thr Lys Pro Arg Glu Glu Gln Tyr
305 310 315 320

Asn Ser Thr Tyr Arg Val Val Ser Val Leu Thr Val Leu His Gln Asp
325 330 335

Trp Leu Asn Gly Lys Glu Tyr Lys Cys Lys Val Ser Asn Lys Ala Leu
340 345 350

Pro Ala Pro Ile Glu Lys Thr Ile Ser Lys Ala Lys Gly Gln Pro Arg
355 360 365

Glu Pro Gln Val Tyr Thr Leu Pro Pro Ser Arg Glu Glu Met Thr Lys
370 375 380

Asn Gln Val Ser Leu Thr Cys Leu Val Lys Gly Phe Tyr Pro Ser Asp
385 390 395 400

Ile Ala Val Glu Trp Glu Ser Asn Gly Gln Pro Glu Asn Asn Tyr Lys
405 410 415

Thr Thr Pro Pro Val Leu Asp Ser Asp Gly Ser Phe Phe Leu Tyr Ser
420 425 430

Lys Leu Thr Val Asp Lys Ser Arg Trp Gln Gln Gly Asn Val Phe Ser
435 440 445

Cys Ser Val Met His Glu Ala Leu His Asn His Tyr Thr Gln Lys Ser
450 455 460

Leu Ser Leu Ser Pro Gly Lys
465 470

<210> 12
<211> 233
<212> PRT
<213> Artificial Sequence

<220>
<223> Light chain of anti-CDH6 antibody

<400> 12

```
Met Val Leu Gln Thr Gln Val Phe Ile Ser Leu Leu Leu Trp Ile Ser
1               5                   10                  15

Gly Ala Tyr Gly Asp Ile Gln Met Thr Gln Ser Pro Ser Ser Leu Ser
            20                  25                  30

Ala Ser Val Gly Asp Arg Val Thr Ile Thr Cys Lys Ala Ser Gln Asn
        35                  40                  45

Ile Tyr Lys Asn Leu Ala Trp Tyr Gln Gln Lys Pro Gly Lys Ala Pro
    50                  55                  60

Lys Leu Leu Ile Tyr Asp Ala Asn Thr Leu Gln Thr Gly Val Pro Ser
65                  70                  75                  80

Arg Phe Ser Gly Ser Gly Ser Gly Ser Asp Phe Thr Leu Thr Ile Ser
                85                  90                  95

Ser Leu Gln Pro Glu Asp Phe Ala Thr Tyr Phe Cys Gln Gln Tyr Tyr
            100                 105                 110

Ser Gly Trp Ala Phe Gly Gln Gly Thr Lys Val Glu Ile Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230
```

## Description

Technical Field

The present invention relates to a pharmaceutical composition, wherein a specific antibody-drug conjugate and a PARP inhibitor are administered in combination, and/or a method of treatment, wherein a specific antibody-drug conjugate and a PARP inhibitor are administered in combination to a subject.

Background Art

Single-strand break and double-strand break are known as types of DNA damage, each of which has a repair mechanism. If the type of DNA damage is single-strand break, it will be repaired through base excision repair predominantly by PARP (poly[adenosine-5'-diphosphate (ADP)-ribose]polymerase) acting thereon. If the type of DNA damage is double-strand break, it will be repaired through homologous recombination repair predominantly by BRCA, ATM, RAD51, and the like, acting thereon (Non-Patent Reference 1).

PARP inhibitors are drugs that have the function of inhibiting PARP (particularly PARP-1 and PARP-2), and thus preventing single-strand break repair. Some cancers including breast cancer and ovarian cancer are known to have an abnormality in double-strand break repair, and PARP inhibitors have been revealed to have antitumor effects due to synthetic lethality against these cancers (Non-Patent References 2 to 5).

Known PARP inhibitors include olaparib (Non-Patent Reference 6), rucaparib (Non-Patent Reference 7), niraparib (Non-Patent Reference 8), and talazoparib (Non-Patent Reference 9).

Combination of a PARP inhibitor with another anticancer agent is also known to provide a similar effect to synthetic lethality (Non-Patent Reference 10). For example, combination of a PARP inhibitor and a topoisomerase I inhibitor is known to show an efficacy even against cancer having no abnormality in double-strand break repair (Non-Patent References 11 to 16). However, such a combination has been reported, in a clinical study, to have a problem in the balance of tolerance and efficacy (Non-Patent Reference 17), and has not been established as the standard therapy yet.

An antibody-drug conjugate (ADC) having a drug with cytotoxicity conjugated to an antibody capable of binding to an antigen expressed on the surface of cancer cells and cellular internalization, can deliver the drug selectively to cancer cells and can thus be expected to cause accumulation of the drug within cancer cells and to kill the cancer cells (Non-Patent References 18 to 22).

As one such antibody-drug conjugate, an antibody-drug conjugate comprising an antibody and a derivative of exatecan, which is a topoisomerase I inhibitor, as its components is known (Patent References 1 to 9, Non-Patent References 23 to 27).

None of the references describes any test result showing a combined effect of the foregoing antibody-drug conjugate and a PARP inhibitor, or any scientific basis for suggesting such a test result.

Citation List

Patent Literature

Patent Reference 1: International Publication No. WO 2014/057687
Patent Reference 2: International Publication No. WO 2014/061277
Patent Reference 3: International Publication No. WO 2015/098099
Patent Reference 4: International Publication No. WO 2015/115091
Patent Reference 5: International Publication No. WO 2015/146132
Patent Reference 6: International Publication No. WO 2015/155976
Patent Reference 7: International Publication No. WO 2015/155998
Patent Reference 8: International Publication No. WO 2018/135501
Patent Reference 9: International Publication No. WO 2018/212136


Non-Patent Literature

Non-Patent Reference 1: Lord CJ, et al., Nature (2012) 481, 287-294.

Non-Patent Reference 2: Benafif S, et al., Onco. Targets Ther. (2015) 8, 519-528.
Non-Patent Reference 3: Fong PC, et al., N. Engl. J. Med. (2009) 361, 123-134.
Non-Patent Reference 4: Fong PC, et al., J. Clin. Oncol. (2010) 28, 2512-2519.
Non-Patent Reference 5: Gelmon KA, et al., Lancet Oncol. (2011) 12, 852-861.
Non-Patent Reference 6: Menear KA, et al., J. Med. Chem. (2008) 51, 6581-6591.
Non-Patent Reference 7: Gillmore AT, et al., Org. Process Res. Dev. (2012) 16, 1897-1904.
Non-Patent Reference 8: Jones P, et al., J. Med. Chem. (2009) 52, 7170-7185.
Non-Patent Reference 9: Shen Y, et al., Clin. Cancer Res. (2013) 19(18), 5003-15.
Non-Patent Reference 10: Oza AM, et al., Lancet Oncol. (2015) 16, 87-97.
Non-Patent Reference 11: Tahara M, et al., Mol. Cancer Ther. (2014) 13, 1170-1180.
Non-Patent Reference 12: Miknyoczki S, et al., Mol. Cancer Ther. (2007) 6, 2290-2302.
Non-Patent Reference 13: Calabrese CR, et al., J. Natl. Cancer Inst. (2004) 96, 56-67.
Non-Patent Reference 14: Smith LM, et al., Clin. cancer res. (2005) 11, 8449-8457.
Non-Patent Reference 15: Genther Williams SM, et al., Cancer Cell Int. (2015) 15:14.
Non-Patent Reference 16: Cardillo TM, et al., Clin. cancer res. (2017) 13, 3405-3415.
Non-Patent Reference 17: Chen EX, Invest. New Drugs (2016) 4, 450-457.
Non-Patent Reference 18: Ducry, L., et al., Bioconjugate Chem. (2010) 21, 5-13.
Non-Patent Reference 19: Alley, S. C., et al., Current Opinion in Chemical Biology (2010) 14, 529-537.
Non-Patent Reference 20: Damle N. K. Expert Opin. Biol. Ther. (2004) 4, 1445-1452.
Non-Patent Reference 21: Senter P. D., et al., Nature Biotechnology (2012) 30, 631-637.
Non-Patent Reference 22: Burris HA et al., J. Clin. Oncol. (2011) 29(4): 398-405.
Non-Patent Reference 23: Ogitani Y. et al., Clinical Cancer Research (2016) 22 (20), 5097-5108.
Non-Patent Reference 24: Ogitani Y. et al., Cancer Science (2016) 107, 1039-1046.
Non-Patent Reference 25: Doi T, et al., Lancet Oncol. (2017) 18, 1512-22.
Non-Patent Reference 26: Takegawa N, et al., Int. J. Cancer (2017) 141, 1682-1689.
Non-Patent Reference 27: Yonesaka K, et al., Int. Oncogene (2018) 141, 1682-1689 (2017).

## Summary of Invention

### Technical Problem

The antibody-drug conjugates used in the present invention (antibody-drug conjugates containing an exatecan derivative as a component) have been confirmed to exert a superior antitumor effect even as a single agent. However, there has been a need for obtaining a method of treatment which can suppress growth of cancer cells in multiple manners and exert a further superior antitumor effect by using the antibody-drug conjugate in combination with another anticancer agent having a different mechanism of action.

An object of the present invention is to provide a pharmaceutical composition, wherein a specific antibody-drug conjugate and a PARP inhibitor are administered in combination, and/or a method of treatment, wherein a specific antibody-drug conjugate and a PARP inhibitor are administered in combination to a subject.

### Solution to Problem

As a result of diligent studies in order to solve the above problems, the present inventors have found that combined administration of a specific antibody-drug conjugate and a PARP inhibitor exhibits a superior combined effect, and thereby completed the present invention.

Thus, the present invention provides the following [1] to [368].

[1] A pharmaceutical composition, wherein

an antibody-drug conjugate and a PARP inhibitor are administered in combination, and
the antibody-drug conjugate is an antibody-drug conjugate in which a drug-linker represented by the following formula:

[Formula 1]

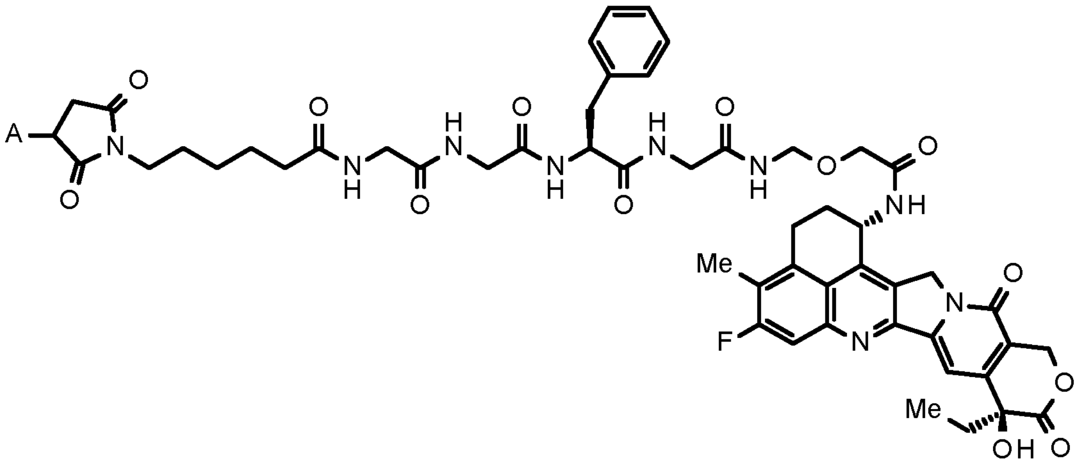

wherein A represents a connecting position to an antibody, is conjugated to the antibody via a thioether bond.

[2] The pharmaceutical composition according to [1], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.
[3] The pharmaceutical composition according to [2], wherein the antibody in the antibody-drug conjugate is an anti-HER2 antibody.
[4] The pharmaceutical composition according to [3], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[5] The pharmaceutical composition according to [3], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[6] The pharmaceutical composition according to [3], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[7] The pharmaceutical composition according to [3], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.
[8] The pharmaceutical composition according to any one of [3] to [7], wherein the average number of units of the drug-linker conjugated per antibody molecule in the antibody-drug conjugate is in the range of from 7 to 8.
[9] The pharmaceutical composition according to [2], wherein the antibody in the antibody-drug conjugate is an anti-HER3 antibody.
[10] The pharmaceutical composition according to [9], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[11] The pharmaceutical composition according to [10], wherein the anti-HER3 antibody lacks a lysine residue at the carboxyl terminus of the heavy chain.
[12] The pharmaceutical composition according to any one of [9] to [11], wherein the average number of units of the drug-linker conjugated per antibody molecule in the antibody-drug conjugate is in the range of from 7 to 8.
[13] The pharmaceutical composition according to [2], wherein the antibody in the antibody-drug conjugate is an anti-TROP2 antibody.
[14] The pharmaceutical composition according to [13], wherein the anti-TROP2 antibody is an antibody comprising

a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[15] The pharmaceutical composition according to [14], wherein the anti-TROP2 antibody lacks a lysine residue at the carboxyl terminus of the heavy chain.
[16] The pharmaceutical composition according to any one of [13] to [15], wherein the average number of units of the drug-linker conjugated per antibody molecule in the antibody-drug conjugate is in the range of from 3.5 to 4.5.
[17] The pharmaceutical composition according to [2], wherein the antibody in the antibody-drug conjugate is an anti-B7-H3 antibody.
[18] The pharmaceutical composition according to [17], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[19] The pharmaceutical composition according to [18], wherein the anti-B7-H3 antibody lacks a lysine residue at the carboxyl terminus of the heavy chain.
[20] The pharmaceutical composition according to any one of [17] to [19], wherein the average number of units of the drug-linker conjugated per antibody molecule in the antibody-drug conjugate is in the range of from 3.5 to 4.5.
[21] The pharmaceutical composition according to [2], wherein the antibody in the antibody-drug conjugate is an anti-GPR20 antibody.
[22] The pharmaceutical composition according to [21], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[23] The pharmaceutical composition according to [22], wherein the anti-GPR20 antibody lacks a lysine residue at the carboxyl terminus of the heavy chain.
[24] The pharmaceutical composition according to any one of [21] to [23], wherein the average number of units of the drug-linker conjugated per antibody molecule in the antibody-drug conjugate is in the range of from 7 to 8.
[25] The pharmaceutical composition according to [2], wherein the antibody in the antibody-drug conjugate is an anti-CDH6 antibody.
[26] The pharmaceutical composition according to [25], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[27] The pharmaceutical composition according to [26], wherein the anti-CDH6 antibody lacks a lysine residue at the carboxyl terminus of the heavy chain.
[28] The pharmaceutical composition according to any one of [25] to [27], wherein the average number of units of the drug-linker conjugated per antibody molecule in the antibody-drug conjugate is in the range of from 7 to 8.
[29] The pharmaceutical composition according to any one of [1] to [28], wherein the PARP inhibitor is olaparib, rucaparib, niraparib, talazoparib, or veliparib, or a pharmacologically acceptable salt thereof.
[30] The pharmaceutical composition according to [29], wherein the PARP inhibitor is olaparib or a pharmacologically acceptable salt thereof.
[31] The pharmaceutical composition according to [29], wherein the PARP inhibitor is rucaparib or a pharmacologically acceptable salt thereof.
[32] The pharmaceutical composition according to [29], wherein the PARP inhibitor is niraparib or a pharmacologically acceptable salt thereof.
[33] The pharmaceutical composition according to [29], wherein the PARP inhibitor is talazoparib or a pharmacologically acceptable salt thereof.
[34] The pharmaceutical composition according to [29], wherein the PARP inhibitor is veliparib or a pharmacologically acceptable salt thereof.
[35] The pharmaceutical composition according to any one of [1] to [34], wherein the antibody-drug conjugate and the PARP inhibitor are separately contained as active components in different formulations, and are administered simultaneously or at different times.
[36] The pharmaceutical composition according to any one of [1] to [35], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.
[37] The pharmaceutical composition according to [36], wherein the pharmaceutical composition is for use in treating breast cancer.
[38] The pharmaceutical composition according to [37], wherein the pharmaceutical composition is for use in treating HER2 low-expressing breast cancer.
[39] The pharmaceutical composition according to [36], wherein the pharmaceutical composition is for use in treating

gastric cancer.

[40] The pharmaceutical composition according to [36], wherein the pharmaceutical composition is for use in treating ovarian cancer.

[41] The pharmaceutical composition according to [36], wherein the pharmaceutical composition is for use in treating lung cancer.

[42] The pharmaceutical composition according to [36], wherein the pharmaceutical composition is for use in treating pancreatic cancer.

[43] A method of treatment, comprising administering an antibody-drug conjugate and a PARP inhibitor in combination to a subject in need of the treatment, wherein the antibody-drug conjugate is an antibody-drug conjugate in which a drug-linker represented by the following formula:

[Formula 2]

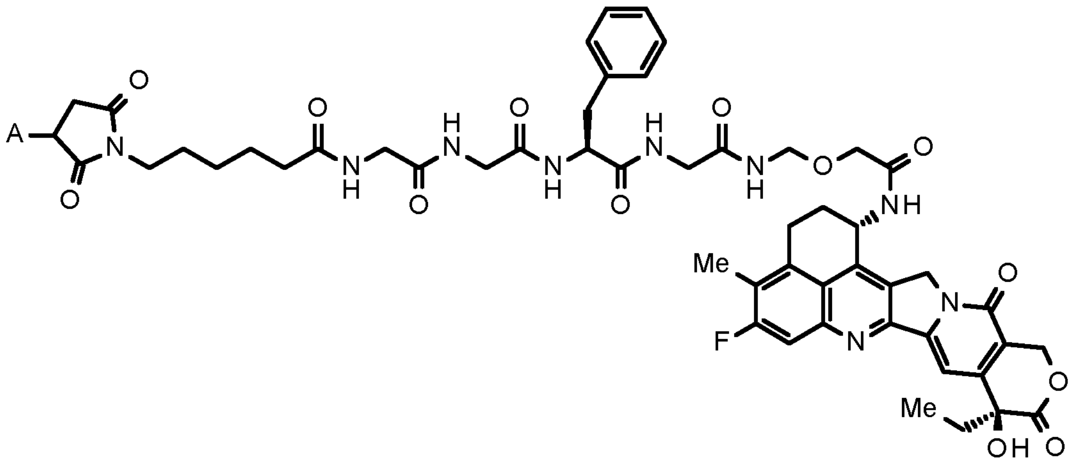

wherein A represents a connecting position to an antibody, is conjugated to the antibody via a thioether bond.

[44] The method of treatment according to [43], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[45] The method of treatment according to [44], wherein the antibody in the antibody-drug conjugate is an anti-HER2 antibody.

[46] The method of treatment according to [45], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[47] The method of treatment according to [45], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[48] The method of treatment according to [45], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[49] The method of treatment according to [45], wherein the anti-HER2 antibody is an antibody comprising a heavy chain consisting of an amino acid sequence consisting of amino acid residues 1 of 449 of SEQ ID NO: 1 and a light chain consisting of an amino acid sequence represented by amino acid residues 1 to 214 of SEQ ID NO: 2.

[50] The method of treatment according to any one of [45] to [49], wherein the average number of units of the drug-linker conjugated per antibody molecule in the antibody-drug conjugate is in the range of from 7 to 8.

[51] The method of treatment according to [44], wherein the antibody in the antibody-drug conjugate is an anti-HER3 antibody.

[52] The method of treatment according to [51], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[53] The method of treatment according to [52], wherein the anti-HER3 antibody lacks a lysine residue at the carboxyl terminus of the heavy chain.
[54] The method of treatment according to any one of [51] to [53], wherein the average number of units of the drug-linker conjugated per antibody molecule in the antibody-drug conjugate is in the range of from 7 to 8.
[55] The method of treatment according to [44], wherein the antibody in the antibody-drug conjugate is an anti-TROP2 antibody.
[56] The method of treatment according to [55], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[57] The method of treatment according to [56], wherein the anti-TROP2 antibody lacks a lysine residue at the carboxyl terminus of the heavy chain.
[58] The method of treatment according to any one of [55] to [57], wherein the average number of units of the drug-linker conjugated per antibody molecule in the antibody-drug conjugate is in the range of from 3.5 to 4.5.
[59] The method of treatment according to [44], wherein the antibody in the antibody-drug conjugate is an anti-B7-H3 antibody.
[60] The method of treatment according to [59], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[61] The method of treatment according to [60], wherein the anti-B7-H3 antibody lacks a lysine residue at the carboxyl terminus of the heavy chain.
[62] The method of treatment according to any one of [59] to [61], wherein the average number of units of the drug-linker conjugated per antibody molecule in the antibody-drug conjugate is in the range of from 3.5 to 4.5.
[63] The method of treatment according to [44], wherein the antibody in the antibody-drug conjugate is an anti-GPR20 antibody.
[64] The method of treatment according to [63], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[65] The method of treatment according to [64], wherein the anti-GPR20 antibody lacks a lysine residue at the carboxyl terminus of the heavy chain.
[66] The method of treatment according to any one of [63] to [65], wherein the average number of units of the drug-linker conjugated per antibody molecule in the antibody-drug conjugate is in the range of from 7 to 8.
[67] The method of treatment according to [44], wherein the antibody in the antibody-drug conjugate is an anti-CDH6 antibody.
[68] The method of treatment according to [67], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[69] The method of treatment according to [68], wherein the anti-CDH6 antibody lacks a lysine residue at the carboxyl terminus of the heavy chain.
[70] The method of treatment according to any one of [67] to [69], wherein the average number of units of the drug-linker conjugated per antibody molecule in the antibody-drug conjugate is in the range of from 7 to 8.
[71] The method of treatment according to any one of [43] to [70], wherein the PARP inhibitor is olaparib, rucaparib, niraparib, talazoparib, or veliparib, or a pharmacologically acceptable salt thereof.
[72] The method of treatment according to [71], wherein the PARP inhibitor is olaparib or a pharmacologically acceptable salt thereof.
[73] The method of treatment according to [71], wherein the PARP inhibitor is rucaparib or a pharmacologically acceptable salt thereof.
[74] The method of treatment according to [71], wherein the PARP inhibitor is niraparib or a pharmacologically acceptable salt thereof.
[75] The method of treatment according to [71], wherein the PARP inhibitor is talazoparib or a pharmacologically acceptable salt thereof.
[76] The method of treatment according to [71], wherein the PARP inhibitor is veliparib or a pharmacologically acceptable salt thereof.
[77] The method of treatment according to any one of [43] to [76], wherein the antibody-drug conjugate and the PARP inhibitor are separately contained as active components in different formulations, and administered simultaneously or at different times.

[78] The method of treatment according to any one of [43] to [77], wherein the method of treatment is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.
[79] The method of treatment according to [78], wherein the method of treatment is for treating breast cancer.
[80] The method of treatment according to [79], wherein the method of treatment is for treating HER2 low-expressing breast cancer.
[81] The method of treatment according to [78], wherein the method of treatment is for treating gastric cancer.
[82] The method of treatment according to [78], wherein the method of treatment is for treating ovarian cancer.
[83] The method of treatment according to [78], wherein the method of treatment is for treating lung cancer.
[84] The method of treatment according to [78], wherein the method of treatment is for treating pancreatic cancer.
[85] An antibody-drug conjugate for use in treating a disease through being administered in combination with a PARP inhibitor, wherein a drug-linker represented by the following formula:

[Formula 3]

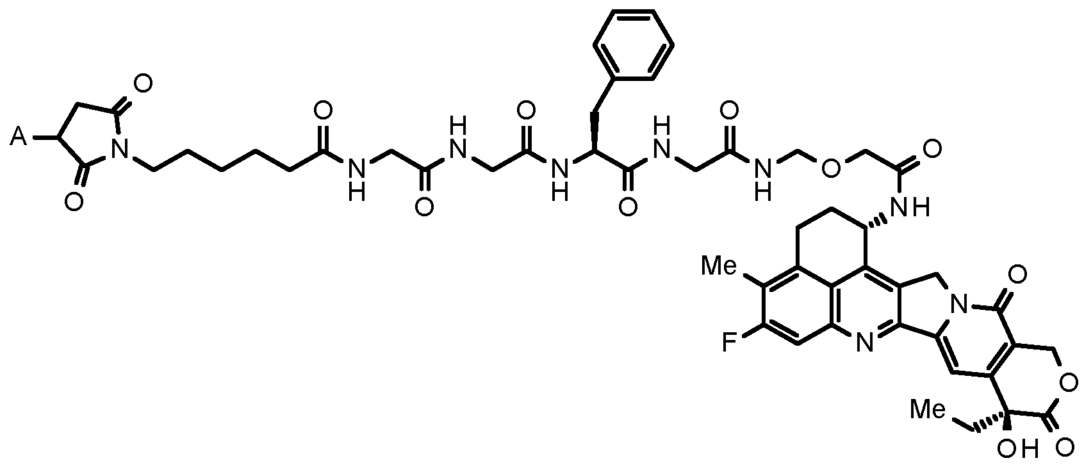

wherein A represents a connecting position to an antibody, is conjugated to the antibody via a thioether bond in the antibody-drug conjugate.
[86] The antibody-drug conjugate according to [85], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.
[87] The antibody-drug conjugate according to [86], wherein the antibody in the antibody-drug conjugate is an anti-HER2 antibody.
[88] The antibody-drug conjugate according to [87], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[89] The antibody-drug conjugate according to [87], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[90] The antibody-drug conjugate according to [87], wherein the anti-HER2 antibody is an antibody comprising a heavy chain consisting of an amino acid sequence represented by SEQ ID NO: 1 and a light chain consisting of an amino acid sequence represented by SEQ ID NO: 2.
[91] The antibody-drug conjugate according to [87], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[92] The antibody-drug conjugate according to any one of [87] to [91], wherein the average number of units of the drug-linker conjugated per antibody molecule in the antibody-drug conjugate is in the range of from 7 to 8.
[93] The antibody-drug conjugate according to [86], wherein the antibody in the antibody-drug conjugate is an anti-HER3 antibody.
[94] The antibody-drug conjugate according to [93], wherein the anti-HER3 antibody is an antibody comprising a heavy chain consisting of an amino acid sequence represented by SEQ ID NO: 3 and a light chain consisting of an amino acid sequence represented by SEQ ID NO: 4.
[95] The antibody-drug conjugate according to [94], wherein the anti-HER3 antibody lacks a lysine residue at the carboxyl terminus of the heavy chain.
[96] The antibody-drug conjugate according to any one of [93] to [95], wherein the average number of units of the drug-linker conjugated per antibody molecule in the antibody-drug conjugate is in the range of from 7 to 8.
[97] The antibody-drug conjugate according to [86], wherein the antibody in the antibody-drug conjugate is an anti-TROP2 antibody.
[98] The antibody-drug conjugate according to [97], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.
[99] The antibody-drug conjugate according to [98], wherein the anti-TROP2 antibody lacks a lysine residue at the carboxyl terminus of the heavy chain.
[100] The antibody-drug conjugate according to any one of [97] to [99], wherein the average number of units of the drug-linker conjugated per antibody molecule in the antibody-drug conjugate is in the range of from 3.5 to 4.5.
[101] The antibody-drug conjugate according to [86], wherein the antibody in the antibody-drug conjugate is an anti-B7-H3 antibody.
[102] The antibody-drug conjugate according to [101], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.
[103] The antibody-drug conjugate according to [102], wherein the anti-B7-H3 antibody lacks a lysine residue at the carboxyl terminus of the heavy chain.
[104] The antibody-drug conjugate according to any one of [101] to [103], wherein the average number of units of the drug-linker conjugated per antibody molecule in the antibody-drug conjugate is in the range of from 3.5 to 4.5.
[105] The antibody-drug conjugate according to [86], wherein the antibody in the antibody-drug conjugate is an anti-GPR20 antibody.
[106] The antibody-drug conjugate according to [105], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.
[107] The antibody-drug conjugate according to [106], wherein the anti-GPR20 antibody lacks a lysine residue at the carboxyl terminus of the heavy chain.
[108] The antibody-drug conjugate according to any one of [105] to [107], wherein the average number of units of the drug-linker conjugated per antibody molecule in the antibody-drug conjugate is in the range of from 7 to 8.
[109] The antibody-drug conjugate according to [86], wherein the antibody in the antibody-drug conjugate is an anti-CDH6 antibody.
[110] The antibody-drug conjugate according to [109], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.
[111] The antibody-drug conjugate according to [110], wherein the anti-CDH6 antibody lacks a lysine residue at the carboxyl terminus of the heavy chain.
[112] The antibody-drug conjugate according to any one of [109] to [111], wherein the average number of units of the drug-linker conjugated per antibody molecule in the antibody-drug conjugate is in the range of from 7 to 8.
[113] The antibody-drug conjugate according to any one of [85] to [112], wherein the PARP inhibitor is olaparib, rucaparib, niraparib, talazoparib, or veliparib, or a pharmacologically acceptable salt thereof.
[114] The antibody-drug conjugate according to [113], wherein the PARP inhibitor is olaparib or a pharmacologically acceptable salt thereof.
[115] The antibody-drug conjugate according to [113], wherein the PARP inhibitor is rucaparib or a pharmacologically acceptable salt thereof.
[116] The antibody-drug conjugate according to [113], wherein the PARP inhibitor is niraparib or a pharmacologically acceptable salt thereof.
[117] The antibody-drug conjugate according to [113], wherein the PARP inhibitor is talazoparib or a pharmacologically acceptable salt thereof.
[118] The antibody-drug conjugate according to [113], wherein the PARP inhibitor is veliparib or a pharmacologically

acceptable salt thereof.

[119] The antibody-drug conjugate according to any one of [85] to [118], wherein the antibody-drug conjugate and the PARP inhibitor are separately contained as active components in different formulations, and are administered simultaneously or at different times.

[120] The antibody-drug conjugate according to any one of [85] to [119], wherein the antibody-drug conjugate is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

[121] The antibody-drug conjugate according to [120], wherein the antibody-drug conjugate is for use in treating breast cancer.

[122] The antibody-drug conjugate according to [121], wherein the antibody-drug conjugate is for use in treating HER2 low-expressing breast cancer.

[123] The antibody-drug conjugate according to [120], wherein the antibody-drug conjugate is for use in treating gastric cancer.

[124] The antibody-drug conjugate according to [120], wherein the antibody-drug conjugate is for use in treating ovarian cancer.

[125] The antibody-drug conjugate according to [120], wherein the antibody-drug conjugate is for use in treating lung cancer.

[126] The antibody-drug conjugate according to [120], wherein the antibody-drug conjugate is for use in treating pancreatic cancer.

[127] Use of an antibody-drug conjugate for the manufacture of a medicament for treating a disease through being administered in combination with a PARP inhibitor, wherein a drug-linker represented by the following formula:

[Formula 4]

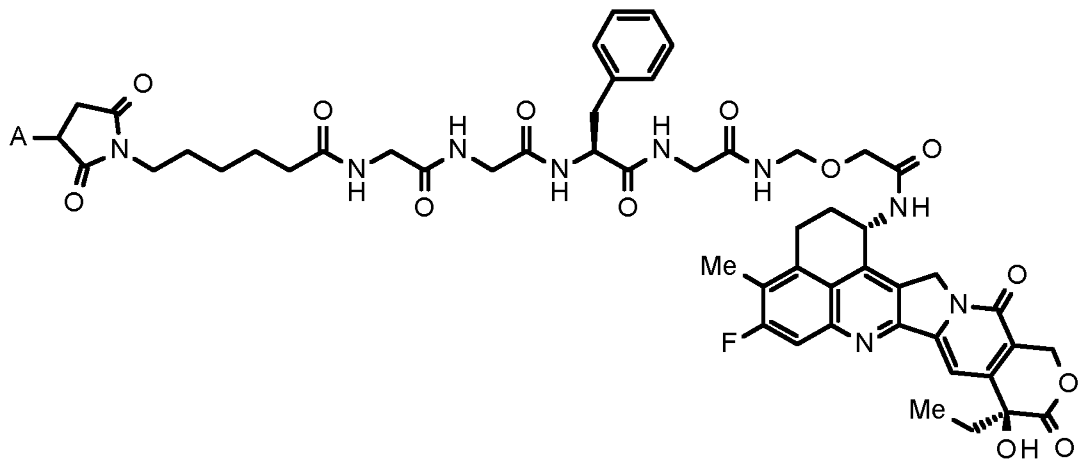

wherein A represents a connecting position to an antibody, is conjugated to the antibody via a thioether bond in the antibody-drug conjugate.

[128] The use according to [127], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[129] The use according to [128], wherein the antibody in the antibody-drug conjugate is an anti-HER2 antibody.

[130] The use according to [129], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[131] The use according to [129], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence

consisting of amino acid residues 1 to 107 of SEQ ID NO: 2.

[132] The use according to [129], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[133] The use according to [129], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[134] The use according to any one of [129] to [133], wherein the average number of units of the drug-linker conjugated per antibody molecule in the antibody-drug conjugate is in the range of from 7 to 8.

[135] The use method according to [128], wherein the antibody in the antibody-drug conjugate is an anti-HER3 antibody.

[136] The use according to [135], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[137] The use according to [136], wherein the anti-HER3 antibody lacks a lysine residue at the carboxyl terminus of the heavy chain.

[138] The use according to any one of [135] to [137], wherein the average number of units of the drug-linker conjugated per antibody molecule in the antibody-drug conjugate is in the range of from 7 to 8.

[139] The use according to [128], wherein the antibody in the antibody-drug conjugate is an anti-TROP2 antibody.

[140] The use according to [139], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[141] The use according to [140], wherein the anti-TROP2 antibody lacks a lysine residue at the carboxyl terminus of the heavy chain.

[142] The use according to any one of [139] to [141], wherein the average number of units of the drug-linker conjugated per antibody molecule in the antibody-drug conjugate is in the range of from 3.5 to 4.5.

[143] The use according to [128], wherein the antibody in the antibody-drug conjugate is an anti-B7-H3 antibody.

[144] The use according to [143], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[145] The use according to [144], wherein the anti-B7-H3 antibody lacks a lysine residue at the carboxyl terminus of the heavy chain.

[146] The use according to any one of [143] to [145], wherein the average number of units of the drug-linker conjugated per antibody molecule in the antibody-drug conjugate is in the range of from 3.5 to 4.5.

[147] The use according to [128], wherein the antibody in the antibody-drug conjugate is an anti-GPR20 antibody.

[148] The use according to [147], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

[149] The use according to [148], wherein the anti-GPR20 antibody lacks a lysine residue at the carboxyl terminus of the heavy chain.

[150] The use according to any one of [147] to [149], wherein the average number of units of the drug-linker conjugated per antibody molecule in the antibody-drug conjugate is in the range of from 7 to 8.

[151] The use according to [128], wherein the antibody in the antibody-drug conjugate is an anti-CDH6 antibody.

[152] The use according to [151], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

[153] The use according to [152], wherein the anti-CDH6 antibody lacks a lysine residue at the carboxyl terminus of the heavy chain.

[154] The use according to any one of [151] to [153], wherein the average number of units of the drug-linker conjugated per antibody molecule in the antibody-drug conjugate is in the range of from 7 to 8.

[155] The use according to any one of [127] to [154], wherein the PARP inhibitor is olaparib, rucaparib, niraparib, talazoparib, or veliparib, or a pharmacologically acceptable salt thereof.

[156] The use according to [155], wherein the PARP inhibitor is olaparib or a pharmacologically acceptable salt thereof.

[157] The use according to [155], wherein the PARP inhibitor is rucaparib or a pharmacologically acceptable salt thereof.

[158] The use according to [155], wherein the PARP inhibitor is niraparib or a pharmacologically acceptable salt thereof.

[159] The use according to [155], wherein the PARP inhibitor is talazoparib or a pharmacologically acceptable salt thereof.
[160] The use according to [155], wherein the PARP inhibitor is veliparib or a pharmacologically acceptable salt thereof.
[161] The use according to any one of [127] to [160], wherein the antibody-drug conjugate and the PARP inhibitor are separately contained as active components in different formulations, and are administered simultaneously or at different times.
[162] The use according to any one of [127] to [161], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.
[163] The use according to [162], wherein the use is for treating breast cancer.
[164] The use according to [163], wherein the use is for treating HER2 low-expressing breast cancer.
[165] The use according to [162], wherein the use is for treating gastric cancer.
[166] The use according to [162], wherein the use is for treating ovarian cancer.
[167] The use according to [162], wherein the use is for treating lung cancer.
[168] The use according to [162], wherein the use is for treating pancreatic cancer.
[169] A pharmaceutical composition wherein an antibody-drug conjugate and a PARP inhibitor are administered in combination, and the antibody-drug conjugate is an antibody-drug conjugate represented by the following formula:

[Formula 5]

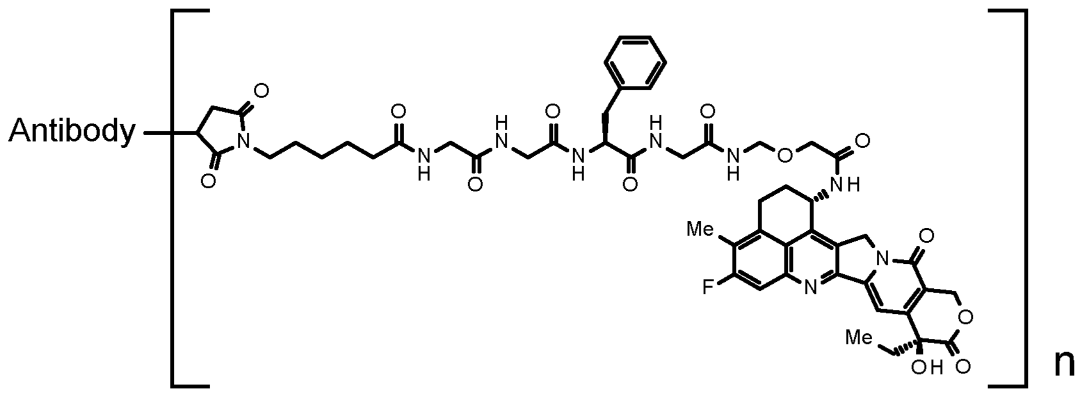

wherein a drug-linker is conjugated to an antibody via a thioether bond, and n indicates the average number of units of the drug-linker conjugated per antibody molecule.
[170] The pharmaceutical composition according to [169], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.
[171] The pharmaceutical composition according to [170], wherein the antibody in the antibody-drug conjugate is an anti-HER2 antibody.
[172] The pharmaceutical composition according to [171], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.
[173] The pharmaceutical composition according to [171], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.
[174] The pharmaceutical composition according to [171], wherein the anti-HER2 antibody is an antibody comprising a heavy chain consisting of an amino acid sequence represented by SEQ ID NO: 1 and a light chain consisting of

an amino acid sequence represented by SEQ ID NO: 2.

[175] The pharmaceutical composition according to [171], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[176] The pharmaceutical composition according to any one of [171] to [175], wherein the average number of units of the drug-linker conjugated per antibody molecule in the antibody-drug conjugate is in the range of from 7 to 8.

[177] The pharmaceutical composition according to [170], wherein the antibody in the antibody-drug conjugate is an anti-HER3 antibody.

[178] The pharmaceutical composition according to [177], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[179] The pharmaceutical composition according to [178], wherein the anti-HER3 antibody lacks a lysine residue at the carboxyl terminus of the heavy chain.

[180] The pharmaceutical composition according to any one of [177] to [179], wherein the average number of units of the drug-linker conjugated per antibody molecule in the antibody-drug conjugate is in the range of from 7 to 8.

[181] The pharmaceutical composition according to [170], wherein the antibody in the antibody-drug conjugate is an anti-TROP2 antibody.

[182] The pharmaceutical composition according to [181], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[183] The pharmaceutical composition according to [182], wherein the anti-TROP2 antibody lacks a lysine residue at the carboxyl terminus of the heavy chain.

[184] The pharmaceutical composition according to any one of [181] to [183], wherein the average number of units of the drug-linker conjugated per antibody molecule in the antibody-drug conjugate is in the range of from 3.5 to 4.5.

[185] The pharmaceutical composition according to [170], wherein the antibody in the antibody-drug conjugate is an anti-B7-H3 antibody.

[186] The pharmaceutical composition according to [185], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[187] The pharmaceutical composition according to [186], wherein the anti-B7-H3 antibody lacks a lysine residue at the carboxyl terminus of the heavy chain.

[188] The pharmaceutical composition according to any one of [185] to [187], wherein the average number of units of the drug-linker conjugated per antibody molecule in the antibody-drug conjugate is in the range of from 3.5 to 4.5.

[189] The pharmaceutical composition according to [170], wherein the antibody in the antibody-drug conjugate is an anti-GPR20 antibody.

[190] The pharmaceutical composition according to [189], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

[191] The pharmaceutical composition according to [190], wherein the anti-GPR20 antibody lacks a lysine residue at the carboxyl terminus of the heavy chain.

[192] The pharmaceutical composition according to any one of [189] to [191], wherein the average number of units of the drug-linker conjugated per antibody molecule in the antibody-drug conjugate is in the range of from 7 to 8.

[193] The pharmaceutical composition according to [170], wherein the antibody in the antibody-drug conjugate is an anti-CDH6 antibody.

[194] The pharmaceutical composition according to [193], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

[195] The pharmaceutical composition according to [194], wherein the anti-CDH6 antibody lacks a lysine residue at the carboxyl terminus of the heavy chain.

[196] The pharmaceutical composition according to any one of [193] to [195], wherein the average number of units of the drug-linker conjugated per antibody molecule in the antibody-drug conjugate is in the range of from 7 to 8.

[197] The pharmaceutical composition according to any one of [169] to [196], wherein the PARP inhibitor is olaparib, rucaparib, niraparib, talazoparib, or veliparib, or a pharmacologically acceptable salt thereof.

[198] The pharmaceutical composition according to [197], wherein the PARP inhibitor is olaparib or a pharmacologically acceptable salt thereof.

[199] The pharmaceutical composition according to [197], wherein the PARP inhibitor is rucaparib or a pharmacologically acceptable salt thereof.

[200] The pharmaceutical composition according to [197], wherein the PARP inhibitor is niraparib or a pharmacologically acceptable salt thereof.

[201] The pharmaceutical composition according to [197], wherein the PARP inhibitor is talazoparib or a pharmacologically acceptable salt thereof.

[202] The pharmaceutical composition according to [197], wherein the PARP inhibitor is veliparib or a pharmacologically acceptable salt thereof.

[203] The pharmaceutical composition according to any one of [169] to [202], wherein the antibody-drug conjugate and the PARP inhibitor are separately contained as active components in different formulations, and are administered simultaneously or at different times.

[204] The pharmaceutical composition according to any one of [169] to [203], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

[205] The pharmaceutical composition according to [204], wherein the pharmaceutical composition is for use in treating breast cancer.

[206] The pharmaceutical composition according to [205], wherein the pharmaceutical composition is for use in treating HER2 low-expressing breast cancer.

[207] The pharmaceutical composition according to [204], wherein the pharmaceutical composition is for use in treating gastric cancer.

[208] The pharmaceutical composition according to [204], wherein the pharmaceutical composition is for use in treating ovarian cancer.

[209] The pharmaceutical composition according to [204], wherein the pharmaceutical composition is for use in treating lung cancer.

[210] The pharmaceutical composition according to [204], wherein the pharmaceutical composition is for use in treating pancreatic cancer.

[211] A method of treatment, comprising administering an antibody-drug conjugate and a PARP inhibitor in combination to a subject in need of the treatment, wherein the antibody-drug conjugate is an antibody-drug conjugate represented by the following formula:

[Formula 6]

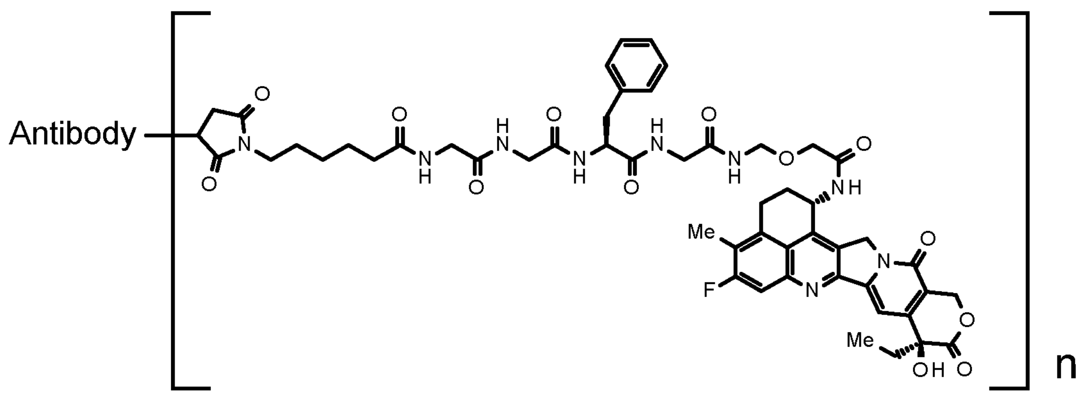

wherein a drug-linker is conjugated to an antibody via a thioether bond, and n indicates the average number of units of the drug-linker conjugated per antibody molecule.

[212] The method of treatment according to [211], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[213] The method of treatment according to [212], wherein the antibody in the antibody-drug conjugate is an anti-HER2 antibody.

[214] The method of treatment according to [213], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid

residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[215] The method of treatment according to [213], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[216] The method of treatment according to [213], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[217] The method of treatment according to [213], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[218] The method of treatment according to any one of [213] to [217], wherein the average number of units of the drug-linker conjugated per antibody molecule in the antibody-drug conjugate is in the range of from 7 to 8.

[219] The method of treatment according to [212], wherein the antibody in the antibody-drug conjugate is an anti-HER3 antibody.

[220] The method of treatment according to [219], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[221] The method of treatment according to [220], wherein the anti-HER3 antibody lacks a lysine residue at the carboxyl terminus of the heavy chain.

[222] The method of treatment according to any one of [219] to [221], wherein the average number of units of the drug-linker conjugated per antibody molecule in the antibody-drug conjugate is in the range of from 7 to 8.

[223] The method of treatment according to [212], wherein the antibody in the antibody-drug conjugate is an anti-TROP2 antibody.

[224] The method of treatment according to [223], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[225] The method of treatment according to [224], wherein the anti-TROP2 antibody lacks a lysine residue at the carboxyl terminus of the heavy chain.

[226] The method of treatment according to any one of [223] to [225], wherein the average number of units of the drug-linker conjugated per antibody molecule in the antibody-drug conjugate is in the range of from 3.5 to 4.5.

[227] The method of treatment according to [212], wherein the antibody in the antibody-drug conjugate is an anti-B7-H3 antibody.

[228] The method of treatment according to [227], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[229] The method of treatment according to [228], wherein the anti-B7-H3 antibody lacks a lysine residue at the carboxyl terminus of the heavy chain.

[230] The method of treatment according to any one of [227] to [229], wherein the average number of units of the drug-linker conjugated per antibody molecule in the antibody-drug conjugate is in the range of from 3.5 to 4.5.

[231] The method of treatment according to [212], wherein the antibody in the antibody-drug conjugate is an anti-GPR20 antibody.

[232] The method of treatment according to [231], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

[233] The method of treatment according to [232], wherein the anti-GPR20 antibody lacks a lysine residue at the carboxyl terminus of the heavy chain.

[234] The method of treatment according to any one of [231] to [233], wherein the average number of units of the drug-linker conjugated per antibody molecule in the antibody-drug conjugate is in the range of from 7 to 8.

[235] The method of treatment according to [212], wherein the antibody in the antibody-drug conjugate is an anti-CDH6 antibody.

[236] The method of treatment according to [235], wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

[237] The method of treatment according to [236], wherein the anti-CDH6 antibody lacks a lysine residue at the carboxyl terminus of the heavy chain.

[238] The method of treatment according to any one of [235] to [237], wherein the average number of units of the drug-linker conjugated per antibody molecule in the antibody-drug conjugate is in the range of from 7 to 8.

[239] The method of treatment according to any one of [211] to [238], wherein the PARP inhibitor is olaparib, rucaparib, niraparib, talazoparib, or veliparib, or a pharmacologically acceptable salt thereof.

[240] The method of treatment according to [239], wherein the PARP inhibitor is olaparib or a pharmacologically acceptable salt thereof.

[241] The method of treatment according to [239], wherein the PARP inhibitor is rucaparib or a pharmacologically acceptable salt thereof.

[242] The method of treatment according to [239], wherein the PARP inhibitor is niraparib or a pharmacologically acceptable salt thereof.

[243] The method of treatment according to [239], wherein the PARP inhibitor is talazoparib or a pharmacologically acceptable salt thereof.

[244] The pharmaceutical composition according to [239], wherein the PARP inhibitor is veliparib or a pharmacologically acceptable salt thereof.

[245] The method of treatment according to any one of [211] to [244], wherein the antibody-drug conjugate and the PARP inhibitor are separately contained as active components in different formulations, and are administered simultaneously or at different times.

[246] The method of treatment according to any one of [211] to [245], wherein the method of treatment is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

[247] The method of treatment according to [246], wherein the method of treatment is for treating breast cancer.

[248] The method of treatment according to [247], wherein the method of treatment is for treating HER2 low-expressing breast cancer.

[249] The method of treatment according to [246], wherein the method of treatment is for treating gastric cancer.

[250] The method of treatment according to [246], wherein the method of treatment is for treating ovarian cancer.

[251] The method of treatment according to [246], wherein the method of treatment is for treating lung cancer.

[252] The method of treatment according to [246], wherein the method of treatment is for treating pancreatic cancer.

[253] An antibody-drug conjugate for use in treating a disease through being administered in combination with a PARP inhibitor, wherein the antibody-drug conjugate is represented by the following formula:

[Formula 7]

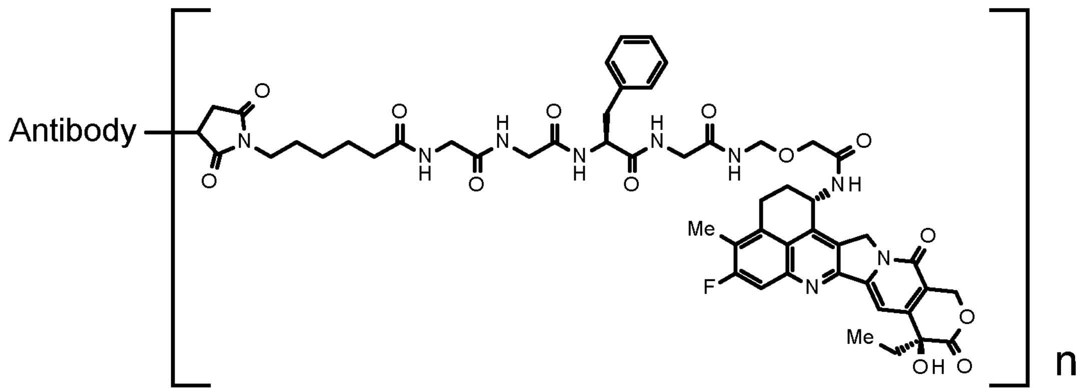

wherein a drug-linker is conjugated to an antibody via a thioether bond, and n indicates the average number of units of the drug-linker conjugated per antibody molecule.

[254] The antibody-drug conjugate according to [253], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[255] The antibody-drug conjugate according to [254], wherein the antibody in the antibody-drug conjugate is an anti-HER2 antibody.

[256] The antibody-drug conjugate according to [255], wherein the anti-HER2 antibody is an antibody comprising a heavy chain comprising CDRH1 consisting of an amino acid sequence consisting of amino acid residues 26 to 33

of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[257] The antibody-drug conjugate according to [255], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[258] The antibody-drug conjugate according to [255], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[259] The antibody-drug conjugate according to [255], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[260] The antibody-drug conjugate according to any one of [255] to [259], wherein the average number of units of the drug-linker conjugated per antibody molecule in the antibody-drug conjugate is in the range of from 7 to 8.

[261] The antibody-drug conjugate according to [254], wherein the antibody in the antibody-drug conjugate is an anti-HER3 antibody.

[262] The antibody-drug conjugate according to [261], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[263] The antibody-drug conjugate according to [262], wherein the anti-HER3 antibody lacks a lysine residue at the carboxyl terminus of the heavy chain.

[264] The antibody-drug conjugate according to any one of [261] to [263], wherein the average number of units of the drug-linker conjugated per antibody molecule in the antibody-drug conjugate is in the range of from 7 to 8.

[265] The antibody-drug conjugate according to [254], wherein the antibody in the antibody-drug conjugate is an anti-TROP2 antibody.

[266] The antibody-drug conjugate according to [265], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[267] The antibody-drug conjugate according to [266], wherein the anti-TROP2 antibody lacks a lysine residue at the carboxyl terminus of the heavy chain.

[268] The antibody-drug conjugate according to any one of [265] to [267], wherein the average number of units of the drug-linker conjugated per antibody molecule in the antibody-drug conjugate is in the range of from 3.5 to 4.5.

[269] The antibody-drug conjugate according to [254], wherein the antibody in the antibody-drug conjugate is an anti-B7-H3 antibody.

[270] The antibody-drug conjugate according to [269], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[271] The antibody-drug conjugate according to [270], wherein the anti-B7-H3 antibody lacks a lysine residue at the carboxyl terminus of the heavy chain.

[272] The antibody-drug conjugate according to any one of [269] to [271], wherein the average number of units of the drug-linker conjugated per antibody molecule in the antibody-drug conjugate is in the range of from 3.5 to 4.5.

[273] The antibody-drug conjugate according to [254], wherein the antibody in the antibody-drug conjugate is an anti-GPR20 antibody.

[274] The antibody-drug conjugate according to [273], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

[275] The antibody-drug conjugate according to [274], wherein the anti-GPR20 antibody lacks a lysine residue at the carboxyl terminus of the heavy chain.

[276] The antibody-drug conjugate according to any one of [273] to [275], wherein the average number of units of the drug-linker conjugated per antibody molecule in the antibody-drug conjugate is in the range of from 7 to 8.

[277] The antibody-drug conjugate according to [254], wherein the antibody in the antibody-drug conjugate is an anti-CDH6 antibody.

[278] The antibody-drug conjugate according to [277], wherein the anti-CDH6 antibody is an antibody comprising a heavy chain consisting of an amino acid sequence consisting of amino acid residues 20 to 471 of SEQ ID NO: 11

and a light chain consisting of an amino acid sequence consisting of amino acid residues 21 to 233 of SEQ ID NO: 12.
[279] The antibody-drug conjugate according to [278], wherein the anti-CDH6 antibody lacks a lysine residue at the carboxyl terminus of the heavy chain.
[280] The antibody-drug conjugate according to any one of [277] to [279], wherein the average number of units of the drug-linker conjugated per antibody molecule in the antibody-drug conjugate is in the range of from 7 to 8.
[281] The antibody-drug conjugate according to any one of [253] to [280], wherein the PARP inhibitor is olaparib, rucaparib, niraparib, talazoparib, or veliparib, or a pharmacologically acceptable salt thereof.
[282] The antibody-drug conjugate according to [281], wherein the PARP inhibitor is olaparib or a pharmacologically acceptable salt thereof.
[283] The antibody-drug conjugate according to [281], wherein the PARP inhibitor is rucaparib or a pharmacologically acceptable salt thereof.
[284] The antibody-drug conjugate according to [281], wherein the PARP inhibitor is niraparib or a pharmacologically acceptable salt thereof.
[285] The antibody-drug conjugate according to [281], wherein the PARP inhibitor is talazoparib or a pharmacologically acceptable salt thereof.
[286] The antibody-drug conjugate according to [281], wherein the PARP inhibitor is veliparib or a pharmacologically acceptable salt thereof.
[287] The antibody-drug conjugate according to any one of [253] to [286], wherein the antibody-drug conjugate and the PARP inhibitor are separately contained as active components in different formulations, and are administered simultaneously or at different times.
[288] The antibody-drug conjugate according to any one of [253] to [287], wherein the antibody-drug conjugate is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.
[289] The antibody-drug conjugate according to [288], wherein the antibody-drug conjugate is for use in treating breast cancer.
[290] The antibody-drug conjugate according to [289], wherein the antibody-drug conjugate is for use in treating HER2 low-expressing breast cancer.
[291] The antibody-drug conjugate according to [288], wherein the antibody-drug conjugate is for use in treating gastric cancer.
[292] The antibody-drug conjugate according to [288], wherein the antibody-drug conjugate is for use in treating ovarian cancer.
[293] The antibody-drug conjugate according to [288], wherein the antibody-drug conjugate is for use in treating lung cancer.
[294] The antibody-drug conjugate according to [288], wherein the antibody-drug conjugate is for use in treating pancreatic cancer.
[295] Use of an antibody-drug conjugate for the manufacture of a medicament for treating a disease through being administered in combination with a PARP inhibitor, wherein the antibody-drug conjugate is represented by the following formula:

[Formula 8]

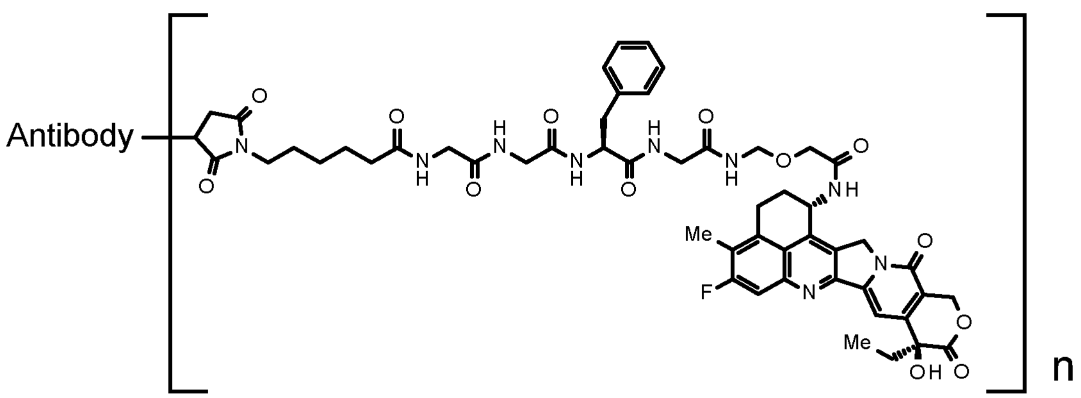

wherein a drug-linker is conjugated to an antibody via a thioether bond, and n indicates the average number of units

of the drug-linker conjugated per antibody molecule.

[296] The use according to [295], wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

[297] The use according to [296], wherein the antibody in the antibody-drug conjugate is an anti-HER2 antibody.

[298] The use according to [297], wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

[299] The use according to [297], wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

[300] The use according to [297], wherein the anti-HER2 antibody is an antibody comprising a heavy chain consisting of an amino acid sequence represented by SEQ ID NO: 1 and a light chain consisting of an amino acid sequence represented by SEQ ID NO: 2.

[301] The use according to [297], wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

[302] The use according to any one of [297] to [301], wherein the average number of units of the drug-linker conjugated per antibody molecule in the antibody-drug conjugate is in the range of from 7 to 8.

[303] The use according to [296], wherein the antibody in the antibody-drug conjugate is an anti-HER3 antibody.

[304] The use according to [303], wherein the anti-HER3 antibody is an antibody comprising a heavy chain consisting of an amino acid sequence represented by SEQ ID NO: 3 and a light chain consisting of an amino acid sequence represented by SEQ ID NO: 4.

[305] The use according to [304], wherein the anti-HER3 antibody lacks a lysine residue at the carboxyl terminus of the heavy chain.

[306] The use according to any one of [303] to [305], wherein the average number of units of the drug-linker conjugated per antibody molecule in the antibody-drug conjugate is in the range of from 7 to 8.

[307] The use according to [296], wherein the antibody in the antibody-drug conjugate is an anti-TROP2 antibody.

[308] The use according to [307], wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

[309] The use according to [308], wherein the anti-TROP2 antibody lacks a lysine residue at the carboxyl terminus of the heavy chain.

[310] The use according to any one of [307] to [309], wherein the average number of units of the drug-linker conjugated per antibody molecule in the antibody-drug conjugate is in the range of from 3.5 to 4.5.

[311] The use according to [296], wherein the antibody in the antibody-drug conjugate is an anti-B7-H3 antibody.

[312] The use according to [311], wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

[313] The use according to [312], wherein the anti-B7-H3 antibody lacks a lysine residue at the carboxyl terminus of the heavy chain.

[314] The use according to any one of [311] to [313], wherein the average number of units of the drug-linker conjugated per antibody molecule in the antibody-drug conjugate is in the range of from 3.5 to 4.5.

[315] The use according to [296], wherein the antibody in the antibody-drug conjugate is an anti-GPR20 antibody.

[316] The use according to [315], wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

[317] The use according to [316], wherein the anti-GPR20 antibody lacks a lysine residue at the carboxyl terminus of the heavy chain.

[318] The use according to any one of [315] to [317], wherein the average number of units of the drug-linker conjugated per antibody molecule in the antibody-drug conjugate is in the range of from 7 to 8.

[319] The use according to [296], wherein the antibody in the antibody-drug conjugate is an anti-CDH6 antibody.

[320] The use according to [319], wherein the anti-CDH6 antibody is an antibody comprising a heavy chain consisting

of an amino acid sequence consisting of amino acid residues 20 to 471 of SEQ ID NO: 11 and a light chain consisting of an amino acid sequence consisting of amino acid residues 21 to 233 of SEQ ID NO: 12.

[321] The use according to [320], wherein the anti-CDH6 antibody lacks a lysine residue at the carboxyl terminus of the heavy chain.

[322] The use according to any one of [319] to [321], wherein the average number of units of the drug-linker conjugated per antibody molecule in the antibody-drug conjugate is in the range of from 7 to 8.

[323] The use according to any one of [295] to [322], wherein the PARP inhibitor is olaparib, rucaparib, niraparib, talazoparib, or veliparib, or a pharmacologically acceptable salt thereof.

[324] The use according to [323], wherein the PARP inhibitor is olaparib or a pharmacologically acceptable salt thereof.

[325] The use according to [323], wherein the PARP inhibitor is rucaparib or a pharmacologically acceptable salt thereof.

[326] The use according to [323], wherein the PARP inhibitor is niraparib or a pharmacologically acceptable salt thereof.

[327] The use according to [323], wherein the PARP inhibitor is talazoparib or a pharmacologically acceptable salt thereof.

[328] The use according to [323], the PARP inhibitor is veliparib or a pharmacologically acceptable salt thereof.

[329] The use according to any one of [295] to [328], wherein the antibody-drug conjugate and the PARP inhibitor are separately contained as active components in different formulations, and are administered simultaneously or at different times.

[330] The use according to any one of [295] to [329], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

[331] The use according to [330], wherein the use is for treating breast cancer.

[332] The use according to [331], wherein the use is for treating HER2 low-expressing breast cancer.

[333] The use according to [330], wherein the use is for treating gastric cancer.

[334] The use according to [330], wherein the use is for treating ovarian cancer.

[335] The use according to [330], wherein the use is for treating lung cancer.

[336] The use according to [330], wherein the use is for treating pancreatic cancer.

[337] A pharmaceutical composition, wherein an anticancer agent and a PARP inhibitor are administered in combination, and the anticancer agent comprises a drug represented by the following formula:

[Formula 9]

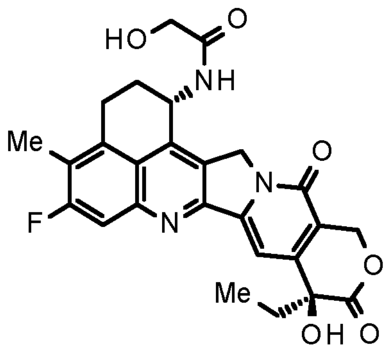

to be released in a tumor.

[338] The pharmaceutical composition according to [337], wherein the PARP inhibitor is olaparib, rucaparib, niraparib, talazoparib, or veliparib, or a pharmacologically acceptable salt thereof.

[339] The pharmaceutical composition according to [338], wherein the PARP inhibitor is olaparib or a pharmacologically acceptable salt thereof.

[340] The pharmaceutical composition according to [338], wherein the PARP inhibitor is rucaparib or a pharmacologically acceptable salt thereof.

[341] The pharmaceutical composition according to [338], wherein the PARP inhibitor is niraparib or a pharmacologically acceptable salt thereof.

[342] The pharmaceutical composition according to [338], wherein the PARP inhibitor is talazoparib or a pharmacologically acceptable salt thereof.

[343] The pharmaceutical composition according to [338], wherein the PARP inhibitor is veliparib or a pharmacologically acceptable salt thereof.

[344] The pharmaceutical composition according to any one of [337] to [343], wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

[345] A method of treatment, comprising administering an anticancer agent and a PARP inhibitor in combination to a subject in need of the treatment, wherein the anticancer agent comprises a drug represented by the following formula:

[Formula 10]

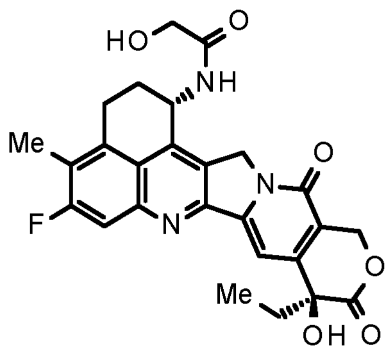

to be released in a tumor.

[346] The method of treatment according to [345], wherein the PARP inhibitor is olaparib, rucaparib, niraparib, talazoparib, or veliparib, or a pharmacologically acceptable salt thereof.

[347] The method of treatment according to [346], wherein the PARP inhibitor is olaparib or a pharmacologically acceptable salt thereof.

[348] The method of treatment according to [346], wherein the PARP inhibitor is rucaparib or a pharmacologically acceptable salt thereof.

[349] The method of treatment according to [346], wherein the PARP inhibitor is niraparib or a pharmacologically acceptable salt thereof.

[350] The method of treatment according to [346], wherein the PARP inhibitor is talazoparib or a pharmacologically acceptable salt thereof.

[351] The method of treatment according to [346], wherein the PARP inhibitor is veliparib or a pharmacologically acceptable salt thereof.

[352] The method of treatment according to any one of [345] to [351], wherein the method of treatment is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

[353] An anticancer agent for use in treating a disease through being administered in combination with a PARP inhibitor, wherein the anticancer agent comprises a drug represented by the following formula:

[Formula 11]

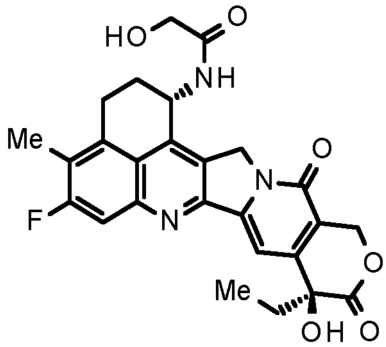

to be released in a tumor.

[354] The anticancer agent according to [353], wherein the PARP inhibitor is olaparib, rucaparib, niraparib, talazoparib, or veliparib, or a pharmacologically acceptable salt thereof.

[355] The anticancer agent according to [354], wherein the PARP inhibitor is olaparib or a pharmacologically acceptable salt thereof.

[356] The anticancer agent according to [354], wherein the PARP inhibitor is rucaparib or a pharmacologically acceptable salt thereof.

[357] The anticancer agent according to [354], wherein the PARP inhibitor is niraparib or a pharmacologically acceptable salt thereof.

[358] The anticancer agent according to [354], wherein the PARP inhibitor is talazoparib or a pharmacologically acceptable salt thereof.

[359] The anticancer agent according to [354], wherein the PARP inhibitor is veliparib or a pharmacologically acceptable salt thereof.

[360] The anticancer agent according to any one of [353] to [359], wherein the anticancer agent is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

[361] Use of an anticancer agent for the manufacture of a medicament for treating a disease through being administered in combination with a PARP inhibitor, wherein the anticancer agent comprises a drug represented by the following formula:

[Formula 12]

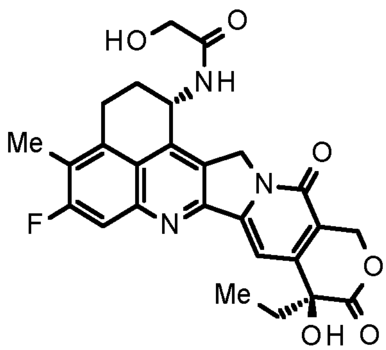

to be released in a tumor.

[362] The use according to [361], wherein the PARP inhibitor is olaparib, rucaparib, niraparib, talazoparib, or veliparib, or a pharmacologically acceptable salt thereof.

[363] The use according to [362], wherein the PARP inhibitor is olaparib or a pharmacologically acceptable salt thereof.

[364] The use according to [362], wherein the PARP inhibitor is rucaparib or a pharmacologically acceptable salt

thereof.
[365] The use according to [362], wherein the PARP inhibitor is niraparib or a pharmacologically acceptable salt thereof.
[366] The use according to [362], wherein the PARP inhibitor is talazoparib or a pharmacologically acceptable salt thereof.
[367] The use according to [362], wherein the PARP inhibitor is veliparib or a pharmacologically acceptable salt thereof.
[368] The use according to any one of [361] to [367], wherein the use is for treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

Advantageous Effects of Invention

The present invention provides a pharmaceutical composition, wherein a specific antibody-drug conjugate and a PARP inhibitor are administered in combination, and/or a method of treatment, wherein a specific antibody-drug conjugate and a PARP inhibitor are administered in combination to a subject.

Brief Description of Drawings

[Figure 1] Figure 1 is a diagram showing the amino acid sequence of a heavy chain of an anti-HER2 antibody (SEQ ID NO: 1).
[Figure 2] Figure 2 is a diagram showing the amino acid sequence of a light chain of an anti-HER2 antibody (SEQ ID NO: 2).
[Figure 3] Figure 3 is a diagram showing the amino acid sequence of a heavy chain of an anti-HER3 antibody (SEQ ID NO: 3).
[Figure 4] Figure 4 is a diagram showing the amino acid sequence of a light chain of an anti-HER3 antibody (SEQ ID NO: 4).
[Figure 5] Figure 5 is a diagram showing the amino acid sequence of a heavy chain of an anti-TROP2 antibody (SEQ ID NO: 5).
[Figure 6] Figure 6 is a diagram showing the amino acid sequence of a light chain of an anti-TROP2 antibody (SEQ ID NO: 6).
[Figure 7] Figure 7 is a diagram showing the amino acid sequence of a heavy chain of an anti-B7-H3 antibody (SEQ ID NO: 7).
[Figure 8] Figure 8 is a diagram showing the amino acid sequence of a light chain of an anti-B7-H3 antibody (SEQ ID NO: 8).
[Figure 9] Figure 9 is a diagram showing the amino acid sequence of a heavy chain of an anti-GPR20 antibody (SEQ ID NO: 9).
[Figure 10] Figure 10 is a diagram showing the amino acid sequence of a light chain of an anti-GPR20 antibody (SEQ ID NO: 10).
[Figure 11] Figure 11 is a diagram showing the amino acid sequence of a heavy chain of an anti-CDH6 antibody (SEQ ID NO: 11).
[Figure 12] Figure 12 is a diagram showing the amino acid sequence of a light chain of an anti-CDH6 antibody (SEQ ID NO: 12).
[Figure 13] Figure 13 is a diagram showing tumor growth suppressing effect in mice with subcutaneously transplanted KPL-4 cells in single administration groups of HER2-ADC (1) and olaparib respectively, and a combined administration group of HER2-ADC (1) and olaparib.
[Figure 14] Figure 14 is a diagram showing tumor growth suppressing effect in mice with subcutaneously transplanted KPL-4 cells in single administration groups of HER2-ADC (1) and talazoparib respectively, and a combined administration group of HER2-ADC (1) and talazoparib.
[Figure 15] Figure 15 is a diagram showing tumor growth suppressing effect in mice with subcutaneously transplanted KPL-4 cells in single administration groups of HER2-ADC (1) and niraparib respectively, and a combined administration group of HER2-ADC (1) and niraparib.
[Figure 16] Figure 16 is a diagram showing tumor growth suppressing effect in mice with subcutaneously transplanted KPL-4 cells in single administration groups of HER2-ADC (1) and rucaparib respectively, and a combined administration group of HER2-ADC (1) and rucaparib.

[Figure 17] Figure 17 is a diagram showing tumor growth suppressing effect in mice with subcutaneously transplanted JIMT-1 cells in single administration groups of HER2-ADC (1) and olaparib respectively, and a combined administration group of HER2-ADC (1) and olaparib.

[Figure 18] Figure 18 is a diagram showing tumor growth suppressing effect in mice with subcutaneously transplanted JIMT-1 cells in single administration groups of HER2-ADC (1) and talazoparib respectively, and a combined administration group of HER2-ADC (1) and talazoparib.

[Figure 19] Figure 19 is a diagram showing tumor growth suppressing effect in mice with subcutaneously transplanted JIMT-1 cells in single administration groups of HER2-ADC (1) and rucaparib respectively, and a combined administration group of HER2-ADC (1) and rucaparib.

[Figure 20] Figure 20 is a diagram showing tumor growth suppressing effect in mice with subcutaneously transplanted NCI-N87 cells in single administration groups of HER2-ADC (1) and olaparib respectively, and a combined administration group of HER2-ADC (1) and olaparib.

[Figure 21] Figure 21 is a diagram showing tumor growth suppressing effect in mice with subcutaneously transplanted NCI-N87 cells in single administration groups of HER2-ADC (1) and talazoparib respectively, and a combined administration group of HER2-ADC (1) and talazoparib.

[Figure 22] Figure 22 is a diagram showing tumor growth suppressing effect in mice with subcutaneously transplanted SK-OV-3 cells in single administration groups of HER2-ADC (1) and olaparib respectively, and a combined administration group of HER2-ADC (1) and olaparib.

[Figure 23] Figure 23 is a diagram showing tumor growth suppressing effect in mice with subcutaneously transplanted SK-OV-3 cells in single administration groups of HER2-ADC (1) and talazoparib respectively, and a combined administration group of HER2-ADC (1) and talazoparib.

[Figure 24] Figure 24 is a diagram showing tumor growth suppressing effect in mice with subcutaneously transplanted MX-1 cells in single administration groups of HER2-ADC (1) and olaparib respectively, and a combined administration group of HER2-ADC (1) and olaparib.

[Figure 25] Figure 25 is a diagram showing tumor growth suppressing effect in mice with subcutaneously transplanted MX-1 cells in single administration groups of HER2-ADC (1) and talazoparib respectively, and a combined administration group of HER2-ADC (1) and talazoparib.

[Figure 26] Figure 26 is a diagram showing tumor growth suppressing effect in mice with subcutaneously transplanted MX-1 cells in single administration groups of HER3-ADC (1) and olaparib respectively, and a combined administration group of HER3-ADC (1) and olaparib.

[Figure 27] Figure 27 is a diagram showing tumor growth suppressing effect in mice with subcutaneously transplanted MX-1 cells in single administration groups of HER3-ADC (1) and talazoparib respectively, and a combined administration group of HER3-ADC (1) and talazoparib.

[Figure 28] Figure 28 is a diagram showing tumor growth suppressing effect in mice with subcutaneously transplanted HCC70 cells in single administration groups of HER3-ADC (1) and olaparib respectively, and a combined administration group of HER3-ADC (1) and olaparib.

[Figure 29] Figure 29 is a diagram showing tumor growth suppressing effect in mice with subcutaneously transplanted HCC70 cells in single administration groups of HER3-ADC (1) and talazoparib respectively, and a combined administration group of HER3-ADC (1) and talazoparib.

[Figure 30] Figure 30 is a diagram showing tumor growth suppressing effect in mice with subcutaneously transplanted HCC1806 cells in single administration groups of TROP2-ADC (1) and olaparib respectively, and a combined administration group of TROP2-ADC (1) and olaparib.

[Figure 31] Figure 31 is a diagram showing tumor growth suppressing effect in mice with subcutaneously transplanted HCC1806 cells in single administration groups of TROP2-ADC (1) and rucaparib respectively, and a combined administration group of TROP2-ADC (1) and rucaparib.

[Figure 32] Figure 32 is a diagram showing tumor growth suppressing effect in mice with subcutaneously transplanted HCC1806 cells in single administration groups of TROP2-ADC (1) and talazoparib respectively, and a combined administration group of TROP2-ADC (1) and talazoparib.

[Figure 33] Figure 33 is a diagram showing tumor growth suppressing effect in mice with subcutaneously transplanted OV-90 cells in single administration groups of CDH6-ADC (1) and talazoparib respectively, and a combined administration group of CDH6-ADC (1) and talazoparib.

[Figure 34] Figure 34 is a diagram showing tumor growth suppressing effect in mice with subcutaneously transplanted OV-90 cells in single administration groups of CDH6-ADC (1) and rucaparib respectively, and a combined administration group of CDH6-ADC (1) and rucaparib.

[Figure 35] Figure 35 is a diagram showing tumor growth suppressing effect in mice with subcutaneously transplanted OV-90 cells in single administration groups of CDH6-ADC (1) and niraparib respectively, and a combined administration group of CDH6-ADC (1) and niraparib.

[Figure 36] Figure 36 is a diagram showing tumor growth suppressing effect in mice with subcutaneously transplanted OV-90 cells in single administration groups of CDH6-ADC (1) and veliparib respectively, and a combined administration group of CDH6-ADC (1) and veliparib.

[Figure 37] Figure 37 is a diagram showing tumor growth suppressing effect in mice with subcutaneously transplanted OV-90 cells in single administration groups of CDH6-ADC (1) and olaparib respectively, and a combined administration group of CDH6-ADC (1) and olaparib.

Description of Embodiments

Hereinafter, preferred modes for carrying out the present invention are described. The embodiments described below are given merely for illustrating one example of a typical embodiment of the present invention and are not intended to limit the scope of the present invention.

1. Antibody-drug conjugate

The antibody-drug conjugate used in the present invention is an antibody-drug conjugate in which a drug-linker represented by the following formula:

[Formula 13]

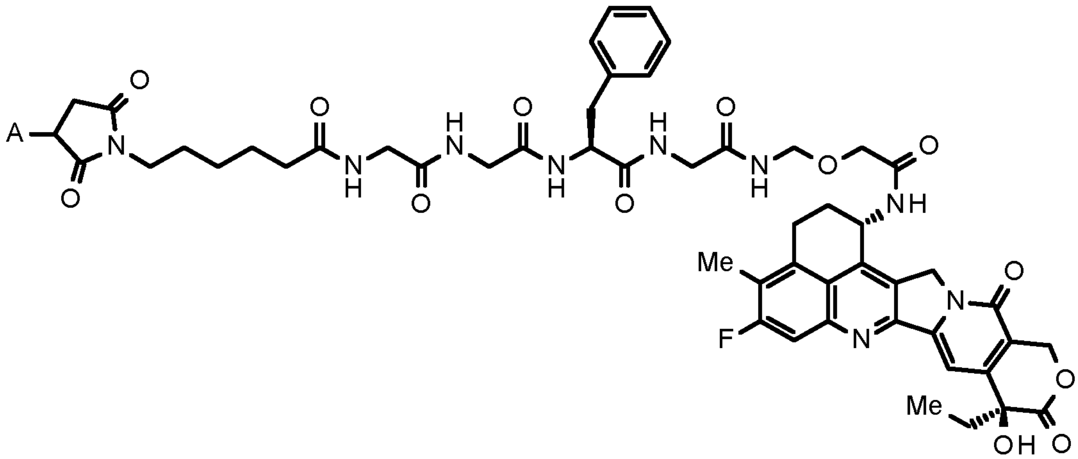

wherein A represents a connecting position to an antibody,
is conjugated to the antibody via a thioether bond.

In the present invention, the partial structure consisting of a linker and a drug in the antibody-drug conjugate is referred to as a "drug-linker". The drug-linker is connected to a thiol group (in other words, the sulfur atom of a cysteine residue) formed at an interchain disulfide bond site (two sites between heavy chains, and two sites between a heavy chain and a light chain) in the antibody.

The drug-linker of the present invention includes exatecan (IUPAC name: (1S,9S)-1-amino-9-ethyl-5-fluoro-1,2,3,9,12,15-hexahydro-9-hydroxy-4-methyl-10H,13H-benzo[de]pyrano[3',4':6,7]indolizino[1,2-b]quinolin-10,13-dione, (also expressed as chemical name: (1S,9S)-1-amino-9-ethyl-5-fluoro-2,3-dihydro-9-hydroxy-4-methyl-1H,12H-benzo[de]pyrano[3',4' :6,7]indolizino[1,2-b]quinolin-10,13(9H,15H)-dione)), which is a topoisomerase I inhibitor, as a component. Exatecan is a camptothecin derivative having an antitumor effect, represented by the following formula:

[Formula 14]

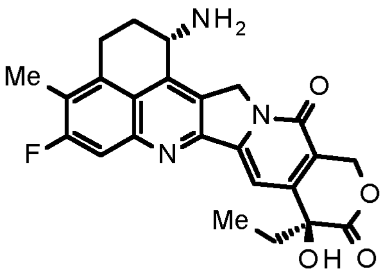

The antibody-drug conjugate used in the present invention can also be represented by the following formula:

[Formula 15]

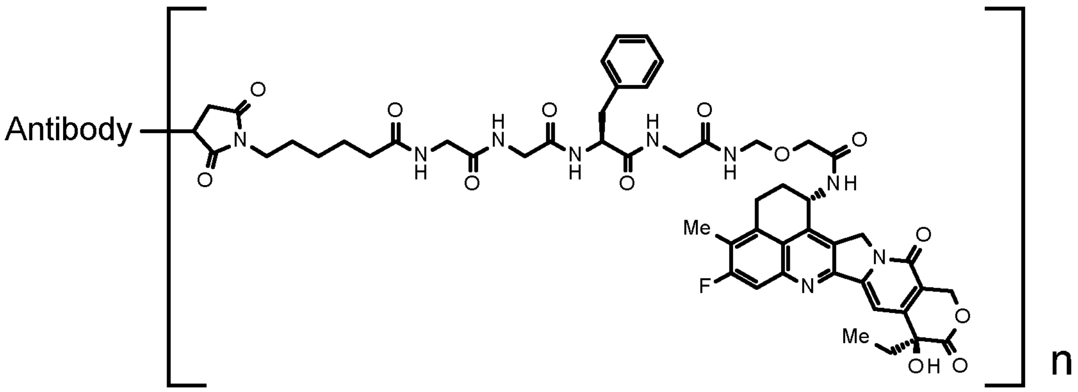

wherein, the drug-linker is conjugated to an antibody via a thioether bond. The meaning of n is the same as that of what is called the average number of conjugated drug molecules (DAR; Drug-to-Antibody Ratio), and indicates the average number of units of the drug-linker conjugated per antibody molecule.

After migrating into cancer cells, the antibody-drug conjugate used in the present invention is cleaved at the linker portion to release the compound represented by the following formula:

[Formula 16]

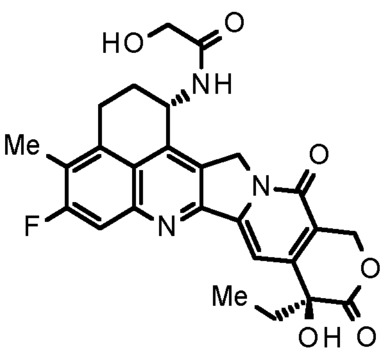

The aforementioned compound is inferred to be the original source of the antitumor activity of the antibody-drug conjugate used in the present invention, and has been confirmed to have a topoisomerase I inhibitory effect (Ogitani Y. et al., Clinical Cancer Research, 2016, Oct 15;22 (20) :5097-5108, Epub 2016 Mar 29.)

The antibody-drug conjugate used in the present invention is also known to have a bystander effect (Ogitani Y. et al., Cancer Science (2016) 107, 1039-1046).

The bystander effect is exerted through a process such that the antibody-drug conjugate used in the present invention is internalized in cancer cells expressing the target, and the aforementioned compound is released and then

exerts an antitumor effect also on cancer cells which are present therearound and not expressing the target.

The bystander effect is also exerted as an excellent antitumor effect when the antibody-drug conjugate according to the present invention is used in combination with a PARP inhibitor.

2. Antibody in the antibody-drug conjugate

The antibody in the antibody-drug conjugate used in the present invention may be derived from any species and is preferably an antibody derived from a human, a rat, a mouse, or a rabbit. In cases when the antibody is derived from species other than human species, it is preferably chimerized or humanized using a well known technique. The antibody of the present invention may be a polyclonal antibody or a monoclonal antibody and is preferably a monoclonal antibody.

The antibody in the antibody-drug conjugate used in the present invention is an antibody preferably having the characteristic of being able to target cancer cells, and is preferably an antibody possessing, for example, the property of being able to recognize a cancer cell, the property of being able to bind to a cancer cell, the property of being internalized in a cancer cell, and/or cytocidal activity against cancer cells.

The binding activity of the antibody against cancer cells can be confirmed using flow cytometry. The internalization of the antibody into tumor cells can be confirmed using (1) an assay of visualizing an antibody incorporated in cells under a fluorescence microscope using a secondary antibody (fluorescently labeled) binding to the therapeutic antibody (Cell Death and Differentiation (2008) 15, 751-761), (2) an assay of measuring a fluorescence intensity incorporated in cells using a secondary antibody (fluorescently labeled) binding to the therapeutic antibody (Molecular Biology of the Cell, Vol. 15, 5268-5282, December 2004), or (3) a Mab-ZAP assay using an immunotoxin binding to the therapeutic antibody wherein the toxin is released upon incorporation into cells to inhibit cell growth (Bio Techniques 28: 162-165, January 2000). As the immunotoxin, a recombinant complex protein of a diphtheria toxin catalytic domain and protein G may be used.

The antitumor activity of the antibody can be confirmed in vitro by determining inhibitory activity against cell growth. For example, a cancer cell line overexpressing a target protein for the antibody is cultured, and the antibody is added at varying concentrations into the culture system to determine inhibitory activity against focus formation, colony formation, and spheroid growth. The antitumor activity can be confirmed in vivo, for example, by administering the antibody to a nude mouse with a transplanted cancer cell line highly expressing the target protein, and determining changes in the cancer cells.

Since the compound conjugated in the antibody-drug conjugate exerts an antitumor effect, it is preferred but not essential that the antibody itself should have an antitumor effect. For the purpose of specifically and selectively exerting the cytotoxic activity of the antitumor compound against cancer cells, it is important and also preferred that the antibody should have the property of being internalized to migrate into cancer cells.

The antibody in the antibody-drug conjugate used in the present invention can be obtained by a procedure known in the art. For example, the antibody of the present invention can be obtained using a method usually carried out in the art, which involves immunizing animals with an antigenic polypeptide and collecting and purifying antibodies produced in vivo. The origin of the antigen is not limited to humans, and the animals may be immunized with an antigen derived from a non-human animal such as a mouse, a rat and the like. In this case, the cross-reactivity of antibodies binding to the obtained heterologous antigen with human antigens can be tested to screen for an antibody applicable to a human disease.

Alternatively, antibody-producing cells which produce antibodies against the antigen can be fused with myeloma cells according to a method known in the art (for example, Kohler and Milstein, Nature (1975) 256, p.495-497; Kennet, R. ed., Monoclonal Antibodies, p.365-367, Plenum Press, N.Y. (1980)), to establish hybridomas, from which monoclonal antibodies can in turn be obtained.

The antigen can be obtained by genetically engineering host cells to produce a gene encoding the antigenic protein. Specifically, vectors that permit expression of the antigen gene are prepared and transferred to host cells so that the gene is expressed. The antigen thus expressed can be purified. The antibody can also be obtained by a method of immunizing animals with the above-described genetically engineered antigen-expressing cells or a cell line expressing the antigen.

The antibody in the antibody-drug conjugate used in the present invention is preferably a recombinant antibody obtained by artificial modification for the purpose of decreasing heterologous antigenicity to humans such as a chimeric antibody or a humanized antibody, or is preferably an antibody having only the gene sequence of an antibody derived from a human, that is, a human antibody. These antibodies can be produced using a known method.

As the chimeric antibody, an antibody in which antibody variable and constant regions are derived from different species, for example, a chimeric antibody in which a mouse- or rat-derived antibody variable region is connected to a human-derived antibody constant region can be exemplified (Proc. Natl. Acad. Sci. USA, 81, 6851-6855, (1984)).

As the humanized antibody, an antibody obtained by integrating only the complementarity determining region

(CDR) of a heterologous antibody into a human-derived antibody (Nature (1986) 321, pp. 522-525), an antibody obtained by grafting a part of the amino acid residues of the framework of a heterologous antibody as well as the CDR sequence of the heterologous antibody to a human antibody by a CDR-grafting method (WO 90/07861), and an antibody humanized using a gene conversion mutagenesis strategy (U.S. Patent No. 5821337) can be exemplified.

As the human antibody, an antibody generated by using a human antibody-producing mouse having a human chromosome fragment including genes of a heavy chain and light chain of a human antibody (see Tomizuka, K. et al., Nature Genetics (1997) 16, p.133-143; Kuroiwa, Y. et. al., Nucl. Acids Res. (1998) 26, p.3447-3448; Yoshida, H. et. al., Animal Cell Technology: Basic and Applied Aspects vol.10, p.69-73 (Kitagawa, Y., Matsuda, T. and Iijima, S. eds.), Kluwer Academic Publishers, 1999; Tomizuka, K. et. al., Proc. Natl. Acad. Sci. USA (2000) 97, p.722-727, etc.) can be exemplified. As an alternative, an antibody obtained by phage display, the antibody being selected from a human antibody library (see Wormstone, I. M. et. al, Investigative Ophthalmology & Visual Science. (2002) 43 (7), p.2301-2308; Carmen, S. et. al., Briefings in Functional Genomics and Proteomics (2002), 1 (2), p.189-203; Siriwardena, D. et. al., Ophthalmology (2002) 109 (3), p.427-431, etc.) can be exemplified.

In the antibody in the antibody-drug conjugate used in present invention, modified variants of the antibody are also included. The modified variant refers to a variant obtained by subjecting the antibody according to the present invention to chemical or biological modification. Examples of the chemically modified variant include variants including a linkage of a chemical moiety to an amino acid skeleton, variants including a linkage of a chemical moiety to an N-linked or O-linked carbohydrate chain, etc. Examples of the biologically modified variant include variants obtained by post-translational modification (such as N-linked or O-linked glycosylation, N- or C-terminal processing, deamidation, isomerization of aspartic acid, or oxidation of methionine), and variants in which a methionine residue has been added to the N terminus by being expressed in a prokaryotic host cell. Further, an antibody labeled so as to enable the detection or isolation of the antibody or an antigen according to the present invention, for example, an enzyme-labeled antibody, a fluorescence-labeled antibody, and an affinity-labeled antibody are also included in the meaning of the modified variant. Such a modified variant of the antibody according to the present invention is useful for improving the stability and blood retention of the antibody, reducing the antigenicity thereof, detecting or isolating an antibody or an antigen, and so on.

Further, by regulating the modification of a glycan which is linked to the antibody according to the present invention (glycosylation, defucosylation, etc.), it is possible to enhance antibody-dependent cellular cytotoxic activity. As the technique for regulating the modification of a glycan of antibodies, International Publication No. WO 99/54342, International Publication No. WO 00/61739, International Publication No. WO 02/31140, International Publication No. WO 2007/133855, International Publication No. WO 2013/120066, etc. are known. However, the technique is not limited thereto. In the antibody according to the present invention, antibodies in which the modification of a glycan is regulated are also included.

It is known that a lysine residue at the carboxyl terminus of the heavy chain of an antibody produced in a cultured mammalian cell is deleted (Journal of Chromatography A, 705: 129-134 (1995)), and it is also known that two amino acid residues (glycine and lysine) at the carboxyl terminus of the heavy chain of an antibody produced in a cultured mammalian cell are deleted and a proline residue newly located at the carboxyl terminus is amidated (Analytical Biochemistry, 360: 75-83 (2007)). However, such deletion and modification of the heavy chain sequence do not affect the antigen-binding affinity and the effector function (complement activation, antibody-dependent cellular cytotoxicity, etc.) of the antibody. Therefore, in the antibody according to the present invention, antibodies subjected to such modification and functional fragments of the antibody are also included, and deletion variants in which one or two amino acids have been deleted at the carboxyl terminus of the heavy chain, variants obtained by amidation of the deletion variants (for example, a heavy chain in which the carboxyl terminal proline residue has been amidated), and the like are also included. The type of deletion variant having a deletion at the carboxyl terminus of the heavy chain of the antibody according to the present invention is not limited to the above variants as long as the antigen-binding affinity and the effector function are conserved. The two heavy chains constituting the antibody according to the present invention may be of one type selected from the group consisting of a full-length heavy chain and the above-described deletion variant, or may be of two types in combination selected therefrom. The ratio of the amount of each deletion variant can be affected by the type of cultured mammalian cells which produce the antibody according to the present invention and the culture conditions; however, an antibody in which one amino acid residue at the carboxyl terminus has been deleted in both of the two heavy chains in the antibody according to the present invention can be preferably exemplified.

As isotypes of the antibody according to the present invention, for example, IgG (IgG1, IgG2, IgG3, IgG4) can be exemplified. Preferably, IgG1 or IgG2 can be exemplified.

Examples of antibodies in the antibody-drug conjugate used in the present invention can include, but are not particularly limited to, an anti-HER2 antibody, an anti-HER3 antibody, an anti-TROP2 antibody, an anti-B7-H3 antibody, an anti-GPR20 antibody, an anti-CDH6 antibody, an anti-CD3 antibody, an anti-CD30 antibody, an anti-CD33 antibody, an anti-CD37 antibody, an anti-CD56 antibody, an anti-CD98 antibody, an anti-DR5 antibody, an anti-EGFR antibody, an anti-EPHA2 antibody, an anti-FGFR2 antibody, an anti-FGFR4 antibody, an anti-FOLR1 antibody, an anti-VEGF antibody, an anti-CD20 antibody, an anti-CD22 antibody, an anti-CD70 antibody, an anti-PSMA antibody, an anti-CEA

antibody, an anti-Mesothelin antibody, an anti-A33 antibody, an anti-CanAg antibody, an anti-Cripto antibody, an anti-G250 antibody, an anti-MUC1 antibody, an anti-GPNMB antibody, an anti-Integrin antibody, an anti-Tenascin-C antibody, and an anti-SLC44A4 antibody. Further, an anti-HER2 antibody, an anti-HER3 antibody, an anti-TROP2 antibody, an anti-B7-H3 antibody, an anti-GPR20 antibody, and an anti-CDH6 antibody can be preferably exemplified.

In the present invention, the term "anti-HER2 antibody" refers to an antibody which binds specifically to HER2 (Human Epidermal Growth Factor Receptor Type 2; ErbB-2), and preferably has an activity of internalization in HER2-expressing cells by binding to HER2.

Examples of the anti-HER2 antibody include trastuzumab (U.S. Patent No. 5821337) and pertuzumab (International Publication No. WO 01/00245). Preferably, trastuzumab can be exemplified.

In the present invention, the term "anti-HER3 antibody" refers to an antibody which binds specifically to HER3 (Human Epidermal Growth Factor Receptor Type 3; ErbB-3), and preferably has an activity of internalization in HER3-expressing cells by binding to HER3.

Examples of the anti-HER3 antibody include patritumab (U3-1287), U1-59 (International Publication No. WO 2007/077028), MM-121 (seribantumab), an anti-ERBB3 antibody described in International Publication No. WO 2008/100624, RG-7116 (lumretuzumab), and LJM-716 (elgemtumab). Preferably, patritumab and U1-59 can be exemplified.

In the present invention, the term "anti-TROP2 antibody" refers to an antibody which binds specifically to TROP2 (TACSTD2: Tumor-associated calcium signal transducer 2; EGP-1), and preferably has an activity of internalization in TROP2-expressing cells by binding to TROP2.

Examples of the anti-TROP2 antibody include hTINA1-H1L1 (International Publication No. WO 2015/098099).

In the present invention, the term "anti-B7-H3 antibody" refers to an antibody which binds specifically to B7-H3 (B cell antigen #7 homolog 3; PD-l3; CD276), and preferably has an activity of internalization in B7-H3-expressing cells by binding to B7-H3.

Examples of the anti-B7-H3 antibody include M30-H1-L4 (International Publication No. WO 2014/057687).

In the present invention, the term "anti-GPR20 antibody" refers to an antibody which binds specifically to GPR20 (G Protein-coupled receptor 20), and preferably has an activity of internalization in GPR20-expressing cells by binding to GPR20.

Examples of the anti-GPR20 antibody include h046-H4e/L7 (International Publication No. WO 2018/135501).

In the present invention, the term "anti-CDH6 antibody" refers to an antibody which binds specifically to CDH6 (Cadherin-6), and preferably has an activity of internalization in CDH6-expressing cells by binding to CDH6.

Examples of the anti-CDH6 antibody include H01L02 (International Publication No. WO 2018/212136).

3. Production of the antibody-drug conjugate

A drug-linker intermediate for use in the production of the antibody-drug conjugate according to the present invention is represented by the following formula.

[Formula 17]

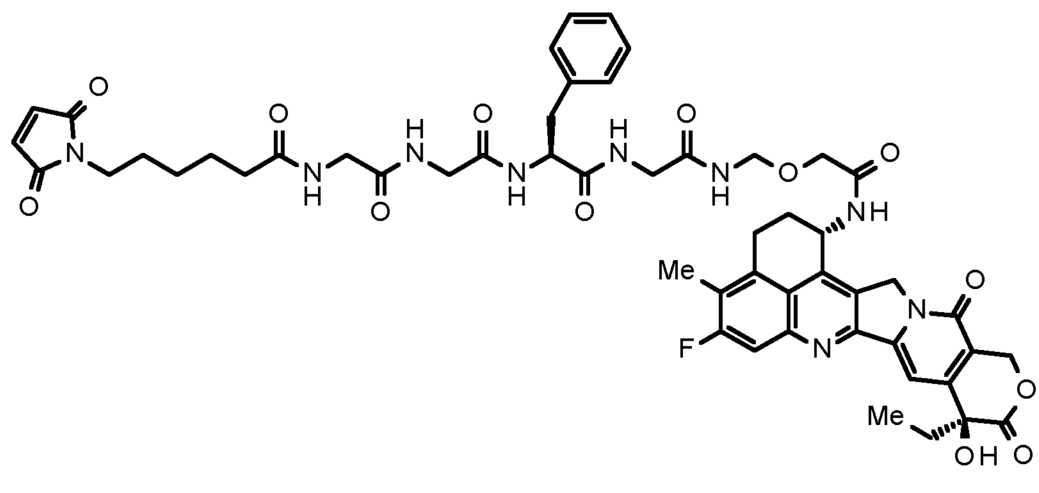

The drug-linker intermediate can be expressed as the chemical name N-[6-(2,5-dioxo-2,5-dihydro-1H-pyrrol-1-yl)hexanoyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycinamide, and can be produced with reference to descriptions in International Publication No. WO 2014/057687, International Publication No. WO 2015/098099, International Publication No. WO 2015/115091, International Publication No. WO 2015/155998, International Publication No. WO 2019/044947, and so on.

The antibody-drug conjugate used in the present invention can be produced by reacting the above-described drug-linker intermediate and an antibody having a thiol group (alternatively referred to as a sulfhydryl group).

The antibody having a sulfhydryl group can be obtained by a method well known in the art (Hermanson, G. T, Bioconjugate Techniques, pp. 56-136, pp. 456-493, Academic Press (1996)). For example, by using 0.3 to 3 molar equivalents of a reducing agent such as tris(2-carboxyethyl)phosphine hydrochloride (TCEP) per interchain disulfide within the antibody and reacting with the antibody in a buffer solution containing a chelating agent such as ethylenediamine tetraacetic acid (EDTA), an antibody having a sulfhydryl group with partially or completely reduced interchain disulfides within the antibody can be obtained.

Further, by using 2 to 20 molar equivalents of the drug-linker intermediate per the antibody having a sulfhydryl group, an antibody-drug conjugate in which 2 to 8 drug molecules are conjugated per antibody molecule can be produced.

The average number of conjugated drug molecules per antibody molecule of the antibody-drug conjugate produced can be determined, for example, by a method of calculation based on measurement of UV absorbance for the antibody-drug conjugate and the conjugation precursor thereof at two wavelengths of 280 nm and 370 nm (UV method), or a method of calculation based on quantification through HPLC measurement for fragments obtained by treating the antibody-drug conjugate with a reducing agent (HPLC method).

Conjugation between the antibody and the drug-linker intermediate and calculation of the average number of conjugated drug molecules per antibody molecule of the antibody-drug conjugate can be performed with reference to descriptions in International Publication No. WO 2014/057687, International Publication No. WO 2015/098099, International Publication No. WO 2015/115091, International Publication No. WO 2015/155998, International Publication No. WO 2018/135501, and International Publication No. WO 2018/212136, and so on.

In the present invention, the term "anti-HER2 antibody-drug conjugate" refers to an antibody-drug conjugate such that the antibody in the antibody-drug conjugate according to the invention is an anti-HER2 antibody.

The anti-HER2 antibody is preferably an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2,

more preferably an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2, and

even more preferably an antibody comprising a heavy chain consisting of an amino acid sequence represented by SEQ ID NO: 1 and a light chain consisting of an amino acid sequence represented by SEQ ID NO: 2, or an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

The average number of units of the drug-linker conjugated per antibody molecule in the anti-HER2 antibody-drug conjugate is preferably 2 to 8, more preferably 3 to 8, even more preferably 7 to 8, even more preferably 7.5 to 8, and even more preferably about 8.

The anti-HER2 antibody-drug conjugate can be produced with reference to descriptions in International Publication No. WO 2015/115091 and so on.

In the present invention, the term "anti-HER3 antibody-drug conjugate" refers to an antibody-drug conjugate such that the antibody in the antibody-drug conjugate according to the invention is an anti-HER3 antibody.

The anti-HER3 antibody is preferably an antibody comprising a heavy chain comprising CDRH1 consisting of an amino acid sequence consisting of amino acid residues 26 to 35 of SEQ ID NO: 3, CDRH2 consisting of an amino acid sequence consisting of amino acid residues 50 to 65 of SEQ ID NO: 3, and CDRH3 consisting of an amino acid sequence consisting of amino acid residues 98 to 106 of SEQ ID NO: 3, and a light chain comprising CDRL1 consisting of an amino acid sequence consisting of amino acid residues 24 to 39 of SEQ ID NO: 4, CDRL2 consisting of an amino acid sequence consisting of amino acid residues 56 to 62 of SEQ ID NO: 4, and CDRL3 consisting of an amino acid sequence consisting of amino acid residues 95 to 103 of SEQ ID NO: 4,

more preferably an antibody comprising a heavy chain comprising a heavy chain variable region consisting of an amino acid sequence consisting of amino acid residues 1 to 117 of SEQ ID NO: 3, and a light chain comprising a light chain variable region consisting of an amino acid sequence consisting of amino acid residues 1 to 113 of SEQ ID NO: 4, and

even more preferably an antibody comprising a heavy chain consisting of an amino acid sequence represented by SEQ ID NO: 3 and a light chain consisting of an amino acid sequence represented by SEQ ID NO: 4, or a variant of the antibody in which a lysine residue at the carboxyl terminus of the heavy chain is deleted.

The average number of units of the drug-linker conjugated per antibody molecule in the anti-HER3 antibody-drug conjugate is preferably 2 to 8, more preferably 3 to 8, even more preferably 7 to 8, even more preferably 7.5 to 8, and even more preferably about 8.

The anti-HER3 antibody-drug conjugate can be produced with reference to descriptions in International Publication No. WO 2015/155998 and so on.

In the present invention, the term "anti-TROP2 antibody-drug conjugate" refers to an antibody-drug conjugate such that the antibody in the antibody-drug conjugate according to the invention is an anti-TROP2 antibody.

The anti-TROP2 antibody is preferably an antibody comprising a heavy chain comprising CDRH1 consisting of an amino acid sequence consisting of amino acid residues 50 to 54 of SEQ ID NO: 5, CDRH2 consisting of an amino acid sequence consisting of amino acid residues 69 to 85 of SEQ ID NO: 5, and CDRH3 consisting of an amino acid sequence consisting of amino acid residues 118 to 129 of SEQ ID NO: 5, and a light chain comprising CDRL1 consisting of an amino acid sequence consisting of amino acid residues 44 to 54 of SEQ ID NO: 6, CDRL2 consisting of an amino acid sequence consisting of amino acid residues 70 to 76 of SEQ ID NO: 6, and CDRL3 consisting of an amino acid sequence consisting of amino acid residues 109 to 117 of SEQ ID NO: 6,

more preferably an antibody comprising a heavy chain comprising a heavy chain variable region consisting of an amino acid sequence consisting of amino acid residues 20 to 140 of SEQ ID NO: 5, and a light chain comprising a light chain variable region consisting of an amino acid sequence consisting of amino acid residues 21 to 129 of SEQ ID NO: 6, and

even more preferably an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6, or a variant of the antibody in which a lysine residue at the carboxyl terminus of the heavy chain is deleted.

The average number of units of the drug-linker conjugated per antibody molecule in the anti-TROP2 antibody-drug conjugate is preferably 2 to 8, more preferably 3 to 5, even more preferably 3.5 to 4.5, and even more preferably about 4.

The anti-TROP2 antibody-drug conjugate can be produced with reference to descriptions in International Publication No. WO 2015/098099 and International Publication No. WO 2017/002776.

In the present invention, the term "anti-B7-H3 antibody-drug conjugate" refers to an antibody-drug conjugate such that the antibody in the antibody-drug conjugate according to the invention is an anti-B7-H3 antibody.

The anti-B7-H3 antibody is preferably an antibody comprising a heavy chain comprising CDRH1 consisting of an amino acid sequence consisting of amino acid residues 50 to 54 of SEQ ID NO: 7, CDRH2 consisting of an amino acid sequence consisting of amino acid residues 69 to 85 of SEQ ID NO: 7, and CDRH3 consisting of an amino acid sequence consisting of amino acid residues 118 to 130 of SEQ ID NO: 7, and a light chain comprising CDRL1 consisting of an amino acid sequence consisting of amino acid residues 44 to 53 of SEQ ID NO: 8, CDRL2 consisting of an amino acid sequence consisting of amino acid residues 69 to 75 of SEQ ID NO: 8, and CDRL3 consisting of an amino acid sequence consisting of amino acid residues 108 to 116 of SEQ ID NO: 8,

more preferably an antibody comprising a heavy chain comprising a heavy chain variable region consisting of an amino acid sequence consisting of amino acid residues 20 to 141 of SEQ ID NO: 7, and a light chain comprising a light chain variable region consisting of an amino acid sequence consisting of amino acid residues 21 to 128 of SEQ ID NO: 8, and

even more preferably an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8, or a variant of the antibody in which a lysine residue at the carboxyl terminus of the heavy chain is deleted.

The average number of units of the drug-linker conjugated per antibody molecule in the anti-B7-H3 antibody-drug conjugate is preferably 2 to 8, more preferably 3 to 5, even more preferably 3.5 to 4.5, and even more preferably

about 4.

The anti-B7-H3 antibody-drug conjugate used in the present invention can be produced with reference to descriptions in International Publication No. WO 2014/057687 and International Publication No. WO 2017/002776.

In the present invention, the term "anti-GPR20 antibody-drug conjugate" refers to an antibody-drug conjugate such that the antibody in the antibody-drug conjugate is an anti-GPR20 antibody.

The anti-GPR20 antibody is preferably an antibody comprising a heavy chain comprising CDRH1 consisting of an amino acid sequence consisting of amino acid residues 45 to 54 of SEQ ID NO: 9, CDRH2 consisting of an amino acid sequence consisting of amino acid residues 69 to 78 of SEQ ID NO: 9, and CDRH3 consisting of an amino acid sequence consisting of amino acid residues 118 to 131 of SEQ ID NO: 9, and a light chain comprising CDRL1 consisting of an amino acid sequence consisting of amino acid residues 44 to 54 of SEQ ID NO: 10, CDRL2 consisting of an amino acid sequence consisting of amino acid residues 70 to 76 of SEQ ID NO: 10, and CDRL3 consisting of an amino acid sequence consisting of amino acid residues 109 to 117 of SEQ ID NO: 10,

more preferably an antibody comprising a heavy chain comprising a heavy chain variable region consisting of an amino acid sequence consisting of amino acid residues 20 to 142 of SEQ ID NO: 9, and a light chain comprising a light chain variable region consisting of an amino acid sequence consisting of amino acid residues 21 to 129 of SEQ ID NO: 10, and

even more preferably an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10, or a variant of the antibody in which a lysine residue at the carboxyl terminus of the heavy chain is deleted.

The average number of units of the drug-linker conjugated per antibody molecule in the anti-GPR20 antibody-drug conjugate used in the present invention is preferably 2 to 8, more preferably 3 to 8, even more preferably 7 to 8, even more preferably 7.5 to 8, and even more preferably about 8.

The anti-GPR20 antibody-drug conjugate used in the present invention can be produced with reference to descriptions in International Publication No. WO 2018/135501 and so on.

In the present invention, the term "anti-CDH6 antibody-drug conjugate" refers to an antibody-drug conjugate such that the antibody in the antibody-drug conjugate is an anti-CDH6 antibody.

The anti-CDH6 antibody is preferably an antibody comprising a heavy chain comprising CDRH1 consisting of an amino acid sequence consisting of amino acid residues 45 to 54 of SEQ ID NO: 11, CDRH2 consisting of an amino acid sequence consisting of amino acid residues 69 to 78 of SEQ ID NO: 11, and CDRH3 consisting of an amino acid sequence consisting of amino acid residues 118 to 130 of SEQ ID NO: 11, and a light chain comprising CDRL1 consisting of an amino acid sequence consisting of amino acid residues 44 to 54 of SEQ ID NO: 12, CDRL2 consisting of an amino acid sequence consisting of amino acid residues 70 to 76 of SEQ ID NO: 12, and CDRL3 consisting of an amino acid sequence consisting of amino acid residues 109 to 116 of SEQ ID NO: 12,

more preferably an antibody comprising a heavy chain comprising a heavy chain variable region consisting of an amino acid sequence consisting of amino acid residues 20 to 141 of SEQ ID NO: 11 and a light chain comprising a light chain variable region consisting of an amino acid sequence consisting of amino acid residues 21 to 128 of SEQ ID NO: 12, and

even more preferably an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12, or a variant of the antibody in which a lysine residue at the carboxyl terminus of the heavy chain is deleted.

The average number of units of the drug-linker conjugated per antibody molecule in the anti-CDH6 antibody-drug conjugate used in the present invention is preferably 2 to 8, more preferably 3 to 8, even more preferably 7 to 8, even more preferably 7.5 to 8, and even more preferably about 8.

The anti-CDH6 antibody-drug conjugate used in the present invention can be produced with reference to descriptions in International Publication No. WO 2018/212136 and so on.

4. PARP inhibitor

In the present invention, "PARP inhibitor" is a drug that has the function of inhibiting PARP (poly[adenosine-5'-diphosphate (ADP)-ribose]polymerase), and thus preventing single-strand break repair (Benafif S, et al., Onco. Targets Ther. (2015) 8, 519-528.) (Fong PC, et al., N. Engl. J. Med. (2009) 361, 123-134.) (Gelmon KA, et al., Lancet Oncol. (2011) 12, 852-861.). PARP includes multiple subtypes, but the PARP inhibitor in the present invention preferably inhibits

PARP-1 and PARP-2. The PARP inhibitor in the present invention is not limited as long as it is a drug having the function of inhibiting PARP and thus prevents single-strand break repair, and olaparib (Menear KA, et al., J. Med. Chem. (2008) 51, 6581-6591.), rucaparib (Gillmore AT, et al., Org. Process Res. Dev. (2012) 16, 1897-1904.), niraparib (Jones P, et al., J. Med. Chem. (2009) 52, 7170-7185.), talazoparib (Shen Y, et al., Clin. Cancer Res. (2013) 19 (18), 5003-15.), veliparib, pamiparib, and fluzoparib, and pharmacologically acceptable salts thereof can be preferably exemplified. Further, olaparib, rucaparib, niraparib, talazoparib, and veliparib, and pharmacologically acceptable salts thereof can be more preferably exemplified.

"Pharmacologically acceptable salt" of the PARP inhibitor in the present invention may be either an acid addition salt or a base addition salt, but is preferably an acid addition salt, examples of which can include lower alkanesulfonates such as camsilate (camphorsulfonate), methanesulfonate, trifluoromethanesulfonate, and ethanesulfonate; arylsulfonates such as tosylate (p-toluenesulfonate), and benzenesulfonate; inorganic acid salts such as phosphate, nitrate, perchlorate, and hydrosulfate; hydrohalic acid salts such as hydrochloride, hydrobromide, hydroiodide, and hydrofluoride; organic acid salts such as acetate, malate, fumarate, succinate, citrate, tartrate, oxalate, and maleate; and amino acid salts such as ornithinate, glutamate, and aspartate.

Moreover, the PARP inhibitor and pharmacologically acceptable salts thereof may also be present as solvates, and these solvates are also included in the PARP inhibitors and pharmacologically acceptable salts thereof in the present invention.

Furthermore, the PARP inhibitor and pharmacologically acceptable salts thereof used in the present invention may also be present as solvates, and these solvates are also included in the PARP inhibitors or pharmacologically acceptable salts thereof used in the present invention.

Olaparib is a compound represented by the following formula:

[Formula 18]

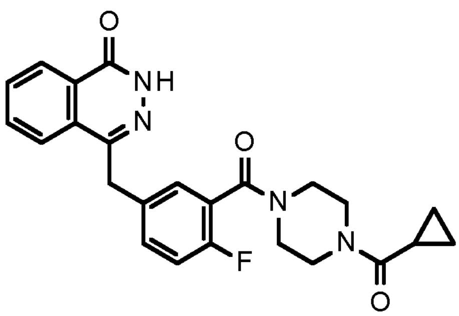

Rucaparib is a compound represented by the following formula:

[Formula 19]

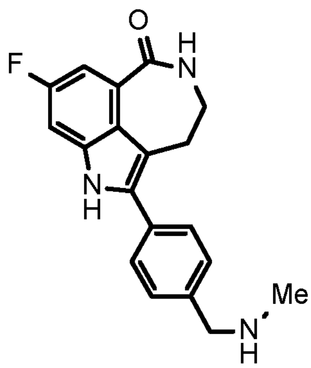

A pharmacologically acceptable salt of rucaparib is preferably camsilate (Rucaparib camsylate), or phosphate (rucaparib phosphate).

Niraparib is a compound represented by the following formula:

[Formula 20]

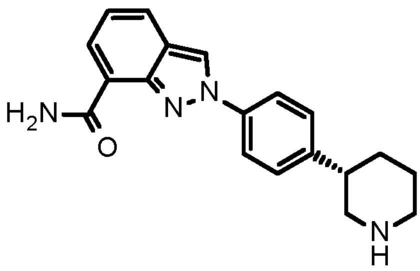

Talazoparib is a compound represented by the following formula:

[Formula 21]

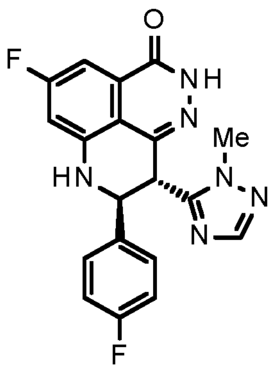

A pharmacologically acceptable salt of talazoparib is preferably tosylate (talazoparib tosylate (may also be called Talazoparib tosilate)).

Veliparib is a compound represented by the following formula:

[Formula 22]

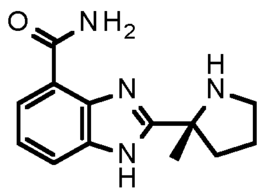

A pharmacologically acceptable salt of veliparib is preferably dihydrochloride (veliparib dihydrochrolide).

5. Medicament

Described in the following are a pharmaceutical composition and a method of treatment according to the present invention, wherein an antibody-drug conjugate and a PARP inhibitor are administered in combination.

The pharmaceutical composition and method of treatment of the present invention may be those in which the antibody-drug conjugate and the PARP inhibitor are separately contained as active components in different formulations and are administered simultaneously or at different times, or may be those in which the antibody-drug conjugate and the PARP inhibitor are contained as active components in a single formulation and administered.

The pharmaceutical composition and method of treatment of the present invention can be used for treating cancer, and can be preferably used for treating at least one disease selected from the group consisting of breast cancer (including triple-negative breast cancer and luminal breast cancer), gastric cancer (also called gastric adenocarcinoma), colorectal cancer (also called colon and rectal cancer, and including colon cancer and rectal cancer), lung cancer

(including small cell lung cancer and non-small cell lung cancer), esophageal cancer, head-and-neck cancer (including salivary gland cancer and pharyngeal cancer), gastroesophageal junction adenocarcinoma, biliary tract cancer (including bile duct cancer), Paget's disease, pancreatic cancer, ovarian cancer, uterine carcinosarcoma, urothelial cancer, prostate cancer, bladder cancer, gastrointestinal stromal tumor, uterine cervix cancer, squamous cell carcinoma, peritoneal cancer, liver cancer, hepatocellular cancer, endometrial cancer, kidney cancer, vulval cancer, thyroid cancer, penis cancer, leukemia, malignant lymphoma, plasmacytoma, myeloma, glioblastoma multiforme, sarcoma, osteosarcoma, and melanoma; and can more preferably be used for treating at least one cancer selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

Among the antibody-drug conjugates used in the present invention, the kind of antibody preferably used in the antibody-drug conjugate can be determined by examining the type of cancer and tumor markers. For example, in the case that HER2 expression is found in the cancer, an anti-HER2 antibody-drug conjugate can be preferably used; in the case that HER3 expression is found in the cancer, an anti-HER3 antibody-drug conjugate can be preferably used; in the case that TROP2 expression is found in the cancer, an anti-TROP2 antibody-drug conjugate can be preferably used; in the case that B7-H3 expression is found in the cancer, an anti-B7-H3 antibody-drug conjugate can be preferably used; in the case that GPR20 expression is found in the cancer, an anti-GPR20 antibody-drug conjugate can be preferably used; and in the case that CDH6 expression is found in the cancer, an anti-CDH6 antibody-drug conjugate can be preferably used.

The presence or absence of HER2, HER3, TROP2, B7-H3, GPR20 and CDH6, and other tumor markers, can be checked by, for example, collecting tumor tissue from a cancer patient, and subjecting the formalin fixed paraffin embedded specimen (FFPE) to an examination at a gene product (protein) level, such as an immunohistochemistry (IHC) method, a flow cytometry, a western blot method, or an examination at a gene transcription level, such as an in situ hybridization method (ISH), a quantitative PCR method (q-PCR), or a microarray analysis; alternatively, it can also be checked by collecting cell-free blood circulating tumor DNA (ctDNA) from a cancer patient and subjecting to an examination which uses a method such as next-generation sequencing (NGS).

In the case that the antibody-drug conjugate used in the present invention is an anti-HER2 antibody-drug conjugate, the pharmaceutical composition and method of treatment of the present invention can be preferably used not only for HER2-overexpressing cancer but also for HER2 low-expressing cancer and HER2-mutated cancer.

In the present invention, the term "HER2-overexpressing cancer" is not particularly limited as long as it is recognized as HER2-overexpressing cancer by those skilled in the art. Preferred examples of the HER2-overexpressing cancer can include cancer given a score of 3+ for the expression of HER2 in an immunohistochemical method, and cancer given a score of 2+ for the expression of HER2 in an immunohistochemical method and determined as positive for the expression of HER2 in an in situ hybridization method. The in situ hybridization method of the present invention includes a fluorescence in situ hybridization method (FISH) and a dual color in situ hybridization method (DISH).

In the present invention, the term "HER2 low-expressing cancer" is not particularly limited as long as it is recognized as HER2 low-expressing cancer by those skilled in the art. Preferred examples of the HER2 low-expressing cancer can include cancer given a score of 2+ for the expression of HER2 in an immunohistochemical method and determined as negative for the expression of HER2 in an in situ hybridization method, and cancer given a score of 1+ for the expression of HER2 in an immunohistochemical method.

The method for scoring the degree of HER2 expression by the immunohistochemical method, or the method for determining positivity or negativity to HER2 expression by the in situ hybridization method is not particularly limited as long as it is recognized by those skilled in the art. Examples of the method can include a method described in the 4th edition of the guidelines for HER2 testing, breast cancer (developed by the Japanese Pathology Board for Optimal Use of HER2 for Breast Cancer).

The HER2 low-expressing cancer for which the pharmaceutical composition and method of treatment of the present invention can be used is preferably HER2 low-expressing breast cancer, HER2 low-expressing gastric cancer, HER2 low-expressing colorectal cancer, or HER2 low-expressing non-small cell lung cancer, and is more preferably HER2 low-expressing breast cancer.

The pharmaceutical composition and method of treatment of the present invention can be preferably used for mammals, and can be more preferably used for humans.

The antitumor effect of the pharmaceutical composition and method of treatment of the present invention can be confirmed by, for example, generating a model in which cancer cells are transplanted to a test animal, and measuring reduction in tumor volume, life-prolonging effects due to applying the pharmaceutical composition and method of treatment of the present invention. Furthermore, comparison with the antitumor effect of single administration of each of the antibody-drug conjugate and the PARP inhibitor used in the present invention can provide confirmation of the combined effect of the antibody-drug conjugate and the PARP inhibitor used in the present invention.

In addition, the antitumor effect of the pharmaceutical composition and method of treatment of the present

invention can be confirmed, in a clinical study, with the Response Evaluation Criteria in Solid Tumors (RECIST) evaluation method, WHO's evaluation method, Macdonald's evaluation method, measurement of body weight, and other methods; and can be determined by indicators such as Complete response (CR), Partial response (PR), Progressive disease (PD), Objective response rate (ORR), Duration of response (DoR), Progression-free survival (PFS), and Overall survival (OS).

The foregoing methods can provide confirmation of superiority in terms of the antitumor effect of the pharmaceutical composition and method of treatment of the present invention compared to existing pharmaceutical compositions and methods of treatment for cancer therapy.

The pharmaceutical composition and method of treatment of the present invention can retard growth of cancer cells, suppress their proliferation, and further can kill cancer cells. These effects can allow cancer patients to be free from symptoms caused by cancer or can achieve an improvement in the QOL of cancer patients and attain a therapeutic effect by sustaining the lives of the cancer patients. Even if the pharmaceutical composition and method of treatment do not accomplish the killing of cancer cells, they can achieve higher QOL of cancer patients while achieving longer-term survival, by inhibiting or controlling the growth of cancer cells.

The pharmaceutical composition of the present invention can be expected to exert a therapeutic effect by application as systemic therapy to patients, and additionally, by local application to cancer tissues.

The pharmaceutical composition of the present invention may be administered as a pharmaceutical composition containing at least one pharmaceutically suitable ingredient. The pharmaceutically suitable ingredient can be suitably selected and applied from formulation additives or the like that are generally used in the art, in accordance with the dosage, administration concentration or the like of the antibody-drug conjugate used in the present invention and the PARP inhibitor. For example, the antibody-drug conjugate used in the present invention can be administered as a pharmaceutical composition containing a buffer such as a histidine buffer, an excipient such as sucrose or trehalose, and a surfactant such as Polysorbate 80 or 20. The pharmaceutical composition containing the antibody-drug conjugate used in the present invention can be preferably used as an injection, can be more preferably used as an aqueous injection or a lyophilized injection, and can be even more preferably used as a lyophilized injection.

In the case that the pharmaceutical composition containing the antibody-drug conjugate used in the present invention is an aqueous injection, it can be preferably diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

In the case that the pharmaceutical composition containing the antibody-drug conjugate used in the present invention is a lyophilized injection, it can be preferably dissolved in water for injection, subsequently a required amount can be diluted with a suitable diluent and then given as an intravenous infusion. For the diluent, a dextrose solution, physiological saline, and the like, can be exemplified, and a dextrose solution can be preferably exemplified, and a 5% dextrose solution can be more preferably exemplified.

Examples of the administration route which may be used to administer the pharmaceutical composition of the present invention include intravenous, intradermal, subcutaneous, intramuscular, and intraperitoneal routes; and preferably include an intravenous route.

The antibody-drug conjugate used in the present invention can be administered to a human once at intervals of 1 to 180 days, and can be preferably administered once a week, once every 2 weeks, once every 3 weeks, or once every 4 weeks, and can be even more preferably administered once every 3 weeks. Also, the antibody-drug conjugate used in the present invention can be administered at a dose of about 0.001 to 100 mg/kg, and can be preferably administered at a dose of 0.8 to 12.4 mg/kg. In the case that the antibody-drug conjugate used in the present invention is an anti-HER2 antibody-drug conjugate, it can be preferably administered once every 3 weeks at a dose of 0.8 mg/kg, 1.6 mg/kg, 3.2 mg/kg, 5.4 mg/kg, 6.4 mg/kg, 7.4 mg/kg, or 8 mg/kg. In the case that the antibody-drug conjugate used in the present invention is an anti-HER3 antibody-drug conjugate, it can be preferably administered once every 3 weeks at a dose of 1.6 mg/kg, 3.2 mg/kg, 4.8 mg/kg, 5.6 mg/kg, 6.4 mg/kg, 8.0 mg/kg, 9.6 mg/kg, or 12.8 mg/kg. In the case that the antibody-drug conjugate used in the present invention is an anti-TROP2 antibody-drug conjugate, it can be preferably administered once every 3 weeks at a dose of 0.27 mg/kg, 0.5 mg/kg, 1.0 mg/kg, 2.0 mg/kg, 4.0 mg/kg, 6.0 mg/kg, or 8.0 mg/kg.

The PARP inhibitor according to the present invention can be administered to a human once or twice at intervals of 1 to 7 days, and can be preferably administered once a day, or twice a day. Also, the PARP inhibitor used in the present invention can be administered at a dose of 0.1 mg to 3000 mg, and can be preferably administered at a dose of 0.25 mg to 600 mg.

If the PARP inhibitor used in the present invention is olaparib or a pharmacologically acceptable salt thereof, it can be preferably administered orally twice a day at a dose of 100 mg, 150 mg, 200 mg, or 300 mg.

If the PARP inhibitor used in the present invention is rucaparib or a pharmacologically acceptable salt thereof, it can be preferably administered orally twice a day at a dose of 200 mg, 250 mg, 300 mg, 400 mg, 500 mg, or 600 mg.

If the PARP inhibitor used in the present invention is niraparib or a pharmacologically acceptable salt thereof,

it can be preferably administered orally once a day at a dose of 100 mg, 200 mg, or 300 mg.

If the PARP inhibitor used in the present invention is talazoparib or a pharmacologically acceptable salt thereof, it can be preferably administered orally once a day at a dose of 0.25 mg, 0.5 mg, 0.75 mg, or 1 mg.

The pharmaceutical composition and method of treatment of the present invention may further contain a cancer therapeutic agent other than the antibody-drug conjugate and the PARP inhibitor according to the present invention. The pharmaceutical composition and method of treatment of the present invention can also be administered in combination with another cancer therapeutic agent, thereby enhancing the antitumor effect. Other cancer therapeutic agents to be used for such purpose may be administered to a subject simultaneously, separately, or sequentially with the pharmaceutical composition of the present invention, or may be administered with varying each dosage interval. Such cancer therapeutic agents are not limited as long as they are agents having antitumor activity, and can be exemplified by at least one selected from the group consisting of irinotecan (CPT-11), cisplatin, carboplatin, oxaliplatin, fluorouracil (5-FU), gemcitabine, capecitabine, paclitaxel, docetaxel, doxorubicin, epirubicin, cyclophosphamide, mitomycin C, tegafur-gimeracil-oteracil combination, cetuximab, panitumumab, bevacizumab, ramucirumab, regorafenib, trifluridine-tipiracil combination, gefitinib, erlotinib, afatinib, methotrexate, pemetrexed, tamoxifen, toremifene, fulvestrant, leuprorelin, goserelin, letrozole, anastrozole, progesterone formulation, trastuzumab, pertuzumab, and lapatinib.

The pharmaceutical composition and method of treatment of the present invention can also be used in combination with radiotherapy. For example, a cancer patient may receive radiotherapy before and/or after or simultaneously with receiving therapy with the pharmaceutical composition of the present invention.

The pharmaceutical composition and method of treatment of the present invention can also be used as an adjuvant chemotherapy in combination with a surgical procedure. The pharmaceutical composition of the present invention may be administered for the purpose of diminishing the size of a tumor before a surgical procedure (referred to as pre-operative adjuvant chemotherapy or neoadjuvant therapy), or may be administered after a surgical procedure for the purpose of preventing the recurrence of a tumor (referred to as post-operative adjuvant chemotherapy or adjuvant therapy).

## Examples

The present invention is specifically described in view of the examples shown below. However, the present invention is not limited to these. Further, it is by no means to be interpreted in a limited way.

### Example 1: Production of the anti-HER2 antibody-drug conjugate (1)

In accordance with a production method described in International Publication No. WO 2015/115091 with use of an anti-HER2 antibody (an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2), an anti-HER2 antibody-drug conjugate in which a drug-linker represented by the following formula:

[Formula 23]

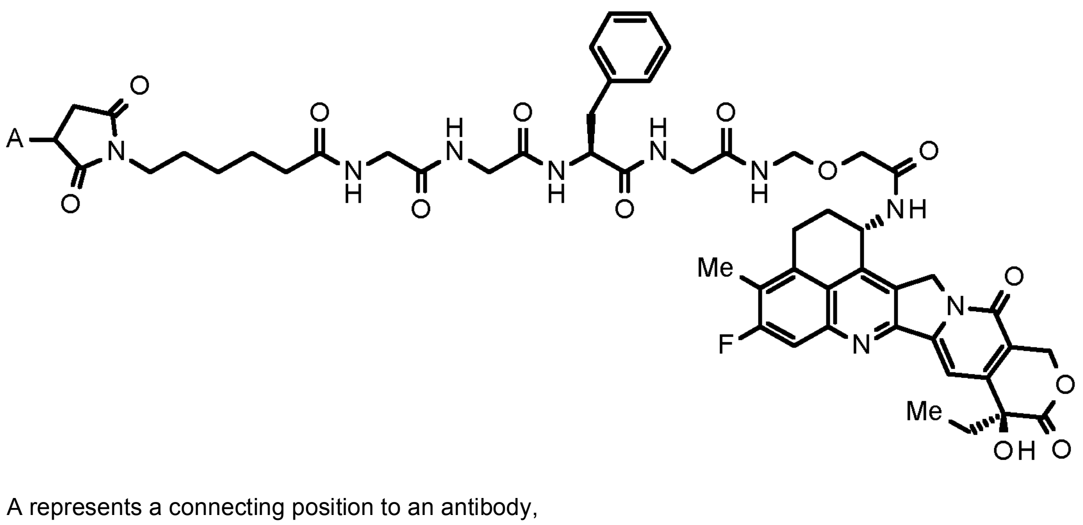

wherein A represents a connecting position to an antibody,
is conjugated to the anti-HER2 antibody via a thioether bond (hereinafter referred to as the "HER2-ADC (1)") was

produced. The DAR of the HER2-ADC (1) is 7.7 or 7.8.

Example 2: Production of the anti-TROP2 antibody-drug conjugate (1)

In accordance with a production method described in International Publication No. WO 2015/098099 and International Publication No. 2017/002776 with use of an anti-TROP2 antibody (an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6), an anti-TROP2 antibody-drug conjugate in which a drug-linker represented by the following formula:

[Formula 24]

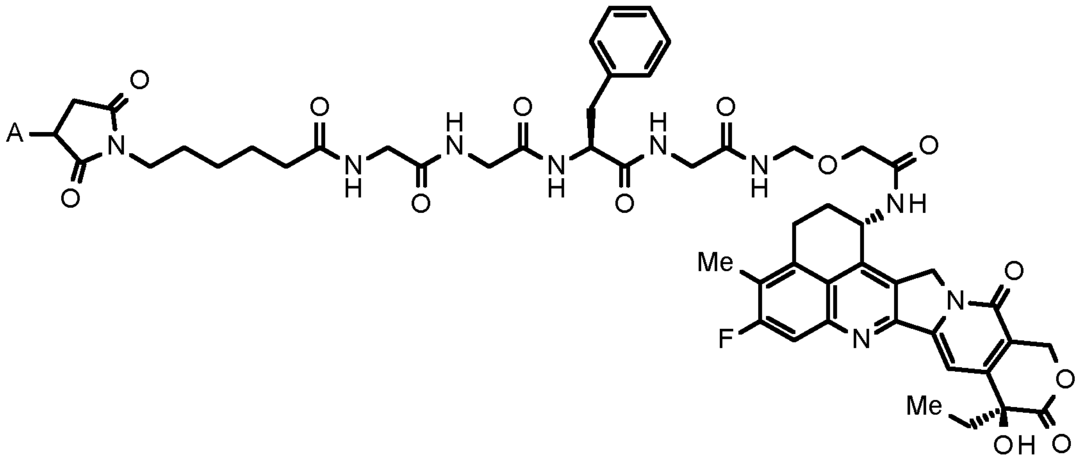

wherein A represents the connecting position to an antibody,
is conjugated to the anti-TROP2 antibody via a thioether bond (hereinafter referred to as the "TROP2-ADC (1)") was produced. The DAR of TROP2-ADC (1) is 3.5 to 4.5.

Example 3: Production of Compound (1)

In accordance with a production method described in International Publication No. WO 2014/057687 and International Publication No. 2015/115091, a compound represented by the following formula:

[Formula 25]

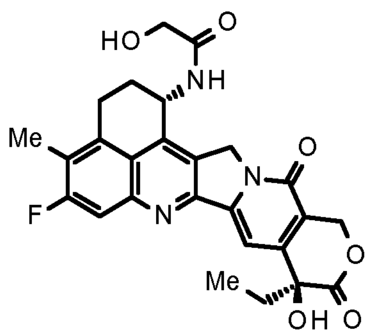

(hereinafter referred to as the "Compound (1)") was produced.

Example 4: Cell growth inhibition study (1)

Human gastric cancer cell line NCI-N87, which was obtained from ATCC (American Type Culture Collection), was used for evaluation. To a 1536-well cell culture plate, olaparib, talazoparib, rucaparib (phosphate), niraparib, or Dimethyl sulfoxide (DMSO) prepared at 2 mM, 400 μM, 80 μM, 16 μM, 3.2 μM, and 640 nM with DMSO were added

individually at 25 nL/well. Furthermore, Compound (1) prepared at 60 nM, 24 nM, 9.6 nM, 3.8 nM, 1.5 nM, and 0.61 nM with RPMI1640 Medium (Thermo Fisher Scientific) containing 10% fetal bovine serum (GE Healthcare); or TROP2-ADC (1) prepared at 32 nM, 11 nM, 3.6 nM, 1.2 nM, 0.40 nM, and 0.13 nM; or HER2-ADC (1) prepared at 8.0 nM, 2.7 nM, 0.89 nM, 0.30 nM, 0.10 nM, and 0.033 nM were added individually at 2.5 μL/well. Then, NCI-N87 cells suspended at 4 × $10^4$ cells/mL with RPMI1640 Medium containing 10% fetal bovine serum were seeded at 2.5 μL/well, and cultured at 37°C under 5%$CO_2$ for 6 days.

After culturing, a solution of CellTiter-Glo 2.0 Assay (Promega) diluted with an equivalent volume of Glo Lysis buffer, 1× (Promega) was added at 2 μL/well and subjected to incubation at room temperature for an hour, followed by measurement of luminescence intensity of each well.

Cell growth inhibition rate (%) in each condition was calculated using the following formula:

$$\text{Cell growth inhibition rate (\%)} = 100 \times (T - B) / (C - B) - 100$$

T: average luminescence intensity of wells added with each sample
B: average luminescence intensity of wells added with DMSO and medium
C: average luminescence intensity of wells added with DMSO and cells

Furthermore, sigmoid fitting was performed for the concentration-dependent transition of cell growth inhibition rate in each combination condition using Genedata Screener Analyzer Version 14 (Genedata, hereinafter referred to as Screener).

For combination effects, differences between estimates of additive effects from the Loewe model (Greco WR. et al., Pharmacol. Rev. 1995 Jun; 47 (2): 331-85) and cell growth inhibition rates (%) subjected to sigmoid fitting were converted to matrices, and Synergy Scores were calculated from the matrix elements using a method described in the reference (Lehar J. et al., Nat Biotechnol. 2009 Jul; 27 (7):659-66). Besides, Synergy Score = 0 indicates an additive effect; Synergy Score > 0 indicates a synergistic effect; and Synergy Score < 0 indicates an antagonistic effect.

Synergy Score in each combination is shown in Table 1. In the cell growth inhibition study for the NCI-N87 cell line, Compound (1), HER2-ADC (1), and TROP2-ADC (1) showed synergistic effects in all of the combinations with olaparib, talazoparib, rucaparib, and niraparib.

[Table 1]

| Synergy Score of each combination in NCI-N87 cell line | | | | |
|---|---|---|---|---|
| | Olaparib | Talazoparib | Rucaparib | Niraparib |
| Compound (1) | 3.21 | 4.58 | 1.93 | 1.23 |
| HER2-ADC (1) | 5.05 | 7.22 | 6.30 | 3.66 |
| TROP2-ADC (1) | 4.65 | 9.50 | 4.05 | 4.94 |

Example 5: Cell growth inhibition study (2)

Human breast cancer cell line KPL-4, which was obtained from Dr. Junichi Kurebayashi in Kawasaki Medical School [British Journal of Cancer, (1999) 79 (5/6). 707-717], was used for evaluation. To a 1536-well cell culture plate, olaparib, talazoparib, rucaparib (phosphate), niraparib, or DMSO prepared at 2 mM, 400 μM, 80 μM, 16 μM, 3.2 μM, and 640 nM with DMSO were added individually at 25 nL/well. Furthermore, Compound (1) prepared at 36 nM, 20 nM, 11 nM, 6.2 nM, 3.4 nM, and 1.9 nM with RPMI1640 Medium containing 10% fetal bovine serum; or TROP2-ADC (1) prepared at 40 nM, 13 nM, 4.4 nM, 1.5 nM, 0.49 nM, and 0.16 nM; or HER2-ADC (1) prepared at 3.7 nM, 1.3 nM, 0.48 nM, 0.17 nM, 0.061 nM, and 0.022 nM were added individually at 2.5 μL/well. Then, KPL-4 cells suspended at 1 × $10^4$ cells/mL with RPMI1640 Medium containing 10% fetal bovine serum were seeded at 2.5 μL/well, and cultured at 37°C under 5%$CO_2$ for 6 days. After culturing, a solution of CellTiter-Glo 2.0 Assay diluted with an equivalent volume of Glo Lysis buffer, 1× was added at 2 μL/well and subjected to incubation at room temperature for an hour, followed by measurement of luminescence intensity of each well.

Analysis for cell growth inhibition rate (%) and combination effect in each condition is performed in a similar manner to Example 4.

Synergy Score in each combination is shown in Table 2. In the cell growth inhibition study for the KPL-4 cell

line, Compound (1), HER2-ADC (1), and TROP2-ADC (1) showed synergistic effects in all of the combinations with olaparib, talazoparib, rucaparib, or niraparib.

[Table 2]

| Synergy Score of each combination in KPL-4 cell line | | | | |
|---|---|---|---|---|
| | Olaparib | Talazoparib | Rucaparib | Niraparib |
| Compound (1) | 3.97 | 4.86 | 4.97 | 3.68 |
| HER2-ADC (1) | 7.11 | 11.0 | 6.50 | 8.12 |
| TROP2-ADC (1) | 14.9 | 21.4 | 13.6 | 10.3 |

Example 6: Cell growth inhibition study (3)

Human lung cancer cell line EBC-1, which was obtained from Health Science Research Resources Bank (current Japanese Collection of Research Bioresources [JCRB] Cell Bank), was used for evaluation. To a 1536-well cell culture plate, olaparib, talazoparib, rucaparib (phosphate), niraparib, or DMSO prepared at 2 mM, 400 μM, 80 μM, 16 μM, 3.2 μM, and 640 nM with DMSO were added individually at 25 nL/well. Furthermore, Compound (1) prepared at 16 nM, 8.0 nM, 4.0 nM, 2.0 nM, 1.0 nM, and 0.50 nM with RPMI1640 Medium containing 10% fetal bovine serum; or TROP2-ADC (1) prepared at 40 nM, 13 nM, 4.4 nM, 1.5 nM, 0.49 nM, and 0.16 nM were added individually at 2.5μL/well. Then, EBC-1 cells suspended at $2 \times 10^4$ cells/mL with RPMI1640 Medium containing 10% fetal bovine serum were seeded at 2.5 μL/well, and cultured at 37°C under 5%$CO_2$ for 6 days. After culturing, a solution of CellTiter-Glo 2.0 Assay diluted with an equivalent volume of Glo Lysis buffer, 1× was added at 2 μL/well and subjected to incubation at room temperature for an hour, followed by measurement of luminescence intensity of each well.

Analysis for cell growth inhibition rate (%) and combination effect in each condition is performed in a similar manner to Example 4. Synergy Score in each combination is shown in Table 3. In the cell growth inhibition study for the EBC-1 cell line, Compound (1) and TROP2-ADC (1) showed synergistic effects in all of the combinations with olaparib, talazoparib, rucaparib, or niraparib.

[Table 3]

| Synergy Score of each combination in EBC-1 cell line | | | | |
|---|---|---|---|---|
| | Olaparib | Talazoparib | Rucaparib | Niraparib |
| Compound (1) | 3.39 | 5.66 | 4.41 | 3.17 |
| TROP2-ADC (1) | 12.3 | 22.4 | 14.4 | 14.7 |

Example 7: Cell growth inhibition study (4)

Human breast cancer cell line HCC70, which was obtained from ATCC, was used for evaluation. To a 1536-well cell culture plate, olaparib, talazoparib, rucaparib (phosphate), niraparib, or DMSO prepared at 2 mM, 400 μM, 80 μM, 16 μM, 3.2 μM, and 640 nM with DMSO were added individually at 25 nL/well. Furthermore, Compound (1) prepared at 800 nM, 200 nM, 50 nM, 13 nM, 3.1 nM, and 0.78 nM with RPMI1640 Medium containing 10% fetal bovine serum; or TROP2-ADC (1) prepared at 5.6 nM, 2.0 nM, 0.71 nM, 0.26 nM, 0.091 nM, and 0.033 nM were added individually at 2.5 μL/well. Then, HCC70 cells suspended at $4 \times 10^4$ cells/mL with RPMI1640 Medium containing 10% fetal bovine serum were seeded at 2.5 μL/well, and cultured at 37°C under 5%$CO_2$ for 6 days. After culturing, a solution of CellTiter-Glo 2.0 Assay diluted with an equivalent volume of Glo Lysis buffer, 1× was added at 2 μL/well and subjected to incubation at room temperature for an hour, followed by measurement of luminescence intensity of each well.

Analysis for cell growth inhibition rate (%) and combination effect in each condition is performed in a similar manner to Example 4. Synergy Score in each combination is shown in Table 4. In the cell growth inhibition study for the HCC70 cell line, Compound (1) and TROP2-ADC (1) showed synergistic effects in all of the combinations with olaparib, talazoparib, rucaparib, or niraparib.

[Table 4]

| Synergy Score of each combination in HCC70 cell line | | | | |
|---|---|---|---|---|
| | Olaparib | Talazoparib | Rucaparib | Niraparib |
| Compound (1) | 1.98 | 4.61 | 2.48 | 1.61 |
| TROP2-ADC (1) | 1.91 | 2.09 | 1.47 | 1.05 |

Example 8: Cell growth inhibition study (5)

Human pancreatic cancer cell line BxPC-3, which was obtained from ATCC, was used for evaluation. To a 1536-well cell culture plate, olaparib, talazoparib, rucaparib (phosphate), niraparib, or DMSO prepared at 2 mM, 400 μM, 80 μM, 16 μM, 3.2 μM, and 640 nM with DMSO were added individually at 25 nL/well. Furthermore, Compound (1) prepared at 80 nM, 32 nM, 13 nM, 5.1 nM, 2.0 nM, and 0.82 nM with RPMI1640 Medium containing 10% fetal bovine serum; or TROP2-ADC (1) prepared at 16 nM, 5.3 nM, 1.8 nM, 0.59 nM, 0.20 nM, and 0.066 nM were added individually at 2.5μL/well. Then, BxPC-3 cells suspended at $4 \times 10^4$ cells/mL with RPMI1640 Medium containing 10% fetal bovine serum were seeded at 2.5 μL/well, and cultured at 37°C under 5%$CO_2$ for 6 days. After culturing, a solution of CellTiter-Glo 2.0 Assay (Promega) diluted with an equivalent volume of Glo Lysis buffer, 1× (Promega) was added at 2 μL/well and subjected to incubation at room temperature for an hour, followed by measurement of luminescence intensity of each well.

Analysis for cell growth inhibition rate (%) and combination effect in each condition is performed in a similar manner to Example 4. Synergy Score in each combination is shown in Table 5. In the cell growth inhibition study for the BxPC-3 cell line, Compound (1) and TROP2-ADC (1) showed synergistic effects in all of the combinations with olaparib, talazoparib, rucaparib, or niraparib.

[Table 5]

| Synergy Score of each combination in BxPC-3 cell line | | | | |
|---|---|---|---|---|
| | Olaparib | Talazoparib | Rucaparib | Niraparib |
| Compound (1) | 1.56 | 3.25 | 2.15 | 1.85 |
| TROP2-ADC (1) | 3.40 | 4.33 | 2.64 | 1.72 |

Example 9: Antitumor study (1)

Mouse: Female 5-6-week-old BALB/c nude mice (CHARLES RIVER LABORATORIES JAPAN, INC.) were subjected to the experiments.

Measurement and calculation formula: In all studies, the major axis and minor axis of tumors were measured twice a week with an electronic digital caliper (CD15-CX, Mitutoyo Corp.), and the tumor volume ($mm^3$) was calculated. The calculation formula is as shown below. Tumor volume ($mm^3$) = $1/2 \times$ Major axis (mm) $\times$ [Minor axis (mm) ]$^2$

The HER2-ADC (1) was diluted with ABS buffer (10 mM acetate buffer [pH 5.5], 5% sorbitol), and intravenously administered in a fluid volume of 10 mL/kg to the tail vein. Olaparib was dissolved with Dimethyl sulfoxide (DMSO), diluted with 10% 2-hydroxy-propyl-β-cyclodextrin/Dulbecco's Phosphate-Buffered Saline, and then intraperitoneally administered in a fluid volume of 10 mL/kg. Talazoparib was dissolved with DMSO, diluted with 0.5% hydroxypropyl methylcellulose, and orally administered in a fluid volume of 10 mL/kg. Rucaparib (camsylate) and niraparib were dissolved with DMSO, diluted with 0.5% methylcellulose, and orally administered in a fluid volume of 10 mL/kg. These methods are common to Examples 9 to 12.

Human breast cancer cell line KPL-4 [British Journal of Cancer, (1999) 79 (5/6). 707-717], which was obtained from Dr. Junichi Kurebayashi in Kawasaki Medical School, was suspended into physiological saline, subcutaneously transplanted at $1.5 \times 10^7$ cells into the right side of female nude mice, and the mice were randomly grouped 17 days after the transplantation (Day 0). The HER2-ADC (1) was intravenously administered to the tail vein at a dose of 7.5 mg/kg on Day 0. The PARP inhibitors were administered once a day, five times a week, for two weeks; at doses of 50 mg/kg for olaparib and niraparib, 0.4 mg/kg for talazoparib, and 150 mg/kg for rucaparib. Single administration groups of each drug, a combined administration group, and a solvent administration group as a control group were set up.

Results of a combination of HER2-ADC (1) and olaparib are shown in Figure 13. Single administration of olaparib showed a tumor growth inhibition (TGI) of 21% on the last day of the study. Single administration of HER2-ADC (1) showed TGI of 83%. On the other hand, combined administration of HER2-ADC (1) and olaparib exhibited a significantly superior tumor growth suppression effect than single administration of olaparib (P < 0.0001 [calculated by Dunnett's test; the same applies hereinafter]. The combination also had a higher tumor growth inhibition than single administration of HER2-ADC (1) (TGI, 95%), indicating a potent combination effect. Here, in the Figure, the abscissa axis represents days after cell transplantation, and the longitudinal axis represents tumor volume. In addition, none of the single and combined administration groups exhibited any particular notable finding such as weight loss. Incidentally, in the following evaluation examples relating to antitumor studies, unless otherwise described, the studies are performed by the procedure used in this evaluation example.

Results of a combination of HER2-ADC (1) and talazoparib are shown in Figure 14. Single administration of talazoparib showed TGI of 31%. Single administration of HER2-ADC (1) showed TGI of 83%. On the other hand, combined administration of HER2-ADC (1) and talazoparib exhibited a significantly superior tumor growth suppression effect than single administration of talazoparib (P < 0.0001). The combination also had a higher tumor growth inhibition than single administration of HER2-ADC (1) (TGI, 99%), indicating a potent combination effect. None of the single and combined administration groups exhibited any particular notable finding such as weight loss.

Results of a combination of HER2-ADC (1) and niraparib are shown in Figure 15. Single administration of niraparib showed TGI of 39%. Single administration of HER2-ADC (1) showed TGI of 83%. On the other hand, combined administration of HER2-ADC (1) and niraparib exhibited a significantly superior tumor growth suppression effect than single administration of niraparib (P = 0.0001). The combination also had a higher tumor growth inhibition than single administration of HER2-ADC (1) (TGI, 97%), indicating a potent combination effect. None of the single and combined administration groups exhibited any particular notable finding such as weight loss.

Results of a combination of HER2-ADC (1) and rucaparib are shown in Figure 16. Single administration of rucaparib showed TGI of 42%. Single administration of HER2-ADC (1) showed TGI of 83%. On the other hand, combined administration of HER2-ADC (1) and rucaparib exhibited a significantly superior tumor growth suppression effect than single administration of rucaparib (P < 0.0001). The combination also had a higher tumor growth inhibition than single administration of HER2-ADC (1) (TGI, 100%), indicating a potent combination effect. None of the single and combined administration groups exhibited any particular notable finding such as weight loss.

Example 10: Antitumor study (2)

Human breast cancer cell line JIMT-1, which was purchased from DSMZ (Deutsche Sammlung von Mikroorganismen und Zellkulturen GmbH), was suspended into physiological saline, subcutaneously transplanted at $5\times10^6$ cells into the right side of female nude mice, and the mice were randomly grouped 10 days after the transplantation (Day 0). The HER2-ADC (1) was intravenously administered to the tail vein at a dose of 10 mg/kg on Day 0. The PARP inhibitors were administered once a day, five times a week, for three weeks; at doses of 50 mg/kg for olaparib, 0.4 mg/kg for talazoparib, and 150 mg/kg for rucaparib. Single administration groups of each drug, a combined administration group, and a solvent administration group as a control group were set up.

Results of a combination of HER2-ADC (1) and olaparib are shown in Figure 17. Single administration of olaparib showed TGI of 26%. Single administration of HER2-ADC (1) showed TGI of 80%. On the other hand, combined administration of HER2-ADC (1) and olaparib exhibited a significantly superior tumor growth suppression effect than single administration of olaparib (P < 0.0001). The combination also had a higher tumor growth inhibition than single administration of HER2-ADC (1) (TGI, 84%). None of the single and combined administration groups exhibited any particular notable finding such as weight loss.

Results of a combination of HER2-ADC (1) and talazoparib are shown in Figure 18. Single administration of talazoparib showed TGI of 13%. Single administration of HER2-ADC (1) showed TGI of 80%. On the other hand, combined administration of HER2-ADC (1) and talazoparib exhibited a significantly superior tumor growth suppression effect than single administration of talazoparib (P < 0.0001). The combination also had a higher tumor growth suppression effect than single administration of HER2-ADC (1) (TGI, 93%), indicating a potent combination effect. None of the single and combined administration groups exhibited any particular notable finding such as weight loss.

Results of a combination of HER2-ADC (1) and rucaparib are shown in Figure 19. Single administration of rucaparib showed TGI of 30%. Single administration of HER2-ADC (1) showed TGI of 80%. On the other hand, combined administration of HER2-ADC (1) and rucaparib exhibited a significantly superior tumor growth suppression effect than single administration of rucaparib (P = 0.0002). The combination also had a higher tumor growth inhibition than single administration of HER2-ADC (1) (TGI, 84%). None of the single and combined administration groups exhibited any particular notable finding such as weight loss.

Example 11: Antitumor study (3)

Human gastric cancer cell line NCI-N87, which was purchased from ATCC (American Type Culture Collection), was suspended into physiological saline, subcutaneously transplanted at $1\times10^7$ cells into the right side of female nude

mice, and the mice were randomly grouped 6 days after the transplantation (Day 0). The HER2-ADC (1) was intravenously administered to the tail vein at a dose of 1 mg/kg on Day 0. The PARP inhibitors were administered once a day, five times a week, for two weeks; at doses of 50 mg/kg for olaparib and 0.4 mg/kg for talazoparib. Single administration groups of each drug, a combined administration group, and a solvent administration group as a control group were set up. In addition, none of the single and combined administration groups exhibited any particular notable finding such as weight loss.

Results of a combination of HER2-ADC (1) and olaparib are shown in Figure 20. Single administration of olaparib showed TGI of 26%. Single administration of HER2-ADC (1) showed TGI of 45%. On the other hand, combined administration of HER2-ADC (1) and olaparib had a higher tumor growth inhibition than single administration of olaparib or HER2-ADC (1) (TGI, 48%). None of the single and combined administration groups exhibited any particular notable finding such as weight loss.

Results of a combination of HER2-ADC (1) and talazoparib are shown in Figure 21. Single administration of talazoparib showed TGI of -2%. Single administration of HER2-ADC (1) showed TGI of 45%. On the other hand, combined administration of HER2-ADC (1) and talazoparib exhibited a significantly superior tumor growth suppression effect than single administration of talazoparib ($P < 0.0001$). The combination also had a higher tumor growth inhibition than single administration of HER2-ADC (1) (TGI, 67%), indicating a significantly superior combination effect ($P = 0.0103$). None of the single and combined administration groups exhibited any particular notable finding such as weight loss.

Example 12: Antitumor study (4)

Human ovarian cancer cell line SK-OV-3 cells, which were purchased from ATCC (American Type Culture Collection), were maintained by nude mouse transplantation, subcutaneously transplanted in the form of a tumor mass of solid tumor (3 × 3 × 3 mm) into the right side of female nude mice, and the mice were randomly grouped 19 days after the transplantation (Day 0). HER2-ADC (1) was intravenously administered to the tail vein at a dose of 3 mg/kg on Day 0 and Day 14. The PARP inhibitors were administered once a day, five times a week, for three weeks; at doses of 60 mg/kg for olaparib and 0.4 mg/kg for talazoparib. Single administration groups of each drug, a combined administration group, and a solvent administration group as a control group were set up. In addition, none of the single and combined administration groups exhibited any particular notable finding such as weight loss.

Results of a combination of HER2-ADC (1) and olaparib are shown in Figure 22. Single administration of olaparib showed TGI of 15%. Single administration of HER2-ADC (1) showed TGI of 39%. On the other hand, combined administration of HER2-ADC (1) and olaparib exhibited a significantly superior tumor growth suppression effect than single administration of olaparib ($P = 0.016$). The combination also had a higher tumor growth inhibition than single administration of HER2-ADC (1) (TGI, 61%). None of the single and combined administration groups exhibited any particular notable finding such as weight loss.

Results of a combination of HER2-ADC (1) and talazoparib are shown in Figure 23. Single administration of talazoparib showed TGI of 12%. Single administration of HER2-ADC (1) showed TGI of 39%. On the other hand, combined administration of HER2-ADC (1) and talazoparib exhibited a significantly superior tumor growth suppression effect than single administration of talazoparib ($P = 0.0004$). The combination also had a higher tumor growth inhibition than single administration of HER2-ADC (1) (TGI, 88%), indicating a significantly superior combination effect ($P = 0.0136$). None of the single and combined administration groups exhibited any particular notable finding such as weight loss.

Example 13: Production of the anti-HER3 antibody-drug conjugate (1)

In accordance with a production method described in International Publication No. WO 2015/155998 with use of an anti-HER3 antibody (an antibody comprising a heavy chain consisting of an amino acid sequence represented by SEQ ID NO: 3 and a light chain consisting of an amino acid sequence represented by SEQ ID NO: 4), an anti-HER3 antibody-drug conjugate in which a drug-linker represented by the following formula:

[Formula 26]

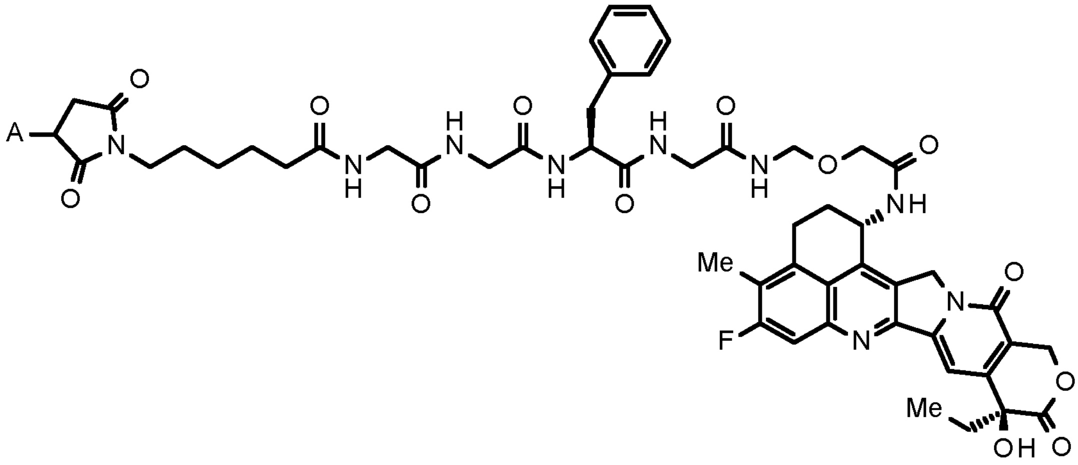

wherein A represents a connecting position to an antibody,
is conjugated to the anti-HER3 antibody via a thioether bond (hereinafter referred to as the "HER3-ADC (1)") was produced. The DAR of the HER3-ADC (1) is 7.6.

Example 14: Production of the anti-CDH6 antibody-drug conjugate (1)

In accordance with a production method described in International Publication No. WO 2018/212136 with use of an anti-CDH6 antibody (an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12), an anti-CDH6 antibody-drug conjugate in which a drug-linker represented by the following formula:

[Formula 27]

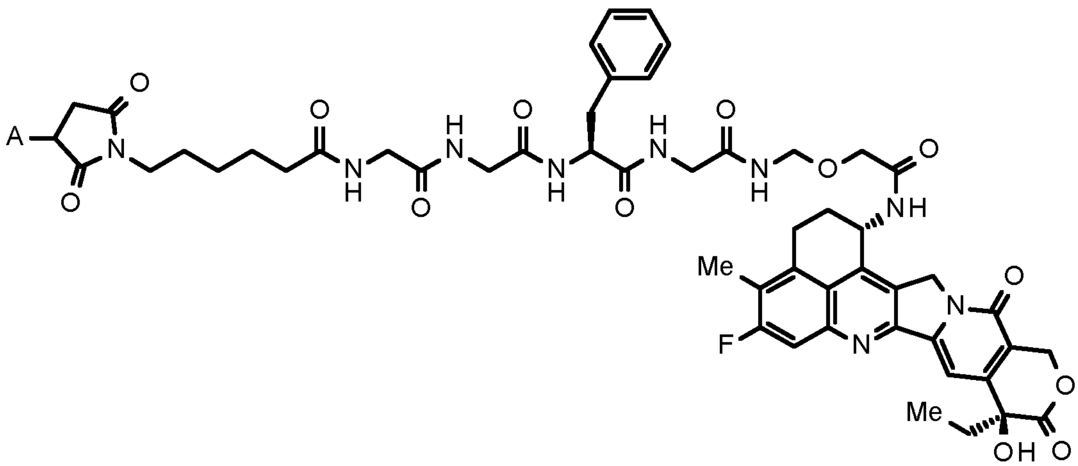

wherein A represents a connecting position to an antibody,
is conjugated to the anti-CDH6 antibody via a thioether bond (hereinafter referred to as the "CDH6-ADC (1)") was produced. The DAR of CDH6-ADC (1) is 7.8.

Example 15: Antitumor study (5)

In Examples 15 to 17, olaparib was suspended into 0.5% hydroxypropyl methylcellulose and 0.1% Tween 80 and orally administered in a fluid volume of 10 mL/kg. Talazoparib was dissolved with dimethyl acetamide, diluted with

5% Kolliphor HS15/Dulbecco's phosphate-buffered saline, and orally administered in a fluid volume of 10 mL/kg.

Human breast cancer cell line MX-1, which was purchased from CLS (Cell Lines Service), was suspended into 50% Matrigel matrix, subcutaneously transplanted at $5\times10^6$ cells into the right side of female nude mice, and the mice were randomly grouped 13 days after the transplantation (Day 0). The HER2-ADC (1) was intravenously administered to the tail vein at a dose of 3 mg/kg on Day 0. The PARP inhibitors were administered once a day, five times a week, for two weeks; at doses of 100 mg/kg for olaparib and 0.4 mg/kg for talazoparib. Single administration groups of each drug, a combined administration group, and a solvent administration group as a control group were set up.

Results of a combination of HER2-ADC (1) and olaparib are shown in Figure 24. Single administration of olaparib showed TGI of 44%. Single administration of HER2-ADC (1) showed TGI of 78%. On the other hand, combined administration of HER2-ADC (1) and olaparib exhibited a significantly superior tumor growth suppression effect than single administration of olaparib (P < 0.0001) and a significantly superior tumor growth suppression effect than single administration of HER2-ADC (1) (P = 0.0001) with TGI of 97%.

Results of a combination of HER2-ADC (1) and talazoparib are shown in Figure 25. Single administration of talazoparib showed TGI of 47%. Single administration of HER2-ADC (1) showed TGI of 78%. On the other hand, combined administration of HER2-ADC (1) and talazoparib exhibited a significantly superior tumor growth suppression effect than single administration of talazoparib (P < 0.0001) and a significantly superior tumor growth suppression effect than single administration of HER2-ADC (1) (P = 0.0001) with TGI of 100%. None of the single and combined administration groups exhibited any particular notable finding such as weight loss.

Example 16: Antitumor study (6)

In Examples 16 and 17, HER3-ADC (1) was diluted with ABS buffer (10 mM acetate buffer (pH 5.5), 5% sorbitol), and intravenously administered in a fluid volume of 10 mL/kg to the tail vein.

Human breast cancer cell line MX-1 was suspended into 50% Matrigel matrix, subcutaneously transplanted at $5\times10^6$ cells into the right side of female nude mice, and the mice were randomly grouped 13 days after the transplantation (Day 0). The HER3-ADC (1) was intravenously administered to the tail vein at a dose of 3 mg/kg on Day 0, Day 7 and Day 15. The PARP inhibitors were administered once a day, five times a week, for two weeks; at doses of 100 mg/kg for olaparib and 0.4 mg/kg for talazoparib. Single administration groups of each drug, a combined administration group, and a solvent administration group as a control group were set up.

Results of a combination of HER3-ADC (1) and olaparib are shown in Figure 26. Single administration of olaparib showed TGI of 23%. Single administration of HER3-ADC (1) showed TGI of 59%. On the other hand, combined administration of HER3-ADC (1) and olaparib exhibited a significantly superior tumor growth suppression effect than single administration of olaparib (P < 0.0001) and a significantly superior tumor growth suppression effect than single administration of HER3-ADC (1) (P = 0.0018) with TGI of 94%.

Results of a combination of HER3-ADC (1) and talazoparib are shown in Figure 27. Single administration of talazoparib showed TGI of 49%. Single administration of HER3-ADC (1) showed TGI of 59%. On the other hand, combined administration of HER3-ADC (1) and talazoparib exhibited a significantly superior tumor growth suppression effect than single administration of talazoparib (P < 0.0001) and a significantly superior tumor growth suppression effect than single administration of HER3-ADC (1) (P < 0.0001) with TGI of 99%. None of the single and combined administration groups exhibited any particular notable finding such as weight loss.

Example 17: Antitumor study (7)

Human breast cancer cell line HCC70 was suspended into physiological saline, subcutaneously transplanted at $1\times10^7$ cells into the right side of female nude mice, and the mice were randomly grouped 13 days after the transplantation (Day 0). The HER3-ADC (1) was intravenously administered to the tail vein at a dose of 10 mg/kg on Day 0, Day 7 and Day 14. The PARP inhibitors were administered once a day, five times a week, for two weeks; at doses of 100 mg/kg for olaparib and 0.4 mg/kg for talazoparib. Single administration groups of each drug, a combined administration group, and a solvent administration group as a control group were set up.

Results of a combination of HER3-ADC (1) and olaparib are shown in Figure 28. Single administration of olaparib showed TGI of 18%. Single administration of HER3-ADC (1) showed TGI of 86%. On the other hand, combined administration of HER3-ADC (1) and olaparib exhibited a significantly superior tumor growth suppression effect than single administration of olaparib (P < 0.0001) with TGI of 97%.

Results of a combination of HER3-ADC (1) and talazoparib are shown in Figure 29. Single administration of talazoparib showed TGI of 19%. Single administration of HER3-ADC (1) showed TGI of 86%. On the other hand, combined administration of HER3-ADC (1) and talazoparib exhibited a significantly superior tumor growth suppression effect than single administration of talazoparib (P < 0.0001) with TGI of 97%. None of the single and combined administration groups exhibited any particular notable finding such as weight loss.

Example 18: Antitumor study (8)

Mouse: Female 5-6-week-old BALB/c nude mice (CHARLES RIVER LABORATORIES JAPAN, INC.) were subjected to the experiments.

Measurement and calculation formula: In all studies, the major axis and minor axis of tumors were measured twice a week with an electronic digital caliper (CD15-CX, Mitutoyo Corp.), and the tumor volume ($mm^3$) was calculated. The calculation formula is as shown below.

$$\text{Tumor volume } (mm^3) = 1/2 \times \text{Major axis (mm)} \times [\text{Minor axis (mm)}]^2$$

Tumor growth inhibition (TGI) was calculated in accordance with the following calculation formula.

$$\text{Tumor growth inhibition } (\%) = 100 \times (1-T/C)$$

T: Average tumor volume of mice of test substance administration group
C: Average tumor volume of mice of control group

TROP2-ADC (1) was diluted with ABS buffer, and intravenously administered in a fluid volume of 10 mL/kg to the tail vein. Olaparib was dissolved with DMSO, diluted with 10% 2-hydroxy-propyl-β-cyclodextrin/Dulbecco's Phosphate-Buffered Saline, and intraperitoneally administered in a fluid volume of 10 mL/kg. Rucaparib (camsylate) was dissolved with DMSO, diluted with physiological saline, and orally administered in a fluid volume of 10 mL/kg. Talazoparib was dissolved with DMSO, diluted with 10% N,N-Dimethylacetamide/5% Kolliphor HS 15/Dulbecco's Phosphate-Buffered Saline, and orally administered in a fluid volume of 10 mL/kg.

Human breast cancer cell line HCC1806, which was purchased from ATCC, was suspended into physiological saline, subcutaneously transplanted at $1\times10^6$ cells into the right side of female nude mice, and the mice were randomly grouped 10 days after the transplantation (Day 0). The TROP2-ADC (1) was administered at a dose of 3 mg/kg on Day 0. The PARP inhibitors were administered once a day, five times a week, for two weeks; at doses of 50 mg/kg for olaparib, 150 mg/kg for rucaparib and 0.8 mg/kg for talazoparib. Single administration groups of each drug, a combined administration group, and a solvent administration group as a control group were set up, and tumor growth inhibition (TGI) on Day 21 was calculated. None of the single and combined administration groups exhibited any particular notable finding such as weight loss.

Results of a combination of TROP2-ADC (1) and olaparib are shown in Figure 30. Single administration of olaparib showed TGI of -9% and single administration of TROP2-ADC (1) showed TGI of 82%. On the other hand, combined administration of TROP2-ADC (1) and olaparib showed TGI of 93% and exhibited a significantly superior tumor growth suppression effect than single administration of olaparib ($P < 0.0001$; calculated by Dunnett's test. The same applies hereinafter) and had a higher tumor growth inhibition than single administration of TROP2-ADC (1).

Results of a combination of TROP2-ADC (1) and rucaparib are shown in Figure 31. Single administration of rucaparib showed TGI of 9% and single administration of TROP2-ADC (1) showed TGI of 82%. On the other hand, combined administration of HER2-ADC (1) and rucaparib showed TGI of 97% and exhibited a significantly superior tumor growth suppression effect than single administration of rucaparib ($P < 0.0001$) and had a higher tumor growth inhibition than single administration of TROP2-ADC (1).

Results of a combination of TROP2-ADC (1) and talazoparib are shown in Figure 32. Single administration of talazoparib showed TGI of 27% and single administration of TROP2-ADC (1) showed TGI of 82%. On the other hand, combined administration of TROP2-ADC (1) and talazoparib showed TGI of 98% and exhibited a significantly superior tumor growth suppression effect than single administration of talazoparib ($P < 0.0001$) and had a significantly superior tumor growth suppression effect ($P = 0.0209$) than single administration of TROP2-ADC (1).

Example 19: Antitumor study (9)

Mouse: Female 5-6-week-old BALB/c nude mice (CHARLES RIVER LABORATORIES JAPAN, INC.) were subjected to the experiments.

Measurement and calculation formula: In all studies, the major axis and minor axis of tumors were measured twice a week with an electronic digital caliper (CD15-CX, Mitutoyo Corp.), and the tumor volume ($mm^3$) was calculated. The calculation formula is as shown below. Tumor volume ($mm^3$) = 1/2 × Major axis (mm) × [Minor axis (mm)]$^2$

Tumor growth inhibition (TGI) was calculated in accordance with the following calculation formula.

$$\text{Tumor growth inhibition (\%)} = 100 \times (1-T/C)$$

T: Average tumor volume of mice of test substance administration group
C: Average tumor volume of mice of control group

CDH6-ADC (1) was diluted with ABS buffer, and intravenously administered in a fluid volume of 10 mL/kg to the tail vein. Olaparib was dissolved with DMSO, diluted with 10% 2-hydroxy-propyl-β-cyclodextrin/Dulbecco's Phosphate-Buffered Saline, and intraperitoneally administered in a fluid volume of 10 mL/kg. Talazoparib was dissolved with DMSO, diluted with 0.5% hydroxypropyl methylcellulose, and orally administered in a fluid volume of 10 mL/kg. Rucaparib (camsylate) and niraparib were dissolved with DMSO, diluted with 0.5% methylcellulose, and orally administered in a fluid volume of 10 mL/kg. Veliparib was dissolved with a 0.05M $CH_3COOH$ solution and orally administered in a fluid volume of 10 mL/kg.

Human ovarian cancer cell line OV-90, which was purchased from ATCC, was suspended into physiological saline, subcutaneously transplanted at $1\times10^7$ cells into the right side of female nude mice, and the mice were randomly grouped 14 days after the transplantation (Day 0). The CDH6-ADC (1) was intravenously administered to the tail vein at a dose of 1 mg/kg on Day 0. The PARP inhibitors were administered once a day, five times a week, for two weeks; at doses of 50 mg/kg for olaparib, 0.4 mg/kg for talazoparib, 150 mg/kg for rucaparib, 50 mg/kg for niraparib and 100 mg/kg for veliparib. Single administration groups of each drug, a combined administration group, and a solvent administration group as a control group were set up. In OV-90 models, a subject developing cachexia-like weight loss appears when tumor size exceeds 1500 $mm^3$. Thus, at Day 17 after the administration, in 2 cases out of 6 cases in the control group, 1 case out of 6 cases in the rucaparib single administration group, and 1 case out of 6 cases in the niraparib single administration group, mice were euthanized due to weight loss. In the single and combined administration groups of CDH6-ADC, there was no subject which exhibited weight loss or was euthanized.

Results of a combination of CDH6-ADC (1) and talazoparib are shown in Figure 33. Single administration of talazoparib showed TGI of 12%. Single administration of CDH6-ADC (1) showed TGI of 66%. On the other hand, combined administration of CDH6-ADC (1) and talazoparib exhibited a significantly superior tumor growth suppression effect than single administration of talazoparib (P = 0.0004; calculated by Dunnett's test. The same applies hereinafter). The combination also had a higher tumor growth inhibition than single administration of CDH6-ADC (1) (TGI, 88%), indicating a potent combination effect.

Results of a combination of CDH6-ADC (1) and rucaparib are shown in Figure 34. Single administration of rucaparib showed TGI of 6%. Single administration of CDH6-ADC (1) showed TGI of 66%. On the other hand, combined administration of CDH6-ADC (1) and rucaparib exhibited a significantly superior tumor growth suppression effect than single administration of rucaparib (P = 0.004). The combination also had a higher tumor growth inhibition than single administration of CDH6-ADC (1) (TGI, 83%), indicating a potent combination effect.

Results of a combination of CDH6-ADC (1) and niraparib are shown in Figure 35. Single administration of niraparib showed TGI of 10%. Single administration of CDH6-ADC (1) showed TGI of 66%. On the other hand, combined administration of CDH6-ADC (1) and niraparib exhibited a significantly superior tumor growth suppression effect than single administration of niraparib (P = 0.0025). The combination also had a higher tumor growth inhibition than single administration of CDH6-ADC (1) (TGI, 76%), indicating a potent combination effect.

Results of a combination of CDH6-ADC (1) and veliparib are shown in Figure 36. Single administration of veliparib showed TGI of 4.4%. Single administration of CDH6-ADC (1) showed TGI of 66%. On the other hand, combined administration of CDH6-ADC (1) and veliparib exhibited a significantly superior tumor growth suppression effect than single administration of veliparib (P = 0.0013). The combination also had a higher tumor growth inhibition than single administration of CDH6-ADC (1) (TGI, 82%), indicating a potent combination effect.

Results of a combination of CDH6-ADC (1) and olaparib are shown in Figure 37. Single administration of olaparib showed TGI of 10%. Single administration of CDH6-ADC (1) showed TGI of 66%. On the other hand, combined administration of CDH6-ADC (1) and olaparib exhibited a significantly superior tumor growth suppression effect than single administration of olaparib (P = 0.0025). The combination also had a higher tumor growth inhibition than single administration of CDH6-ADC (1) (TGI, 79%), indicating a potent combination effect.

Free Text of Sequence Listing

SEQ ID NO: 1 - Amino acid sequence of a heavy chain of the anti-HER2 antibody

SEQ ID NO: 2 - Amino acid sequence of a light chain of the anti-HER2 antibody
SEQ ID NO: 3 - Amino acid sequence of a heavy chain of the anti-HER3 antibody
SEQ ID NO: 4 - Amino acid sequence of a light chain of the anti-HER3 antibody
SEQ ID NO: 5 - Amino acid sequence of a heavy chain of the anti-TROP2 antibody
SEQ ID NO: 6 - Amino acid sequence of a light chain of the anti-TROP2 antibody
SEQ ID NO: 7 - Amino acid sequence of a heavy chain of the anti-B7-H3 antibody
SEQ ID NO: 8 - Amino acid sequence of a light chain of the anti-B7-H3 antibody
SEQ ID NO: 9 - Amino acid sequence of a heavy chain of the anti-GPR20 antibody
SEQ ID NO: 10 - Amino acid sequence of a light chain of the anti-GPR20 antibody
SEQ ID NO: 11 - Amino acid sequence of a heavy chain of the anti-CDH6 antibody
SEQ ID NO: 12 - Amino acid sequence of a light chain of the anti-CDH6 antibody

## Claims

1. A pharmaceutical composition, wherein

an antibody-drug conjugate and a PARP inhibitor are administered in combination, and
the antibody-drug conjugate is an antibody-drug conjugate in which a drug-linker represented by the following formula:

[Formula 1]

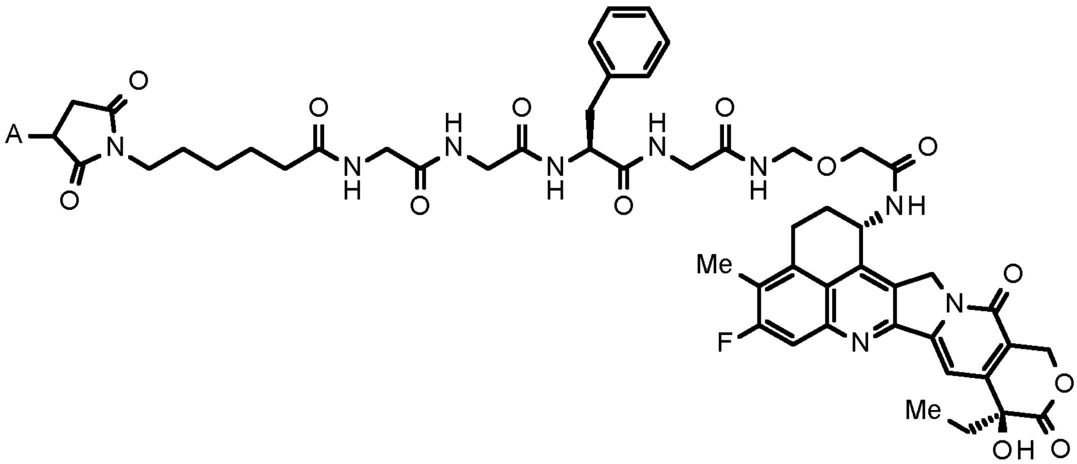

wherein A represents a connecting position to an antibody, is conjugated to the antibody via a thioether bond.

2. The pharmaceutical composition according to claim 1, wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

3. The pharmaceutical composition according to claim 2, wherein the antibody in the antibody-drug conjugate is an anti-HER2 antibody.

4. The pharmaceutical composition according to claim 3, wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

5. The pharmaceutical composition according to claim 3, wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

6. The pharmaceutical composition according to claim 3, wherein the anti-HER2 antibody is an antibody comprising a heavy chain consisting of an amino acid sequence represented by SEQ ID NO: 1 and a light chain consisting of an amino acid sequence represented by SEQ ID NO: 2.

7. The pharmaceutical composition according to claim 3, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**8.** The pharmaceutical composition according to any one of claims 3 to 7, wherein the average number of units of the drug-linker conjugated per antibody molecule in the antibody-drug conjugate is in the range of from 7 to 8.

**9.** The pharmaceutical composition according to claim 2, wherein the antibody in the antibody-drug conjugate is an anti-HER3 antibody.

**10.** The pharmaceutical composition according to claim 9, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**11.** The pharmaceutical composition according to claim 10, wherein the anti-HER3 antibody lacks a lysine residue at the carboxyl terminus of the heavy chain.

**12.** The pharmaceutical composition according to any one of claims 9 to 11, wherein the average number of units of the drug-linker conjugated per antibody molecule in the antibody-drug conjugate is in the range of from 7 to 8.

**13.** The pharmaceutical composition according to claim 2, wherein the antibody in the antibody-drug conjugate is an anti-TROP2 antibody.

**14.** The pharmaceutical composition according to claim 13, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**15.** The pharmaceutical composition according to claim 14, wherein the anti-TROP2 antibody lacks a lysine residue at the carboxyl terminus of the heavy chain.

**16.** The pharmaceutical composition according to any one of claims 13 to 15, wherein the average number of units of the drug-linker conjugated per antibody molecule in the antibody-drug conjugate is in the range of from 3.5 to 4.5.

**17.** The pharmaceutical composition according to claim 2, wherein the antibody in the antibody-drug conjugate is an anti-B7-H3 antibody.

**18.** The pharmaceutical composition according to claim 17, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**19.** The pharmaceutical composition according to claim 18, wherein the anti-B7-H3 antibody lacks a lysine residue at the carboxyl terminus of the heavy chain.

**20.** The pharmaceutical composition according to any one of claims 17 to 19, wherein the average number of units of the drug-linker conjugated per antibody molecule in the antibody-drug conjugate is in the range of from 3.5 to 4.5.

**21.** The pharmaceutical composition according to claim 2, wherein the antibody in the antibody-drug conjugate is an anti-GPR20 antibody.

**22.** The pharmaceutical composition according to claim 21, wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

**23.** The pharmaceutical composition according to claim 22, wherein the anti-GPR20 antibody lacks a lysine residue at the carboxyl terminus of the heavy chain.

**24.** The pharmaceutical composition according to any one of claims 21 to 23, wherein the average number of units of the drug-linker conjugated per antibody molecule in the antibody-drug conjugate is in the range of from 7 to 8.

**25.** The pharmaceutical composition according to claim 2, wherein the antibody in the antibody-drug conjugate is an anti-CDH6 antibody.

**26.** The pharmaceutical composition according to claim 25, wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

**27.** The pharmaceutical composition according to claim 26, wherein the anti-CDH6 antibody lacks a lysine residue at the carboxyl terminus of the heavy chain.

**28.** The pharmaceutical composition according to any one of claims 25 to 27, wherein the average number of units of the drug-linker conjugated per antibody molecule in the antibody-drug conjugate is in the range of from 7 to 8.

**29.** The pharmaceutical composition according to any one of claims 1 to 28, wherein the PARP inhibitor is olaparib, rucaparib, niraparib, talazoparib, or veliparib, or a pharmacologically acceptable salt thereof.

**30.** The pharmaceutical composition according to claim 29, wherein the PARP inhibitor is olaparib or a pharmacologically acceptable salt thereof.

**31.** The pharmaceutical composition according to claim 29, wherein the PARP inhibitor is rucaparib or a pharmacologically acceptable salt thereof.

**32.** The pharmaceutical composition according to claim 29, wherein the PARP inhibitor is niraparib or a pharmacologically acceptable salt thereof.

**33.** The pharmaceutical composition according to claim 29, wherein the PARP inhibitor is talazoparib or a pharmacologically acceptable salt thereof.

**34.** The pharmaceutical composition according to claim 29, wherein the PARP inhibitor is veliparib or a pharmacologically acceptable salt thereof.

**35.** The pharmaceutical composition according to any one of claims 1 to 34, wherein the antibody-drug conjugate and the PARP inhibitor are separately contained as active components in different formulations, and are administered simultaneously or at different times.

**36.** The pharmaceutical composition according to any one of claims 1 to 35, wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

**37.** The pharmaceutical composition according to claim 36, wherein the pharmaceutical composition is for use in treating breast cancer.

**38.** The pharmaceutical composition according to claim 37, wherein the pharmaceutical composition is for use in treating HER2 low-expressing breast cancer.

**39.** The pharmaceutical composition according to claim 36, wherein the pharmaceutical composition is for use in treating gastric cancer.

**40.** The pharmaceutical composition according to claim 36, wherein the pharmaceutical composition is for use in treating ovarian cancer.

**41.** The pharmaceutical composition according to claim 36, wherein the pharmaceutical composition is for use in treating lung cancer.

**42.** The pharmaceutical composition according to claim 36, wherein the pharmaceutical composition is for use in treating pancreatic cancer.

**43.** A pharmaceutical composition wherein an antibody-drug conjugate and a PARP inhibitor are administered in combination, and the antibody-drug conjugate is an antibody-drug conjugate represented by the following formula:

[Formula 2]

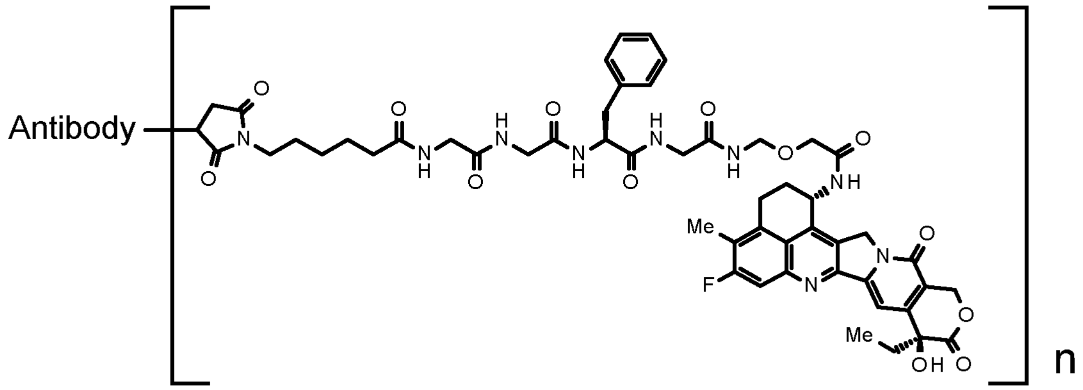

wherein a drug-linker is conjugated to an antibody via a thioether bond, and n indicates the average number of units of the drug-linker conjugated per antibody molecule.

**44.** The pharmaceutical composition according to claim 43, wherein the antibody in the antibody-drug conjugate is an anti-HER2 antibody, an anti-HER3 antibody, an anti-TROP2 antibody, an anti-B7-H3 antibody, an anti-GPR20 antibody, or an anti-CDH6 antibody.

**45.** The pharmaceutical composition according to claim 44, wherein the antibody in the antibody-drug conjugate is an anti-HER2 antibody.

**46.** The pharmaceutical composition according to claim 45, wherein the anti-HER2 antibody is an antibody comprising a heavy chain comprising CDRH1 consisting of an amino acid sequence consisting of amino acid residues 26 to 33 of SEQ ID NO: 1, CDRH2 consisting of an amino acid sequence consisting of amino acid residues 51 to 58 of SEQ ID NO: 1, and CDRH3 consisting of an amino acid sequence consisting of amino acid residues 97 to 109 of SEQ ID NO: 1, and a light chain comprising CDRL1 consisting of an amino acid sequence consisting of amino acid residues 27 to 32 of SEQ ID NO: 2, CDRL2 consisting of an amino acid sequence consisting of amino acid residues 50 to 52 of SEQ ID NO: 2, and CDRL3 consisting of an amino acid sequence consisting of amino acid residues 89 to 97 of SEQ ID NO: 2.

**47.** The pharmaceutical composition according to claim 45, wherein the anti-HER2 antibody is an antibody comprising a heavy chain comprising a heavy chain variable region consisting of an amino acid sequence consisting of amino acid residues 1 to 120 of SEQ ID NO: 1 and a light chain comprising a light chain variable region consisting of an amino acid sequence consisting of amino acid residues 1 to 107 of SEQ ID NO: 2.

**48.** The pharmaceutical composition according to claim 45, wherein the anti-HER2 antibody is an antibody comprising a heavy chain consisting of an amino acid sequence represented by SEQ ID NO: 1 and a light chain consisting of an amino acid sequence represented by SEQ ID NO: 2.

**49.** The pharmaceutical composition according to claim 45, wherein the anti-HER2 antibody is an antibody comprising a heavy chain consisting of an amino acid sequence consisting of amino acid residues 1 to 449 of SEQ ID NO: 1 and a light chain consisting of an amino acid sequence consisting of amino acid residues 1 to 214 of SEQ ID NO: 2.

**50.** The pharmaceutical composition according to any one of claims 45 to 49, wherein the average number of units of the drug-linker conjugated per antibody molecule in the antibody-drug conjugate is in the range of from 7 to 8.

**51.** The pharmaceutical composition according to claim 44, wherein the antibody in the antibody-drug conjugate is an anti-HER3 antibody.

**52.** The pharmaceutical composition according to claim 51, wherein the anti-HER3 antibody is an antibody comprising a heavy chain consisting of an amino acid sequence represented by SEQ ID NO: 3 and a light chain consisting of an amino acid sequence represented by SEQ ID NO: 4.

**53.** The pharmaceutical composition according to claim 52, wherein the anti-HER3 antibody lacks a lysine residue at the carboxyl terminus of the heavy chain.

**54.** The pharmaceutical composition according to any one of claims 51 to 53, wherein the average number of units of the drug-linker conjugated per antibody molecule in the antibody-drug conjugate is in the range of from 7 to 8.

**55.** The pharmaceutical composition according to claim 44, wherein the antibody in the antibody-drug conjugate is an anti-TROP2 antibody.

**56.** The pharmaceutical composition according to claim 55, wherein the anti-TROP2 antibody is an antibody comprising a heavy chain consisting of an amino acid sequence consisting of amino acid residues 20 to 470 of SEQ ID NO: 5 and a light chain consisting of an amino acid sequence consisting of amino acid residues 21 to 234 of SEQ ID NO: 6.

**57.** The pharmaceutical composition according to claim 56, wherein the anti-TROP2 antibody lacks a lysine residue at the carboxyl terminus of the heavy chain.

**58.** The pharmaceutical composition according to any one of claims 55 to 57, wherein the average number of units of the drug-linker conjugated per antibody molecule in the antibody-drug conjugate is in the range of from 3.5 to 4.5.

**59.** The pharmaceutical composition according to claim 44, wherein the antibody in the antibody-drug conjugate is an anti-B7-H3 antibody.

**60.** The pharmaceutical composition according to claim 59, wherein the anti-B7-H3 antibody is an antibody comprising a heavy chain consisting of an amino acid sequence consisting of amino acid residues 20 to 471 of SEQ ID NO: 7 and a light chain consisting of an amino acid sequence consisting of amino acid residues 21 to 233 of SEQ ID NO: 8.

**61.** The pharmaceutical composition according to claim 60, wherein the anti-B7-H3 antibody lacks a lysine residue at the carboxyl terminus of the heavy chain.

**62.** The pharmaceutical composition according to any one of claims 59 to 61, wherein the average number of units of the drug-linker conjugated per antibody molecule in the antibody-drug conjugate is in the range of from 3.5 to 4.5.

**63.** The pharmaceutical composition according to claim 44, wherein the antibody in the antibody-drug conjugate is an anti-GPR20 antibody.

**64.** The pharmaceutical composition according to claim 63, wherein the anti-GPR20 antibody is an antibody comprising a heavy chain consisting of an amino acid sequence consisting of amino acid residues 20 to 472 of SEQ ID NO: 9 and a light chain consisting of an amino acid sequence consisting of amino acid residues 21 to 234 of SEQ ID NO: 10.

**65.** The pharmaceutical composition according to claim 64, wherein the anti-GPR20 antibody lacks a lysine residue at the carboxyl terminus of the heavy chain.

**66.** The pharmaceutical composition according to any one of claims 63 to 65, wherein the average number of units of the drug-linker conjugated per antibody molecule in the antibody-drug conjugate is in the range of from 7 to 8.

**67.** The pharmaceutical composition according to claim 44, wherein the antibody in the antibody-drug conjugate is an anti-CDH6 antibody.

**68.** The pharmaceutical composition according to claim 67, wherein the anti-CDH6 antibody is an antibody comprising a heavy chain consisting of an amino acid sequence consisting of amino acid residues 20 to 471 of SEQ ID NO: 11 and a light chain consisting of an amino acid sequence consisting of amino acid residues 21 to 233 of SEQ ID NO: 12.

**69.** The pharmaceutical composition according to claim 68, wherein the anti-CDH6 antibody lacks a lysine residue at the carboxyl terminus of the heavy chain.

**70.** The pharmaceutical composition according to any one of claims 67 to 69, wherein the average number of units of the drug-linker conjugated per antibody molecule in the antibody-drug conjugate is in the range of from 7 to 8.

**71.** The pharmaceutical composition according to any one of claims 43 to 70, wherein the PARP inhibitor is olaparib, rucaparib, niraparib, talazoparib, or veliparib, or a pharmacologically acceptable salt thereof.

**72.** The pharmaceutical composition according to claim 71, wherein the PARP inhibitor is olaparib or a pharmacologically acceptable salt thereof.

**73.** The pharmaceutical composition according to claim 71, wherein the PARP inhibitor is rucaparib or a pharmacologically acceptable salt thereof.

**74.** The pharmaceutical composition according to claim 71, wherein the PARP inhibitor is niraparib or a pharmacologically acceptable salt thereof.

**75.** The pharmaceutical composition according to claim 71, wherein the PARP inhibitor is talazoparib or a pharmacologically acceptable salt thereof.

**76.** The pharmaceutical composition according to claim 71, wherein the PARP inhibitor is veliparib or a pharmacologically acceptable salt thereof.

**77.** The pharmaceutical composition according to any one of claims 43 to 76, wherein the antibody-drug conjugate and the PARP inhibitor are separately contained as active components in different formulations, and are administered simultaneously or at different times.

**78.** The pharmaceutical composition according to any one of claims 43 to 77, wherein the pharmaceutical composition is for use in treating at least one selected from the group consisting of breast cancer, gastric cancer, colorectal cancer, lung cancer, esophageal cancer, head-and-neck cancer, gastroesophageal junction adenocarcinoma, biliary tract cancer, Paget's disease, pancreatic cancer, ovarian cancer, bladder cancer, prostate cancer, uterine carcinosarcoma, gastrointestinal stromal tumor, kidney cancer, and sarcoma.

**79.** The pharmaceutical composition according to claim 78, wherein the pharmaceutical composition is for use in treating breast cancer.

**80.** The pharmaceutical composition according to claim 79, wherein the pharmaceutical composition is for use in treating HER2 low-expressing breast cancer.

**81.** The pharmaceutical composition according to claim 78, wherein the pharmaceutical composition is for use in treating gastric cancer.

**82.** The pharmaceutical composition according to claim 78, wherein the pharmaceutical composition is for use in treating ovarian cancer.

**83.** The pharmaceutical composition according to claim 78, wherein the pharmaceutical composition is for use in treating lung cancer.

**84.** The pharmaceutical composition according to claim 78, wherein the pharmaceutical composition is for use in treating pancreatic cancer.

[Figure 1]

SEQ ID No: 1 - Amino acid sequence of a heavy chain of the anti-HER2 antibody

E V Q L V E S G G G L V Q P G G S L R L S C A A S G F N I K D T Y I H W V R
Q A P G K G L E W V A R I Y P T N G Y T R Y A D S V K G R F T I S A D T S K
N T A Y L Q M N S L R A E D T A V Y Y C S R W G G D G F Y A M D Y W G Q G T
L V T V S S A S T K G P S V F P L A P S S K S T S G G T A A L G C L V K D Y
F P E P V T V S W N S G A L T S G V H T F P A V L Q S S G L Y S L S S V V T
V P S S S L G T Q T Y I C N V N H K P S N T K V D K K V E P K S C D K T H T
C P P C P A P E L L G G P S V F L F P P K P K D T L M I S R T P E V T C V V
V D V S H E D P E V K F N W Y V D G V E V H N A K T K P R E E Q Y N S T Y R
V V S V L T V L H Q D W L N G K E Y K C K V S N K A L P A P I E K T I S K A
K G Q P R E P Q V Y T L P P S R E E M T K N Q V S L T C L V K G F Y P S D I
A V E W E S N G Q P E N N Y K T T P P V L D S D G S F F L Y S K L T V D K S
R W Q Q G N V F S C S V M H E A L H N H Y T Q K S L S L S P G K

[Figure 2]

SEQ ID No: 2 - Amino acid sequence of a light chain of the anti-HER2 antibody

D I Q M T Q S P S S L S A S V G D R V T I T C R A S Q D V N T A V A W Y Q Q
K P G K A P K L L I Y S A S F L Y S G V P S R F S G S R S G T D F T L T I S
S L Q P E D F A T Y Y C Q Q H Y T T P P T F G Q G T K V E I K R T V A A P S
V F I F P P S D E Q L K S G T A S V V C L L N N F Y P R E A K V Q W K V D N
A L Q S G N S Q E S V T E Q D S K D S T Y S L S S T L T L S K A D Y E K H K
V Y A C E V T H Q G L S S P V T K S F N R G E C

[Figure 3]

SEQ ID No: 3 - Amino acid sequence of a heavy chain of the anti-HER3 antibody

QVQLQQWGAGLLKPSETLSLTCAVYGGSFSGYYWSWIR
QPPGKGLEWIGEINHSGSTNYNPSLKSRVTISVETSKN
QFSLKLSSVTAADTAVYYCARDKWTWYFDLWGRGTLVT
VSSASTKGPSVFPLAPSSKSTSGGTAALGCLVKDYFPE
PVTVSWNSGALTSGVHTFPAVLQSSGLYSLSSVVTVPS
SSLGTQTYICNVNHKPSNTKVDKRVEPKSCDKTHTCPP
CPAPELLGGPSVFLFPPKPKDTLMISRTPEVTCVVVDV
SHEDPEVKFNWYVDGVEVHNAKTKPREEQYNSTYRVVS
VLTVLHQDWLNGKEYKCKVSNKALPAPIEKTISKAKGQ
PREPQVYTLPPSREEMTKNQVSLTCLVKGFYPSDIAVE
WESNGQPENNYKTTPPVLDSDGSFFLYSKLTVDKSRWQ
QGNVFSCSVMHEALHNHYTQKSLSLSPGK

[Figure 4]

SEQ ID No: 4 - Amino acid sequence of a light chain of the anti-HER3 antibody

DIEMTQSPDSLAVSLGERATINCRSSQSVLYSSSNRNY
LAWYQQNPGQPPKLLIYWASTRESGVPDRFSGSGSGTD
FTLTISSLQAEDVAVYYCQQYYSTPRTFGQGTKVEIKR
TVAAPSVFIFPPSDEQLKSGTASVVCLLNNFYPREAKV
QWKVDNALQSGNSQESVTEQDSKDSTYSLSSTLTLSKA
DYEKHKVYACEVTHQGLSSPVTKSFNRGEC

[Figure 5]

SEQ ID No: 5 - Amino acid sequence of a heavy chain of the anti-TROP2 antibody

MKHLWFFLLLVAAPRWVLSQVQLVQSGAEVKKPGASVK
VSCKASGYTFTTAGMQWVRQAPGQGLEWMGWINTHSGV
PKYAEDFKGRVTISADTSTSTAYLQLSSLKSEDTAVYY
CARSGFGSSYWYFDVWGQGTLVTVSSASTKGPSVFPLA
PSSKSTSGGTAALGCLVKDYFPEPVTVSWNSGALTSGV
HTFPAVLQSSGLYSLSSVVTVPSSSLGTQTYICNVNHK
PSNTKVDKRVEPKSCDKTHTCPPCPAPELLGGPSVFLF
PPKPKDTLMISRTPEVTCVVVDVSHEDPEVKFNWYVDG
VEVHNAKTKPREEQYNSTYRVVSVLTVLHQDWLNGKEY
KCKVSNKALPAPIEKTISKAKGQPREPQVYTLPPSREE
MTKNQVSLTCLVKGFYPSDIAVEWESNGQPENNYKTTP
PVLDSDGSFFLYSKLTVDKSRWQQGNVFSCSVMHEALH
NHYTQKSLSLSPGK

Signal sequence (1-19), Variable region (20-140), Constant region (141-470)

[Figure 6]

SEQ ID No: 6 - Amino acid sequence of a light chain of the anti-TROP2 antibody

MVLQTQVFISLLLWISGAYGDIQMTQSPSSLSASVGDR
VTITCKASQDVSTAVAWYQQKPGKAPKLLIYSASYRYT
GVPSRFSGSGSGTDFTLTISSLQPEDFAVYYCQQHYIT
PLTFGQGTKLEIKRTVAAPSVFIFPPSDEQLKSGTASV
VCLLNNFYPREAKVQWKVDNALQSGNSQESVTEQDSKD
STYSLSSTLTLSKADYEKHKVYACEVTHQGLSSPVTKS
FNRGEC

Signal sequence (1-20), Variable region (21-129), Constant region (130-234)

[Figure 7]

SEQ ID No: 7 - Amino acid sequence of a heavy chain of the anti-B7-H3 antibody

MKHLWFFLLLVAAPRWVLSQVQLVQSGAEVKKPGSSVK
VSCKASGYTFTNYVMHWVRQAPGQGLEWMGYINPYNDD
VKYNEKFKGRVTITADESTSTAYMELSSLRSEDTAVYY
CARWGYYGSPLYYFDYWGQGTLVTVSSASTKGPSVFPL
APSSKSTSGGTAALGCLVKDYFPEPVTVSWNSGALTSG
VHTFPAVLQSSGLYSLSSVVTVPSSSLGTQTYICNVNH
KPSNTKVDKRVEPKSCDKTHTCPPCPAPELLGGPSVFL
FPPKPKDTLMISRTPEVTCVVVDVSHEDPEVKFNWYVD
GVEVHNAKTKPREEQYNSTYRVVSVLTVLHQDWLNGKE
YKCKVSNKALPAPIEKTISKAKGQPREPQVYTLPPSRE
EMTKNQVSLTCLVKGFYPSDIAVEWESNGQPENNYKTT
PPVLDSDGSFFLYSKLTVDKSRWQQGNVFSCSVMHEAL
HNHYTQKSLSLSPGK

Signal sequence (1-19), Variable region (20-141), Constant region (142-471)

[Figure 8]

SEQ ID No: 8 - Amino acid sequence of a light chain of the anti-B7-H3 antibody

MVLQTQVFISLLLWISGAYGEIVLTQSPATLSLSPGER
ATLSCRASSRLIYMHWYQQKPGQAPRPLIYATSNLASG
IPARFSGSGSGTDFTLTISSLEPEDFAVYYCQQWNSNP
PTFGQGTKVEIKRTVAAPSVFIFPPSDEQLKSGTASVV
CLLNNFYPREAKVQWKVDNALQSGNSQESVTEQDSKDS
TYSLSSTLTLSKADYEKHKVYACEVTHQGLSSPVTKSF
NRGEC

Signal sequence (1-20), Variable region (21-128), Constant region (129-233)

[Figure 9]

SEQ ID NO: 9 - Amino acid sequence of a heavy chain of the anti-GPR20 antibody

MKHLWFFLLLVAAPRWVLSEVQLVQSGAEVKKPGASVK
VSCKASGYTFTSYYISWIRQAPGQGLKYMGFINPGSGH
TNYNEKFKGRVTITADKSSSTATMELSSLRSEDTAVYY
CARGAGGFLRIITKFDYWGQGTLVTVSSASTKGPSVFP
LAPSSKSTSGGTAALGCLVKDYFPEPVTVSWNSGALTS
GVHTFPAVLQSSGLYSLSSVVTVPSSSLGTQTYICNVN
HKPSNTKVDKRVEPKSCDKTHTCPPCPAPELLGGPSVF
LFPPKPKDTLMISRTPEVTCVVVDVSHEDPEVKFNWYV
DGVEVHNAKTKPREEQYNSTYRVVSVLTVLHQDWLNGK
EYKCKVSNKALPAPIEKTISKAKGQPREPQVYTLPPSR
EEMTKNQVSLTCLVKGFYPSDIAVEWESNGQPENNYKT
TPPVLDSDGSFFLYSKLTVDKSRWQQGNVFSCSVMHEA
LHNHYTQKSLSLSPGK

Signal sequence (1-19), Variable region (20-142), Constant region (143-472)

[Figure 10]

SEQ ID NO: 10 - Amino acid sequence of a light chain of the anti-GPR20 antibody

MVLQTQVFISLLLWISGAYGDTQLTQSPSSLSASVGDR
VTITCRASKSVSTYIHWYQQKPGKQPKLLIYSAGNLES
GVPSRFSGSGSGTDFTLTISSLQPEDFANYYCQQINEL
PYTFGQGTKVEIKRTVAAPSVFIFPPSDEQLKSGTASV
VCLLNNFYPREAKVQWKVDNALQSGNSQESVTEQDSKD
STYSLSSTLTLSKADYEKHKVYACEVTHQGLSSPVTKS
FNRGEC

Signal sequence (1-20), Variable region (21-129), Constant region (130-234)

[Figure 11]

SEQ ID NO: 11 - Amino acid sequence of a heavy chain of the anti-CDH6 antibody

MKHLWFFLLLVAAPRWVLSEVQLVQSGAEVKKPGASVK
VSCKASGYTFTRNFMHWVRQAPGQGLEWMGWIYPGDGE
TEYAQKFQGRVTITADTSTSTAYMELSSLRSEDTAVYY
CARGVYGGFAGGYFDFWGQGTLVTVSSASTKGPSVFPL
APSSKSTSGGTAALGCLVKDYFPEPVTVSWNSGALTSG
VHTFPAVLQSSGLYSLSSVVTVPSSSLGTQTYICNVNH
KPSNTKVDKRVEPKSCDKTHTCPPCPAPELLGGPSVFL
FPPKPKDTLMISRTPEVTCVVVDVSHEDPEVKFNWYVD
GVEVHNAKTKPREEQYNSTYRVVSVLTVLHQDWLNGKE
YKCKVSNKALPAPIEKTISKAKGQPREPQVYTLPPSRE
EMTKNQVSLTCLVKGFYPSDIAVEWESNGQPENNYKTT
PPVLDSDGSFFLYSKLTVDKSRWQQGNVFSCSVMHEAL
HNHYTQKSLSLSPGK

Signal sequence (1-19), Variable region (20-141), Constant region (142-471)

[Figure 12]

SEQ ID NO: 12 - Amino acid sequence of a light chain of the anti-CDH6 antibody

MVLQTQVFISLLLWISGAYGDIQMTQSPSSLSASVGDR
VTITCKASQNIYKNLAWYQQKPGKAPKLLIYDANTLQT
GVPSRFSGSGSGSDFTLTISSLQPEDFATYFCQQYYSG
WAFGQGTKVEIKRTVAAPSVFIFPPSDEQLKSGTASVV
CLLNNFYPREAKVQWKVDNALQSGNSQESVTEQDSKDS
TYSLSSTLTLSKADYEKHKVYACEVTHQGLSSPVTKSF
NRGEC

Signal sequence (1-20), Variable region (21-128), Constant region (129-233)

[Figure 13]

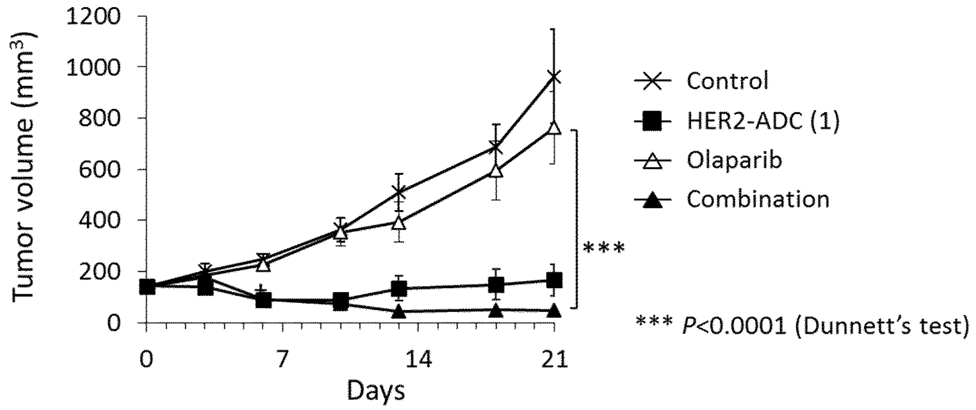


[Figure 14]

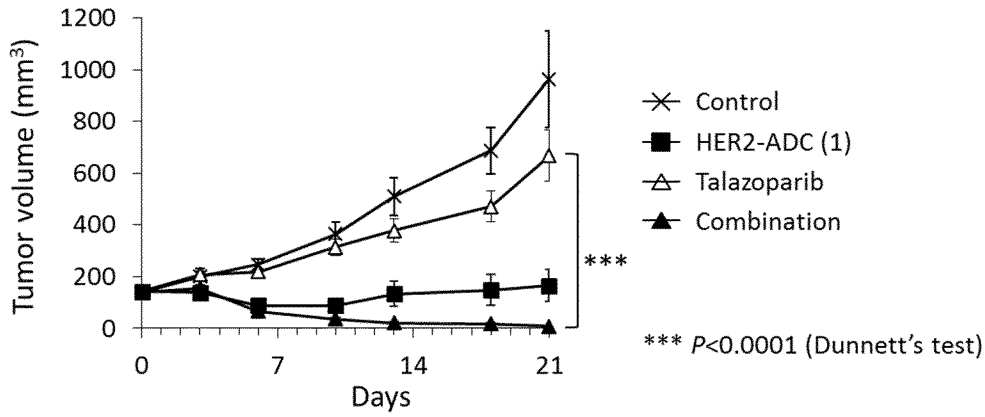


[Figure 15]

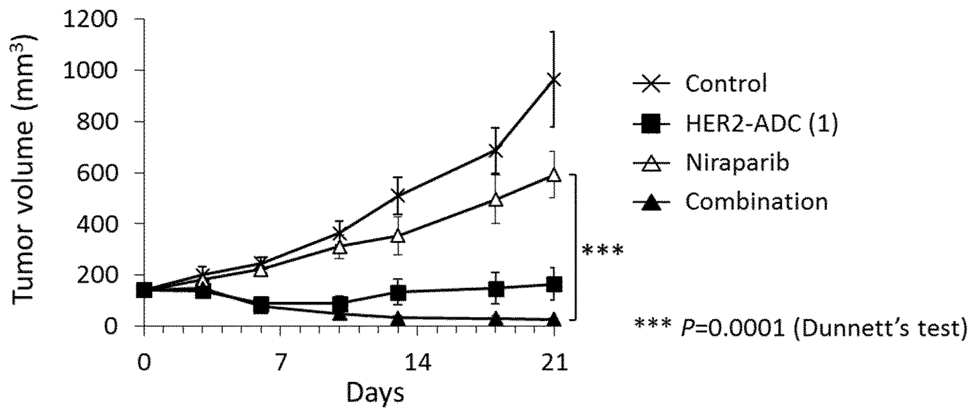

[Figure 16]

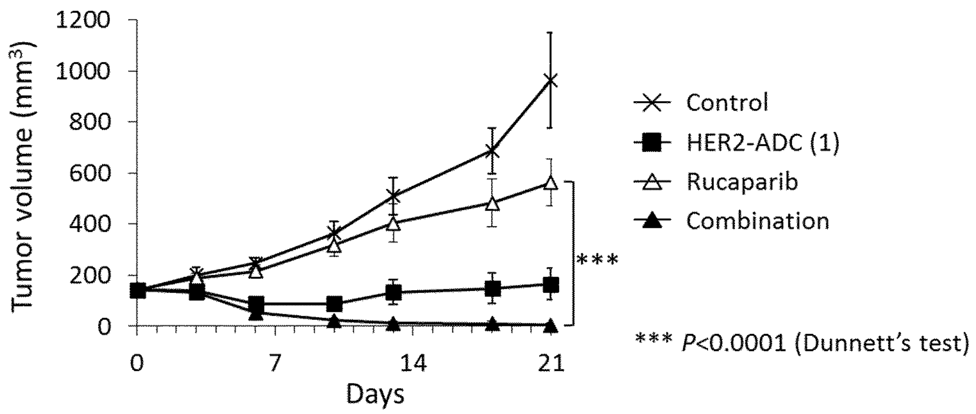


[Figure 17]

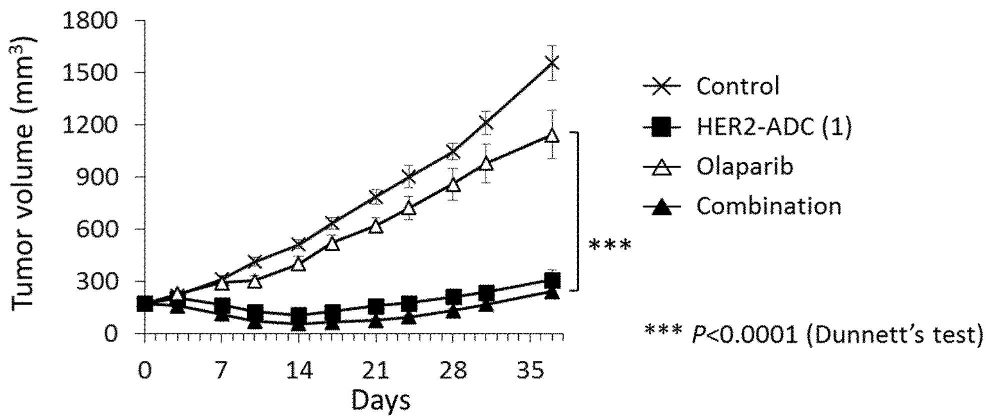


[Figure 18]

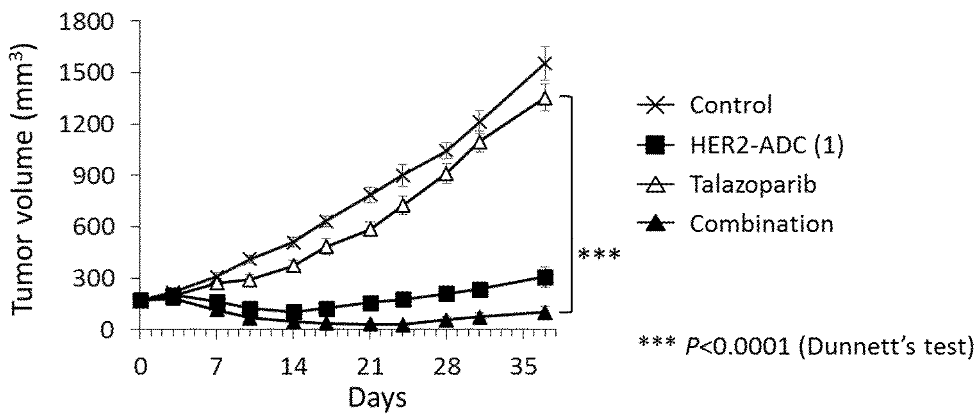

[Figure 19]

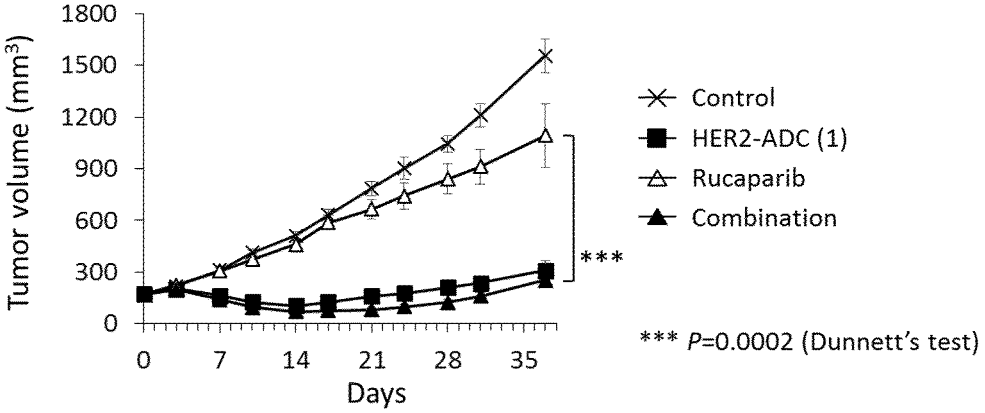


[Figure 20]

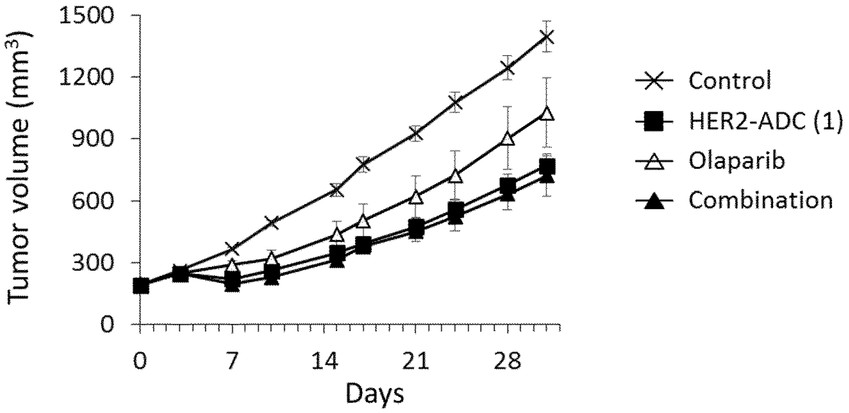


[Figure 21]

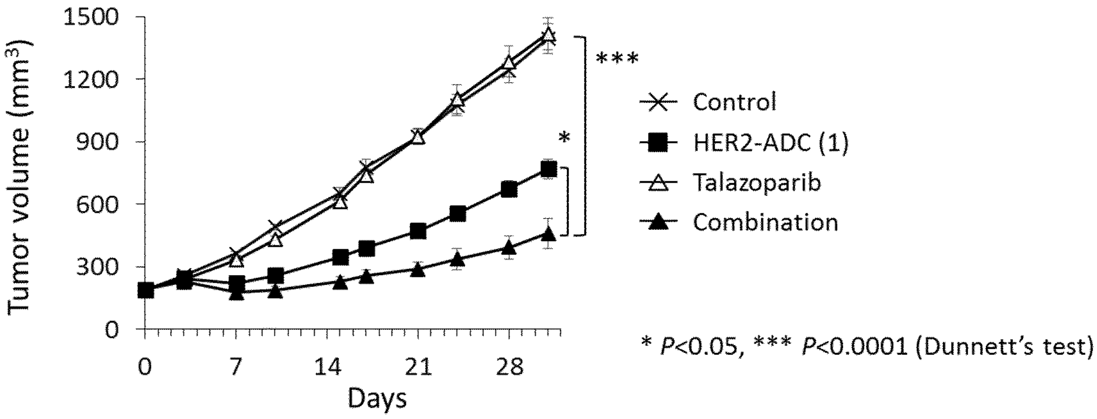

[Figure 22]

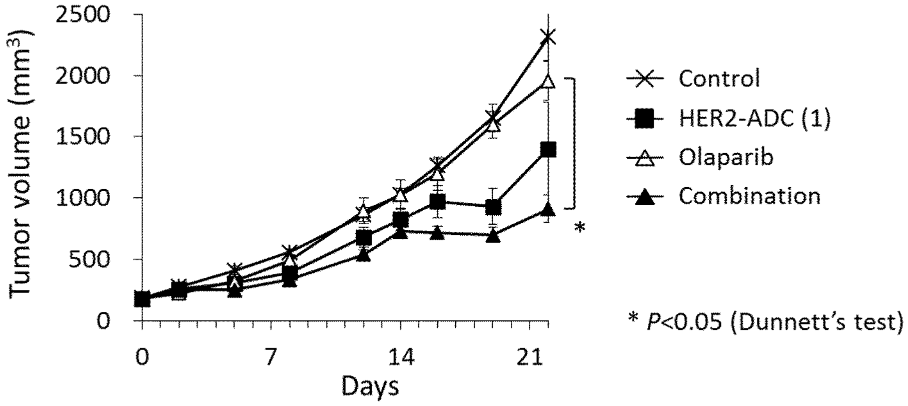


[Figure 23]

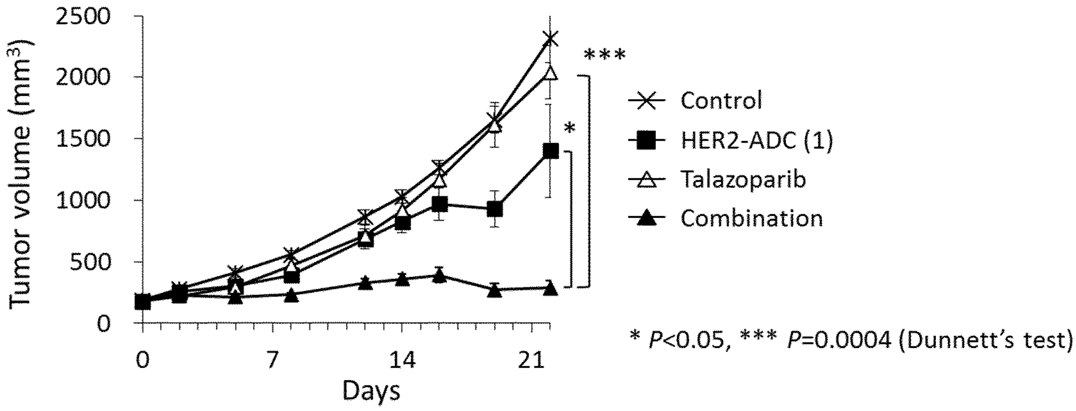


[Figure 24]

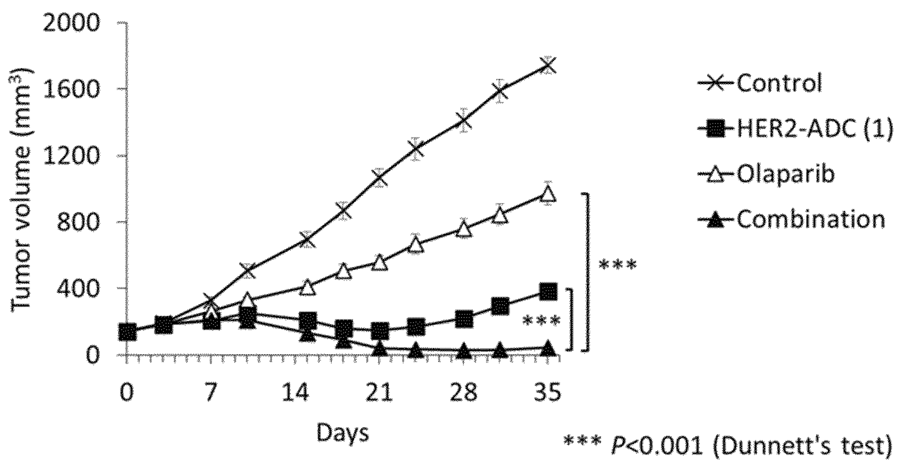

[Figure 25]

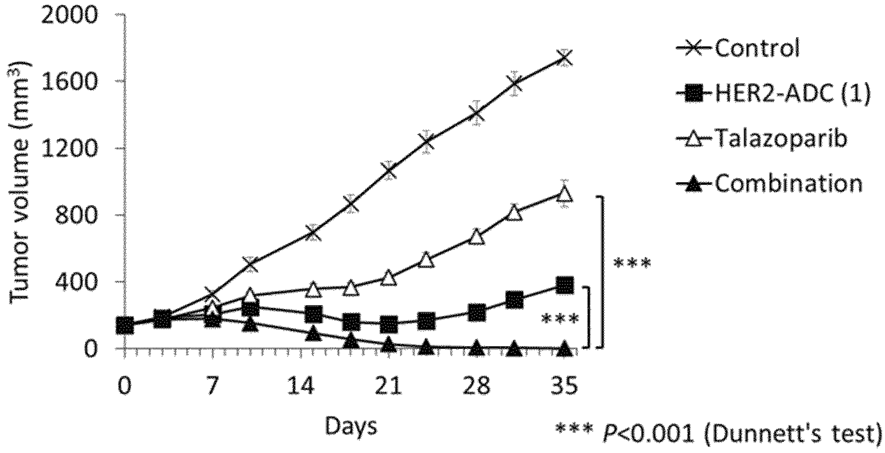


[Figure 26]

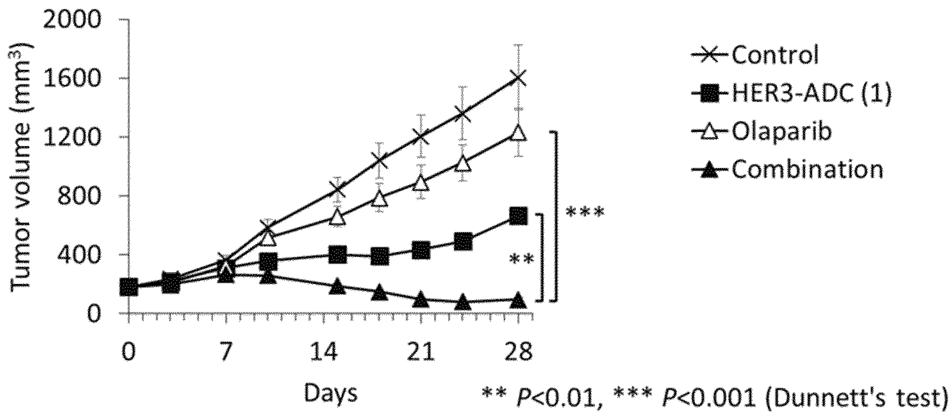


[Figure 27]

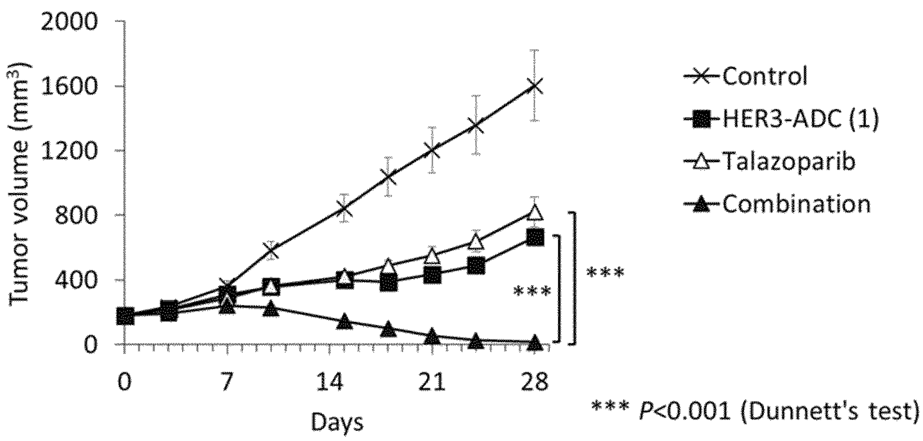

[Figure 28]

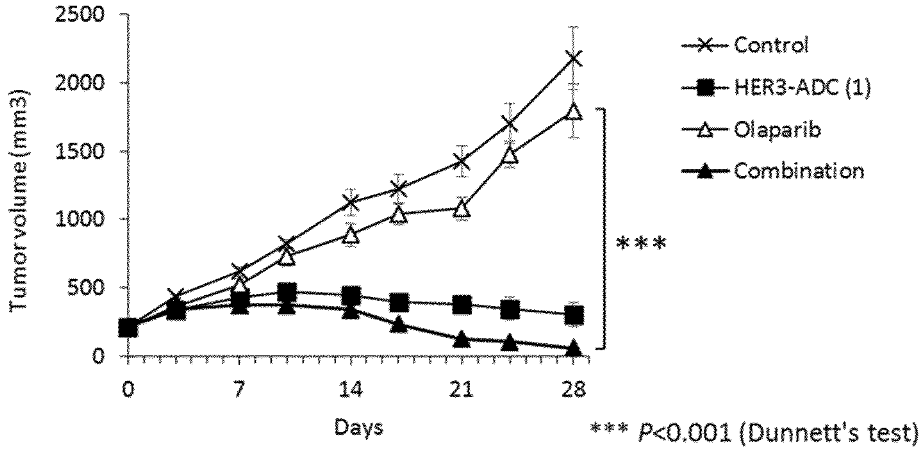


[Figure 29]

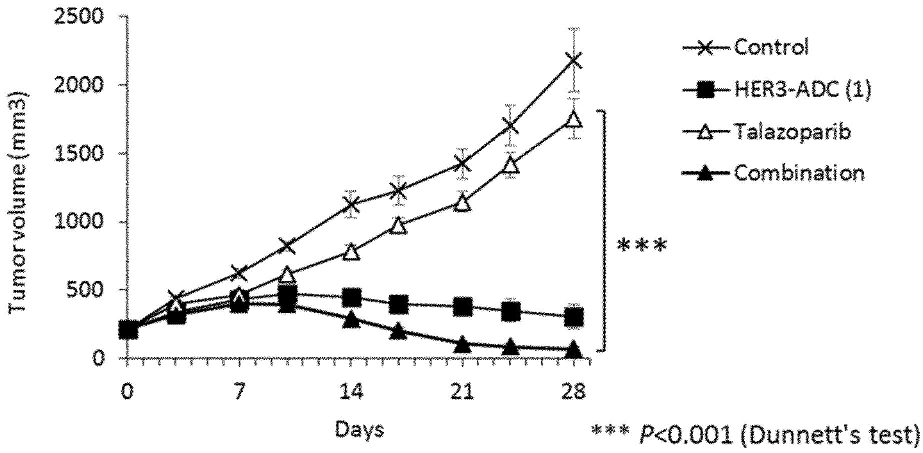


[Figure 30]

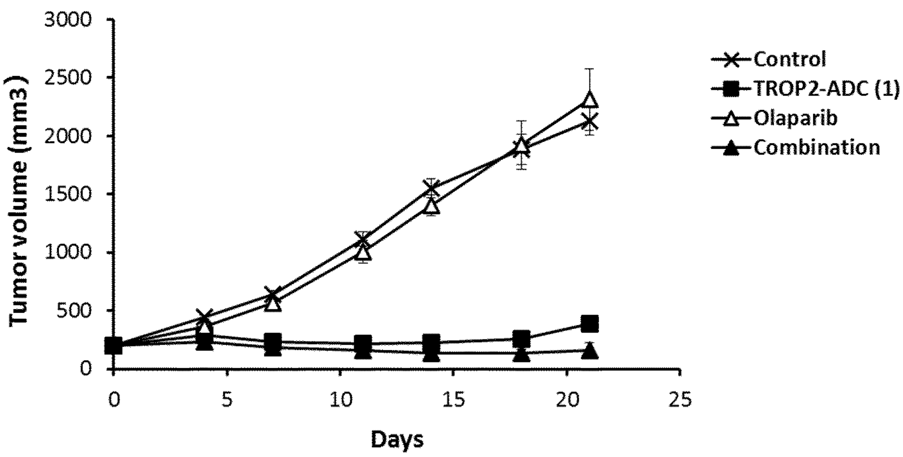

[Figure 31]

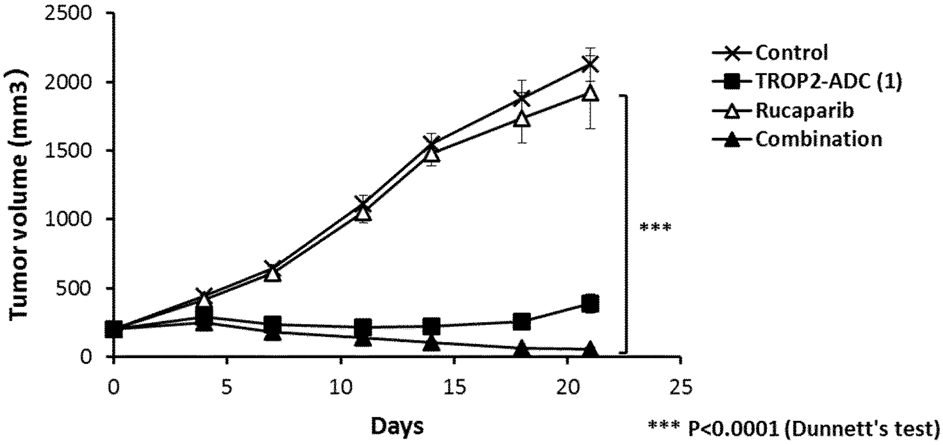


[Figure 32]

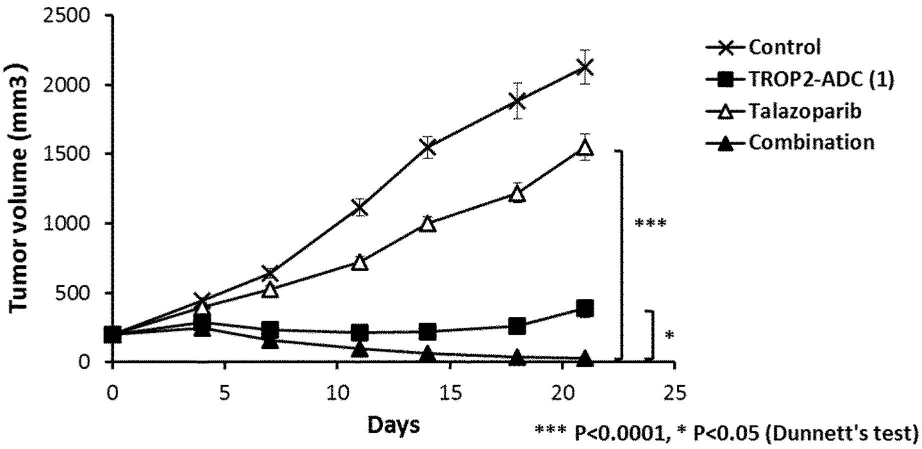


[Figure 33]

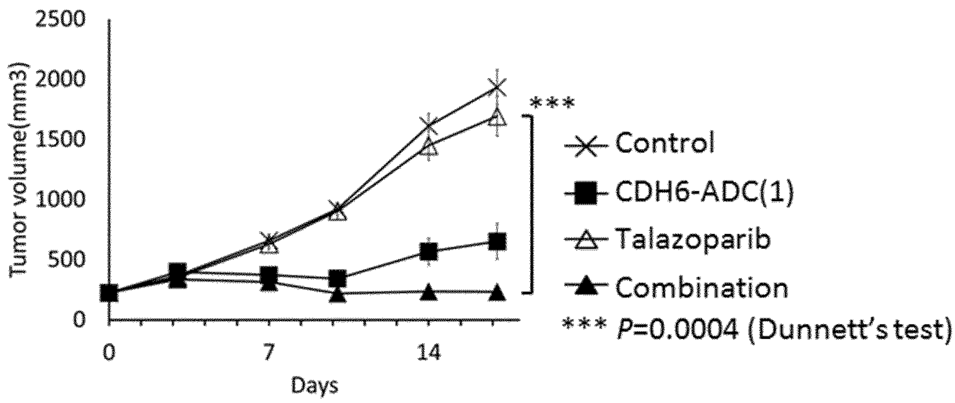

[Figure 34]

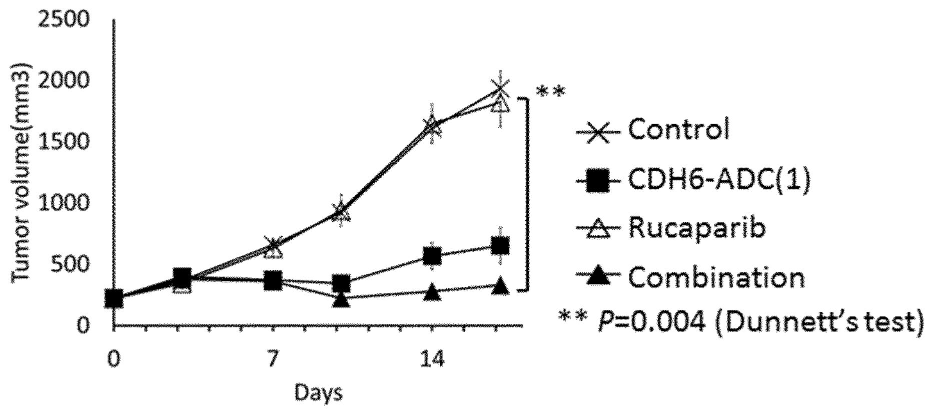


[Figure 35]

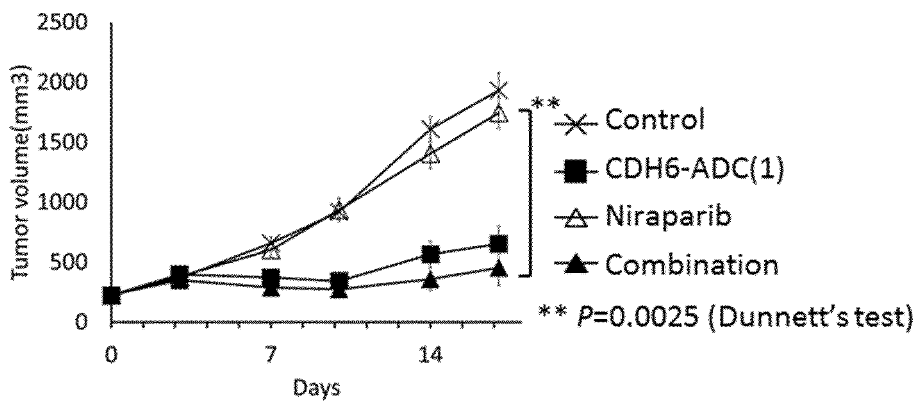


[Figure 36]

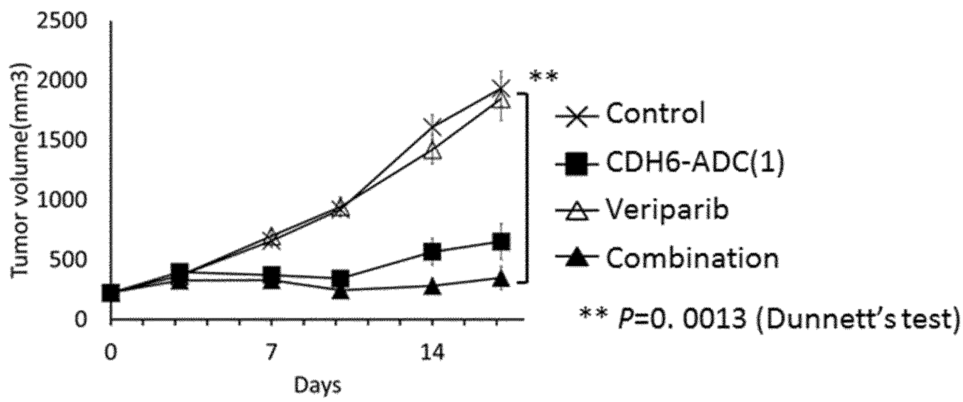

[Figure 37]

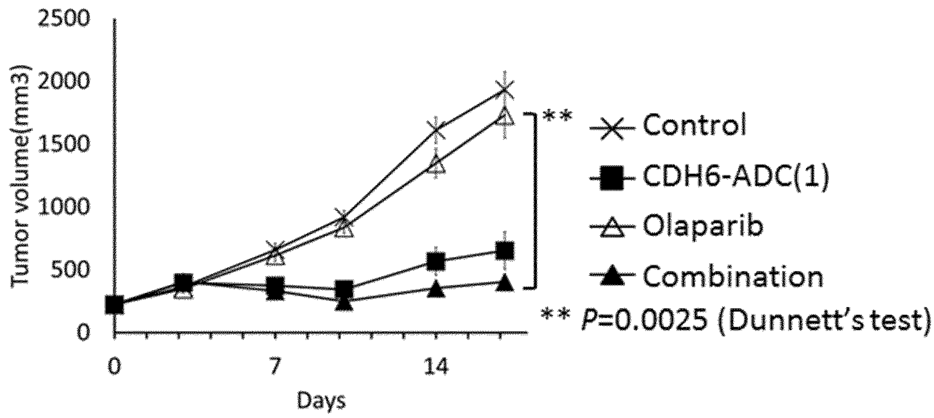
2500
2000
1500
1000
500
0
Tumor volume(mm3)
0
7
14
Days
**
Control
CDH6-ADC(1)
Olaparib
Combination
** P=0.0025 (Dunnett's test)

## INTERNATIONAL SEARCH REPORT

International application No. PCT/JP2019/048171

A. CLASSIFICATION OF SUBJECT MATTER
Int. Cl. A61K31/4184(2006.01)i, A61K31/454(2006.01)i, A61K31/4745(2006.01)i, A61K31/502(2006.01)i, A61K31/5025(2006.01)i, A61K31/55(2006.01)i, A61K45/00(2006.01)i, A61P35/00(2006.01)i, A61P43/00(2006.01)i, C07K16/28(2006.01)i, A61K47/68(2017.01)i, C12N15/13(2006.01)i
FI: A61K47/68, A61K45/00, A61K31/55, A61K31/454, A61K31/5025, A61P35/00, A61P43/00 121, A61K31/4745, AG1K31/502, A61K31/4184, C07K16/28 C12N15/1
According to International Patent Classification (IPC) or to both national classification and IPC

B. FIELDS SEARCHED
Minimum documentation searched (classification system followed by classification symbols)
Int. Cl. A61K31/4184, A61K31/454, AG1K31/4745, AG1K31/502, A61K31/5025, A61K31/55, A61K45/00, A61P35/00, A61P43/00, C07K16/28, A61K47/68, C12N15/13

Documentation searched other than minimum documentation to the extent that such documents are included in the fields searched
Published examined utility model applications of Japan 1922-1996
Published unexamined utility model applications of Japan 1971-2020
Registered utility model specifications of Japan 1996-2020
Published registered utility model applications of Japan 1994-2020

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2015/115091 A1 (DAIICHI SANKYO CO., LTD.) 06 August 2015, claims, paragraphs [0004], examples 30, 31, SEQ ID NO: 1, 2 | 1-8, 29-50, 71-84 |
| Y | WO 2015/155998 A1 (DAIICHI SANKYO CO., LTD.) 15 October 2015, claims, paragraphs [0003], [0047], examples 13, 15, 16a, 16, SEQ ID NO: 583, 584 | 1, 2, 9-12, 29-44, 51-54, 71-84 |
| Y | WO 2015/098099 A1 (DAIICHI SANKYO CO., LTD.) 02 July 2015, claims, paragraphs [0005], [0043], examples 17, 20, SEQ ID NO: 12, 18 | 1, 2, 13-16, 29-44, 55-58, 71-84 |
| Y | WO 2014/057687 A1 (DAIICHI SANKYO CO., LTD.) 17 April 2014, claims, paragraphs [0005], [0086], examples 58, 59, SEQ ID NO: 9, 16 | 1, 2, 17-20, 29-44, 59-62, 71-84 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21.01.2020 | 04.02.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/048171

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2018/135501 A1 (DAIICHI SANKYO CO., LTD.) 26 July 2018, claims, paragraphs [0113], [0124]-[0126], [0129], SEQ ID NO: 50, 64 | 1, 2, 21-24, 29-44, 63-66, 71-84 |
| Y | WO 2018/212136 A1 (DAIICHI SANKYO CO., LTD.) 22 November 2018, claims, paragraphs [0129], examples 7-9, SEQ ID NO: 61, 69 | 1, 2, 25-44, 67-84 |
| Y | BERETTA, G. L. et al., Camptothecin Resistance in Cancer: Insights into the molecular mechanisms of a DNA-damaging drug, Current Medicinal Chemistry, 2013, vol. 20, pages 1541-1565, in particular, abstract、page 1542, left column, third paragraph, fig. 3, page 1554, left column, second paragraph, page 1555, left column, fourth paragraph, table 2 | 1-84 |
| Y | CARDILLO, Thomas M. et al., Synthetic lethality exploitation by an anti-Trop-2-SN-38 antibody-drug conjugate, IMMU-132, plus PARP inhibitors in BRCA1/2-wild-type triple negative breast cancer, Clinical Cancer Research, 2017, vol. 23, no. 13, pages 3405-3415, in particular, abstract | 1-84 |

Form PCT/ISA/210 (continuation of second sheet) (January 2015)

**INTERNATIONAL SEARCH REPORT**
Information on patent family members

International application No.
PCT/JP2019/048171

| Patent Documents referred to in the Report | Publication Date | Patent Family | Publication Date |
|---|---|---|---|
| WO 2015/115091 A1 | 06.08.2015 | US 2016/0333112 A1 claims, examples 30, 31, Seq ID No. 1, 2<br>EP 3101032 A1 | |
| WO 2015/155998 A1 | 15.10.2015 | JP 2017-503784 A claims, paragraphs [0003]-[0005], [0047], examples 13, 15, 16a, 16d, Seq ID No. 583, 584<br>US 2017/0021031 A1<br>EP 3129063 A1 | |
| WO 2015/098099 A1 | 02.07.2015 | US 2016/0297890 A1 claims, paragraphs [0004], [0115], examples 17, 20, Seq ID No. 12, 18<br>EP 3088419 A1 | |
| WO 2014/057687 A1 | 17.04.2014 | US 2015/0297748 A1 claims, paragraphs [0004], [0257], examples 58, 59, Seq ID No. 9, 16<br>EP 2907824 A1 | |
| WO 2018/135501 A1 | 26.07.2018 | US 2019/0225686 A1 claims, paragraphs [0174], [0192], [0188], Seq ID No. 50, 64 | |
| WO 2018/212136 A1 | 22.11.2018 | TW 201900683 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014057687 A
- WO 2014061277 A
- WO 2015098099 A
- WO 2015115091 A
- WO 2015146132 A
- WO 2015155976 A
- WO 2015155998 A
- WO 2018135501 A
- WO 2018212136 A
- WO 9007861 A
- US 5821337 A
- WO 9954342 A
- WO 0061739 A
- WO 0231140 A
- WO 2007133855 A
- WO 2013120066 A
- WO 0100245 A
- WO 2007077028 A
- WO 2008100624 A
- WO 2019044947 A
- WO 2017002776 A


### Non-patent literature cited in the description


- **LORD CJ et al.** *Nature,* 2012, vol. 481, 287-294
- **BENAFIF S et al.** *Onco. Targets Ther.,* 2015, vol. 8, 519-528
- **FONG PC et al.** *N. Engl. J. Med.,* 2009, vol. 361, 123-134
- **FONG PC et al.** *J. Clin. Oncol.,* 2010, vol. 28, 2512-2519
- **GELMON KA et al.** *Lancet Oncol.,* 2011, vol. 12, 852-861
- **MENEAR KA et al.** *J. Med. Chem.,* 2008, vol. 51, 6581-6591
- **GILLMORE AT et al.** *Org. Process Res. Dev.,* 2012, vol. 16, 1897-1904
- **JONES P et al.** *J. Med. Chem.,* 2009, vol. 52, 7170-7185
- **SHEN Y et al.** *Clin. Cancer Res.,* 2013, vol. 19 (18), 5003-15
- **OZA AM et al.** *Lancet Oncol.,* 2015, vol. 16, 87-97
- **TAHARA M et al.** *Mol. Cancer Ther.,* 2014, vol. 13, 1170-1180
- **MIKNYOCZKI S et al.** *Mol. Cancer Ther.,* 2007, vol. 6, 2290-2302
- **CALABRESE CR et al.** *J. Natl. Cancer Inst.,* 2004, vol. 96, 56-67
- **SMITH LM et al.** *Clin. cancer res.,* 2005, vol. 11, 8449-8457
- **GENTHER WILLIAMS SM et al.** *Cancer Cell Int.,* 2015, vol. 15, 14
- **CARDILLO TM et al.** *Clin. cancer res.,* 2017, vol. 13, 3405-3415
- **CHEN EX.** *Invest. New Drugs,* 2016, vol. 4, 450-457
- **DUCRY, L. et al.** *Bioconjugate Chem.,* 2010, vol. 21, 5-13
- **ALLEY, S. C. et al.** *Current Opinion in Chemical Biology,* 2010, vol. 14, 529-537
- **DAMLE N. K.** *Expert Opin. Biol. Ther.,* 2004, vol. 4, 1445-1452
- **SENTER P. D. et al.** *Nature Biotechnology,* 2012, vol. 30, 631-637
- **BURRIS HA et al.** *J. Clin. Oncol.,* 2011, vol. 29 (4), 398-405
- **OGITANI Y. et al.** *Clinical Cancer Research,* 2016, vol. 22 (20), 5097-5108
- **OGITANI Y. et al.** *Cancer Science,* 2016, vol. 107, 1039-1046
- **DOI T et al.** *Lancet Oncol.,* 2017, vol. 18, 1512-22
- **TAKEGAWA N et al.** *Int. J. Cancer,* 2017, vol. 141, 1682-1689
- **YONESAKA K et al.** *Int. Oncogene (2018),* 2017, vol. 141, 1682-1689
- **OGITANI Y. et al.** *Clinical Cancer Research,* 15 October 2016, vol. 22 (20), 5097-5108
- *Cell Death and Differentiation,* 2008, vol. 15, 751-761
- *Molecular Biology of the Cell,* December 2004, vol. 15, 5268-5282

- *Bio Techniques,* January 2000, vol. 28, 162-165
- **KOHLER ; MILSTEIN.** *Nature,* 1975, vol. 256, 495-497
- Monoclonal Antibodies. Plenum Press, N.Y, 1980, 365-367
- *Proc. Natl. Acad. Sci. USA,* 1984, vol. 81, 6851-6855
- *Nature,* 1986, vol. 321, 522-525
- **TOMIZUKA, K. et al.** *Nature Genetics,* 1997, vol. 16, 133-143
- **KUROIWA, Y.** *Nucl. Acids Res.,* 1998, vol. 26, 3447-3448
- **YOSHIDA, H.** Animal Cell Technology: Basic and Applied Aspects. Kluwer Academic Publishers, 1999, vol. 10, 69-73
- **TOMIZUKA, K.** *Proc. Natl. Acad. Sci. USA,* 2000, vol. 97, 722-727
- **WORMSTONE, I. M.** *Investigative Ophthalmology & Visual Science,* 2002, vol. 43 (7), 2301-2308
- **CARMEN, S.** *Briefings in Functional Genomics and Proteomics,* 2002, vol. 1 (2), 189-203
- **SIRIWARDENA, D.** *Ophthalmology,* 2002, vol. 109 (3), 427-431
- *Journal of Chromatography A,* 1995, vol. 705, 129-134
- *Analytical Biochemistry,* 2007, vol. 360, 75-83
- **HERMANSON, G. T.** Bioconjugate Techniques. Academic Press, 1996, 56-136, 456-493
- **GRECO WR. et al.** *Pharmacol. Rev.,* June 1995, vol. 47 (2), 331-85
- **LEHAR J. et al.** *Nat Biotechnol.,* vol. 27 (7), 659-66
- **DR. JUNICHI KUREBAYASHI.** *Kawasaki Medical School [British Journal of Cancer,* 1999, vol. 79 (5/6), 707-717
- *British Journal of Cancer,* 1999, vol. 79 (5/6), 707-717